(19) Europäisches Patentamt
European Patent Office
Office européen des brevets

(11) EP 3 858 994 A1

# (12) EUROPEAN PATENT APPLICATION

published in accordance with Art. 153(4) EPC

(43) Date of publication:
**04.08.2021 Bulletin 2021/31**

(21) Application number: **19867148.9**

(22) Date of filing: **27.09.2019**

(51) Int Cl.:
*C12N 15/13* (2006.01) *A61K 39/395* (2006.01)
*A61P 43/00* (2006.01) *C07K 16/28* (2006.01)
*C07K 16/46* (2006.01) *C12N 1/15* (2006.01)
*C12N 1/19* (2006.01) *C12N 1/21* (2006.01)
*C12N 5/10* (2006.01) *C12N 15/63* (2006.01)
*C12P 21/08* (2006.01)

(86) International application number:
**PCT/JP2019/038421**

(87) International publication number:
**WO 2020/067541 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2018 JP 2018185367**

(71) Applicant: **Kyowa Kirin Co., Ltd.**
**Tokyo 100-0004 (JP)**

(72) Inventors:
- **NIWA, Rinpei**
  **Tokyo 100-0004 (JP)**
- **USAMI, Katsuaki**
  **Tokyo 100-0004 (JP)**

(74) Representative: **Hoffmann Eitle**
**Patent- und Rechtsanwälte PartmbB**
**Arabellastraße 30**
**81925 München (DE)**

## (54) ANTIBODY COMPOSITION

(57) An object of the present invention is to provide an antibody composition, which more selectively exhibits an effector function against a target cell coexpressing two types of antigens that are different from each other and damages the target cell, and also can maintain affinity for the individual target antigens sufficiently high. The present invention relates to an antibody composition against a first antigen and a second antigen that are different from each other, composed of a first IgG half-molecule and a second IgG half-molecule.

EP 3 858 994 A1

SEQUENCE LISTING

<110> Kyowa Kirin Co.,Ltd.

<120> Antibody composition

<130> W528038

<150> JP2018-185367
<151> 2018-09-28

<160> 78

<170> PatentIn version 3.5

<210> 1
<211> 336
<212> DNA
<213> Artificial Sequence

<220>
<223> Description of the artificial sequence: base sequence of CCR6 (KG1684) VL

<400> 1
gatatcgtga tgacacaatc cccactgtcc ctgcctgtca cccctggaga gcctgcctcc 60

atctcttgcc ggtcaagtca gagccttgta catactgatg gaaacaccta cttgcattgg 120

tacctgcaga agccaggcca gtctccacag ctcctcatct atcgggtttc caacagattt 180

tctggggtgc cagacaggtt cagtggcagt gggtcaggga cagatttcac cctcaagatc 240

agcagggttg aggccgagga cgtgggagtt tattactgct cacaaagaac atactttcct 300

ctcacgttcg gtcaggggac caagctggag atcaaa 336

<210> 2
<211> 112
<212> PRT
<213> Artificial Sequence

<220>
<223> Description of the artificial sequence: amino acid sequence of CCR6 (KG1684) VL

<400> 2

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Thr
20 25 30

Asp Gly Asn Thr Tyr Leu His Trp Tyr Leu Gln Lys Pro Gly Gln Ser
35 40 45

Pro Gln Leu Leu Ile Tyr Arg Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Arg
85 90 95

Thr Tyr Phe Pro Leu Thr Phe Gly Gln Gly Thr Lys Leu Glu Ile Lys
100 105 110

<210> 3
<211> 342
<212> DNA
<213> Artificial Sequence

<220>
<223> Description of the artificial sequence: base sequence of CCR6 (KG1684) VH

<400> 3
caggtgcagc tgcaggagtc cggacctggc ctggtgaagc cgtcccagac cctgtccctc 60

acctgcactg tctctgggtt ctcagtgtcc agctcccgtg tacactggat ccgccagcct 120

ccaggaaagg gtctggagtg gatcggagtc atatattatg atggaaaaac agattataat 180

gcaggactta aatccagagt gaccatcagc cgcgacacct ccaagtccca attctcccta 240

aaactgtcca gtgtgaccgc cgccgacaca gccgtgtatt attgtgccgg cggggttatt 300

atggatgcct ggggtcaagg aaccctcgtc actgtctcct cc 342

<210> 4
<211> 114
<212> PRT
<213> Artificial Sequence

<220>
<223> Description of the artificial sequence: amino acid sequence of CCR6 (KG1684) VH

<400> 4

Gln Val Gln Leu Gln Glu Ser Gly Pro Gly Leu Val Lys Pro Ser Gln
1 5 10 15

Thr Leu Ser Leu Thr Cys Thr Val Ser Gly Phe Ser Val Ser Ser Ser
20 25 30

Arg Val His Trp Ile Arg Gln Pro Pro Gly Lys Gly Leu Glu Trp Ile
35 40 45

Gly Val Ile Tyr Tyr Asp Gly Lys Thr Asp Tyr Asn Ala Gly Leu Lys
50 55 60

Ser Arg Val Thr Ile Ser Arg Asp Thr Ser Lys Ser Gln Phe Ser Leu
65 70 75 80

Lys Leu Ser Ser Val Thr Ala Ala Asp Thr Ala Val Tyr Tyr Cys Ala
85 90 95

Gly Gly Val Ile Met Asp Ala Trp Gly Gln Gly Thr Leu Val Thr Val
100 105 110

Ser Ser

<210> 5
<211> 324
<212> DNA
<213> Artificial Sequence

<220>
<223> Description of the artificial sequence: base sequence of IgG1 CK

<400> 5
cgtacggtgg ctgcaccatc tgtcttcatc ttcccgccat ctgatgagca gttgaaatct 60

ggaactgcct ctgttgtgtg cctgctgaat aacttctatc ccagagaggc caaagtacag 120

tggaaggtgg ataacgccct ccaatcgggt aactcccagg agagtgtcac agagcaggac 180

agcaaggaca gcacctacag cctcagcagc accctgacgc tgagcaaagc agactacgag 240

aaacacaaag tctacgcctg cgaagtcacc catcagggcc tgagctcgcc cgtcacaaag 300

agcttcaaca ggggagagtg ttga 324

<210> 6
<211> 107
<212> PRT
<213> Artificial Sequence

<220>
<223> Description of the artificial sequence: amino acid sequence of IgG1 CK

<400> 6

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser

50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 7
<211> 993
<212> DNA
<213> Artificial Sequence

<220>
<223> Description of the artificial sequence: base sequence of IgG1 CH

<400> 7
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 8
<211> 330
<212> PRT
<213> Artificial Sequence

<220>
<223> Description of the artificial sequence: amino acid sequence of IgG1 CH

<400> 8

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220
```

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 9
<211> 993
<212> DNA
<213> Artificial Sequence

<220>
<223> Description of the artificial sequence: base sequence of IgG1_P238A CH

<400> 9
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

gcgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 10
<211> 330
<212> PRT
<213> Artificial Sequence

<220>
<223> Description of the artificial sequence: amino acid sequence of IgG1_P238A CH

<400> 10

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Ala Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 11
<211> 993
<212> DNA
<213> Artificial Sequence

<220>
<223> Description of the artificial sequence: base sequence of IgG1_D265A CH

<400> 11
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggccgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 12
<211> 330
<212> PRT
<213> Artificial Sequence

<220>
<223> Description of the artificial sequence: amino acid sequence of IgG1_D265A CH

<400> 12

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Ala Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 13
<211> 993
<212> DNA
<213> Artificial Sequence

<220>
<223> Description of the artificial sequence: base sequence of

IgG1_S267L CH

<400> 13
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgctc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 14
<211> 330
<212> PRT
<213> Artificial Sequence

<220>
<223> Description of the artificial sequence: amino acid sequence of IgG1_S267L CH

<400> 14

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

```
Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Leu His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
```

305 310 315 320

Gln Lys Ser Leu Ser Leu Ser Pro Gly Lys
325 330

<210> 15
<211> 993
<212> DNA
<213> Artificial Sequence

<220>
<223> Description of the artificial sequence: base sequence of IgG1_P238A/D265A CH

<400> 15
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

gcgtcagtct tcctcttccc cccaaaaccc aaggacaccc tcatgatctc ccggacccct 420

gaggtcacat gcgtggtggt ggccgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 16
<211> 330
<212> PRT
<213> Artificial Sequence

<220>
<223> Description of the artificial sequence: amino acid sequence of IgG1_P238A/D265A CH

<400> 16

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Ala Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Ala Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255
```

```
Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>  17
<211>  993
<212>  DNA
<213>  Artificial Sequence

<220>
<223>  Description of the artificial sequence: base sequence of
       IgG1_P238A/S267L CH

<400>  17
gctagcacca agggcccatc ggtcttcccc ctggcaccct cctccaagag cacctctggg        60

ggcacagcgg ccctgggctg cctggtcaag gactacttcc ccgaaccggt gacggtgtcg       120

tggaactcag gcgccctgac cagcggcgtg cacaccttcc cggctgtcct acagtcctca       180

ggactctact ccctcagcag cgtggtgacc gtgccctcca gcagcttggg cacccagacc       240

tacatctgca acgtgaatca caagcccagc aacaccaagg tggacaagaa agttgagccc       300

aaatcttgtg acaaaactca cacatgccca ccgtgcccag cacctgaact cctgggggga       360

gcgtcagtct tcctcttccc cccaaaaccc aaggacaccc tcatgatctc ccggacccct       420

gaggtcacat gcgtggtggt ggacgtgctc cacgaagacc ctgaggtcaa gttcaactgg       480

tacgtggacg gcgtggaggt gcataatgcc aagacaaagc cgcgggagga gcagtacaac       540

agcacgtacc gtgtggtcag cgtcctcacc gtcctgcacc aggactggct gaatggcaag       600

gagtacaagt gcaaggtctc caacaaagcc ctcccagccc ccatcgagaa aaccatctcc       660

aaagccaaag ggcagccccg agaaccacag gtgtacaccc tgcccccatc ccgggatgag       720

ctgaccaaga accaggtcag cctgacctgc ctggtcaaag gcttctatcc cagcgacatc       780

gccgtggagt gggagagcaa tgggcagccg gagaacaact acaagaccac gcctcccgtg       840

ctggactccg acggctcctt cttcctctac agcaagctca ccgtggacaa gagcaggtgg       900

cagcagggga acgtcttctc atgctccgtg atgcatgagg ctctgcacaa ccactacacg       960
```

cagaagagcc tctccctgtc tccgggtaaa tga 993

<210> 18
<211> 330
<212> PRT
<213> Artificial Sequence

<220>
<223> Description of the artificial sequence: amino acid sequence of IgG1_P238A/S267L CH

<400> 18

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Ala Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Leu His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn

195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 19
<211> 993
<212> DNA
<213> Artificial Sequence

<220>
<223> Description of the artificial sequence: base sequence of IgG1_D265A/S267L CH

<400> 19
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggccgtgctc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 20
<211> 330
<212> PRT
<213> Artificial Sequence

<220>
<223> Description of the artificial sequence: amino acid sequence of IgG1_D265A/S267L CH

<400> 20

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Ala Val Leu His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 21
<211> 993
<212> DNA
<213> Artificial Sequence

<220>
<223> Description of the artificial sequence: base sequence of IgG1_P238A/D265A/S267L CH

<400> 21
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

gcgtcagtct tcctcttccc cccaaaaccc aaggacaccc tcatgatctc ccggacccct 420

gaggtcacat gcgtggtggt ggccgtgctc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 22
<211> 330
<212> PRT
<213> Artificial Sequence

<220>
<223> Description of the artificial sequence: amino acid sequence of IgG1_P238A/D265A/S267L CH

<400> 22

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
```

85 90 95

Lys Val Glu Pro Lys Ser Cys Asp Lys Thr His Thr Cys Pro Pro Cys
100 105 110

Pro Ala Pro Glu Leu Leu Gly Gly Ala Ser Val Phe Leu Phe Pro Pro
115 120 125

Lys Pro Lys Asp Thr Leu Met Ile Ser Arg Thr Pro Glu Val Thr Cys
130 135 140

Val Val Val Ala Val Leu His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 23
<211> 993
<212> DNA
<213> Artificial Sequence

<220>
<223> Description of the artificial sequence: base sequence of IgG1_K326W CH

<400> 23
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caactgggcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 24
<211> 330
<212> PRT
<213> Artificial Sequence

<220>
<223> Description of the artificial sequence: amino acid sequence of IgG1_K326W CH

<400> 24

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Trp Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285
```

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 25
<211> 993
<212> DNA
<213> Artificial Sequence

<220>
<223> Description of the artificial sequence: base sequence of IgG1_L328V CH

<400> 25
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc gtcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 26
<211> 330
<212> PRT
<213> Artificial Sequence

<220>
<223> Description of the artificial sequence: amino acid sequence of IgG1_L328V CH

<400> 26

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Val Pro Ala Pro Ile Glu Lys Thr Ile Ser Lys Ala Lys Gly
    210                 215                 220

```
Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 27
<211> 993
<212> DNA
<213> Artificial Sequence

<220>
<223> Description of the artificial sequence: base sequence of IgG1_P329Y CH

<400> 27

```
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctctacgccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780
```

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 28
<211> 330
<212> PRT
<213> Artificial Sequence

<220>
<223> Description of the artificial sequence: amino acid sequence of IgG1_P329Y CH

<400> 28

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Tyr Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 29
<211> 993
<212> DNA
<213> Artificial Sequence

<220>
<223> Description of the artificial sequence: base sequence of IgG1_K326W/L328V CH

<400> 29
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caactgggcc gtcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 30
<211> 330
<212> PRT
<213> Artificial Sequence

<220>
<223> Description of the artificial sequence: amino acid sequence of IgG1_K326W/L328V CH

<400> 30

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Trp Ala Val Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 31
<211> 993
<212> DNA
<213> Artificial Sequence

<220>
<223> Description of the artificial sequence: base sequence of

IgG1_K326W/P329Y CH

<400> 31
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caactgggcc ctctacgccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

<210> 32
<211> 330
<212> PRT
<213> Artificial Sequence

<220>
<223> Description of the artificial sequence: amino acid sequence of IgG1_K326W/P329Y CH

<400> 32

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

```
Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Trp Ala Leu Tyr Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
```

305 310 315 320

Gln Lys Ser Leu Ser Leu Ser Pro Gly Lys
325 330

<210> 33
<211> 993
<212> DNA
<213> Artificial Sequence

<220>
<223> Description of the artificial sequence: base sequence of IgG1_L328V/P329Y CH

<400> 33
gctagcacca agggcccatc ggtcttcccc ctggcaccct cctccaagag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacatctgca acgtgaatca caagcccagc aacaccaagg tggacaagaa agttgagccc 300
aaatcttgtg acaaaactca cacatgccca ccgtgcccag cacctgaact cctgggggga 360
ccgtcagtct tcctcttccc cccaaaaccc aaggacaccc tcatgatctc ccggacccct 420
gaggtcacat gcgtggtggt ggacgtgagc cacgaagacc ctgaggtcaa gttcaactgg 480
tacgtggacg gcgtggaggt gcataatgcc aagacaaagc cgcgggagga gcagtacaac 540
agcacgtacc gtgtggtcag cgtcctcacc gtcctgcacc aggactggct gaatggcaag 600
gagtacaagt gcaaggtctc caacaaagcc gtctacgccc ccatcgagaa aaccatctcc 660
aaagccaaag ggcagccccg agaaccacag gtgtacaccc tgcccccatc ccgggatgag 720
ctgaccaaga accaggtcag cctgacctgc ctggtcaaag gcttctatcc cagcgacatc 780
gccgtggagt gggagagcaa tgggcagccg gagaacaact acaagaccac gcctcccgtg 840
ctggactccg acggctcctt cttcctctac agcaagctca ccgtggacaa gagcaggtgg 900
cagcagggga acgtcttctc atgctccgtg atgcatgagg ctctgcacaa ccactacacg 960
cagaagagcc tctccctgtc tccgggtaaa tga 993

<210> 34
<211> 330
<212> PRT
<213> Artificial Sequence

<220>
<223> Description of the artificial sequence: amino acid sequence of IgG1_L328V/P329Y CH

<400> 34

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Val Tyr Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255
```

```
Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 35
<211> 993
<212> DNA
<213> Artificial Sequence

<220>
<223> Description of the artificial sequence: base sequence of IgG1_K326W/L328V/P329Y CH

<400> 35

```
gctagcacca agggcccatc ggtcttcccc ctggcaccct cctccaagag cacctctggg      60
ggcacagcgg ccctgggctg cctggtcaag gactacttcc ccgaaccggt gacggtgtcg     120
tggaactcag gcgccctgac cagcggcgtg cacaccttcc cggctgtcct acagtcctca     180
ggactctact ccctcagcag cgtggtgacc gtgccctcca gcagcttggg cacccagacc     240
tacatctgca acgtgaatca caagcccagc aacaccaagg tggacaagaa agttgagccc     300
aaatcttgtg acaaaactca cacatgccca ccgtgcccag cacctgaact cctgggggga     360
ccgtcagtct tcctcttccc cccaaaaccc aaggacaccc tcatgatctc ccggacccct     420
gaggtcacat gcgtggtggt ggacgtgagc cacgaagacc ctgaggtcaa gttcaactgg     480
tacgtggacg gcgtggaggt gcataatgcc aagacaaagc cgcgggagga gcagtacaac     540
agcacgtacc gtgtggtcag cgtcctcacc gtcctgcacc aggactggct gaatggcaag     600
gagtacaagt gcaaggtctc caactgggcc gtctacgccc ccatcgagaa aaccatctcc     660
aaagccaaag ggcagccccg agaaccacag gtgtacaccc tgcccccatc ccgggatgag     720
ctgaccaaga accaggtcag cctgacctgc ctggtcaaag gcttctatcc cagcgacatc     780
gccgtggagt gggagagcaa tgggcagccg gagaacaact acaagaccac gcctcccgtg     840
ctggactccg acggctcctt cttcctctac agcaagctca ccgtggacaa gagcaggtgg     900
cagcagggga acgtcttctc atgctccgtg atgcatgagg ctctgcacaa ccactacacg     960
```

cagaagagcc tctccctgtc tccgggtaaa tga 993

<210> 36
<211> 330
<212> PRT
<213> Artificial Sequence

<220>
<223> Description of the artificial sequence: amino acid sequence of IgG1_K326W/L328V/P329Y CH

<400> 36

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn

195 200 205

Trp Ala Val Tyr Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 37
<211> 993
<212> DNA
<213> Artificial Sequence

<220>
<223> Description of the artificial sequence: base sequence of IgG1114_AA_AAA_D265A CH

<400> 37
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggccgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 38
<211> 330
<212> PRT
<213> Artificial Sequence

<220>
<223> Description of the artificial sequence: amino acid sequence of IgG1114_AA_AAA_D265A CH

<400> 38

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Ala Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 39
<211> 993
<212> DNA
<213> Artificial Sequence

<220>
<223> Description of the artificial sequence: base sequence of IgG1114_AA_AAA_P329Y CH

<400> 39
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

```
tggaactcag gcgccctgac cagcggcgtg cacaccttcc cggctgtcct acagtcctca      180

ggactctact ccctcagcag cgtggtgacc gtgccctcca gcagcttggg cacccagacc      240

tacatctgca acgtgaatca caagcccagc aacaccaagg tggacaagaa agttgagccc      300

aaatcttgtg acaaaactca cacagcgcca ccggcgccag cacctgaact cctgggggga      360

ccgtcagtct tcctcttccc cccaaaaccc aaggacaccc tcatgatctc ccggacccct      420

gaggtcacat gcgtggtggt ggacgtgagc cacgaagacc ctgaggtcaa gttcaactgg      480

tacgtggacg gcgtggaggt gcataatgcc aagacaaagc cgcgggagga gcagtacaac      540

gccacgtacc gtgtggtcag cgtcctcacc gtcctgcacc aggactggct gaatggcaag      600

gagtacaagt gcaaggtctc caacaaagcc ctctacgccc ccatcgcggc aaccatctcc      660

aaagccaaag ggcagccccg agagccacag gtgtacaccc tgcccccatc ccaggaggag      720

atgaccaaga accaggtcag cctgacctgc ctggtcaaag gcttctaccc cagcgacatc      780

gccgtggagt gggagagcaa tgggcagccg gagaacaact acaagaccac gcctcccgtg      840

ctggactccg acggctcctt cttcctctac agcaggctaa ccgtggacaa gagcaggtgg      900

caggagggga atgtcttctc atgctccgtg atgcatgagg ctctgcacaa ccactacaca      960

cagaagagcc tctccctgtc tctgggtaaa tga                                   993
```

```
<210>  40
<211>  330
<212>  PRT
<213>  Artificial Sequence

<220>
<223>  Description of the artificial sequence: amino acid sequence of
       IgG1114_AA_AAA_P329Y CH

<400>  40

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
```

85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Tyr Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 41
<211> 336
<212> DNA
<213> Artificial Sequence

<220>
<223> Description of the artificial sequence: base sequence of CD4 (ibalizumab) VL

<400> 41
gacatcgtga tgacccagtc ccctgactcc ctggctgtgt ccctgggcga gcgcgtgacc 60

atgaactgca agtcctccca gtccctgctg tactccacca accagaagaa ctacctggct 120

tggtaccagc agaagcctgg ccagtcccct aagctgctga tctactgggc ttccacccgc 180

gagtccggcg tgcctgaccg cttctccggc tccggctccg gcaccgactt caccctgacc 240

atctcctccg tgcaggctga ggacgtggct gtgtactact gccagcagta ctactcctac 300

cgcaccttcg gcggcggcac caagctggag atcaag 336

<210> 42
<211> 112
<212> PRT
<213> Artificial Sequence

<220>
<223> Description of the artificial sequence: amino acid sequence of CD4 (ibalizumab) VL

<400> 42

Asp Ile Val Met Thr Gln Ser Pro Asp Ser Leu Ala Val Ser Leu Gly
1               5                   10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

<210> 43

<211> 366
<212> DNA
<213> Artificial Sequence

<220>
<223> Description of the artificial sequence: base sequence of CD4 (ibalizumab) VH

<400> 43
caggtgcagc tgcagcagtc cggccctgag gtggtgaagc ctggcgcttc cgtgaagatg 60

tcctgcaagg cttccggcta caccttcacc tcctacgtga tccactgggt gcgccagaag 120

cctggccagg gcctggactg gatcggctac atcaacccct acaacgacgg caccgactac 180

gacgagaagt tcaagggcaa ggctaccctg acctccgaca cctccacctc caccgcttac 240

atggagctgt cctccctgcg ctccgaggac accgctgtgt actactgcgc tcgcgagaag 300

gacaactacg ctaccggcgc ttggttcgct tactggggcc agggcaccct ggtgaccgtg 360

tcctcc 366

<210> 44
<211> 122
<212> PRT
<213> Artificial Sequence

<220>
<223> Description of the artificial sequence: amino acid sequence of CD4 (ibalizumab) VH

<400> 44

Gln Val Gln Leu Gln Gln Ser Gly Pro Glu Val Val Lys Pro Gly Ala
1 5 10 15

Ser Val Lys Met Ser Cys Lys Ala Ser Gly Tyr Thr Phe Thr Ser Tyr
20 25 30

Val Ile His Trp Val Arg Gln Lys Pro Gly Gln Gly Leu Asp Trp Ile
35 40 45

Gly Tyr Ile Asn Pro Tyr Asn Asp Gly Thr Asp Tyr Asp Glu Lys Phe
50 55 60

Lys Gly Lys Ala Thr Leu Thr Ser Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Glu Lys Asp Asn Tyr Ala Thr Gly Ala Trp Phe Ala Tyr Trp
100 105 110

Gly Gln Gly Thr Leu Val Thr Val Ser Ser
115 120

<210> 45
<211> 321
<212> DNA
<213> Artificial Sequence

<220>
<223> Description of the artificial sequence: base sequence of CD70 (2H5) VL

<400> 45
gaaattgtgt tgacacagtc tccagccacc ctgtctttgt ctccagggga aagagccacc 60

ctctcctgca gggccagtca gagtgttagc agctacttag cctggtacca acagaaacct 120

ggccaggctc ccaggctcct catctatgat gcatccaaca gggccactgg catcccagcc 180

aggttcagtg gcagtgggtc tgggacagac ttcactctca ccatcagcag cctagagcct 240

gaagattttg cagtttatta ctgtcagcag cgtaccaact ggccgctcac tttcggcgga 300

gggaccaagg tggagatcaa a 321

<210> 46
<211> 107
<212> PRT
<213> Artificial Sequence

<220>
<223> Description of the artificial sequence: amino acid sequence of CD70 (2H5) VL

<400> 46

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Thr Asn Trp Pro Leu
85 90 95

Thr Phe Gly Gly Gly Thr Lys Val Glu Ile Lys

100 105

<210> 47
<211> 354
<212> DNA
<213> Artificial Sequence

<220>
<223> Description of the artificial sequence: base sequence of CD70 (2H5) VH

<400> 47
caggtgcagc tggtggagtc tgggggaggc gtggtccagc ctgggaggtc cctgagactc 60

tcctgtgcag cctctggatt taccttcagt agctatatta tgcactgggt ccgccaggct 120

ccaggcaagg ggctggagtg ggtggcagtt atatcatatg atggaagaaa caaatactac 180

gcagactccg tgaagggccg attcaccatc tccagagaca attccaagaa cacgctgtat 240

ctgcaaatga acagcctgag agctgaggac acggctgtgt attactgtgc gagagatacg 300

gatggctacg attttgacta ctggggccag ggaaccctgg tcaccgtctc ctca 354

<210> 48
<211> 118
<212> PRT
<213> Artificial Sequence

<220>
<223> Description of the artificial sequence: amino acid sequence of CD70 (2H5) VH

<400> 48

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ile Met His Trp Val Arg Gln Ala Pro Gly Lys Gly Leu Glu Trp Val
35 40 45

Ala Val Ile Ser Tyr Asp Gly Arg Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Asp Thr Asp Gly Tyr Asp Phe Asp Tyr Trp Gly Gln Gly Thr
100 105 110

Leu Val Thr Val Ser Ser
115

<210> 49
<211> 993
<212> DNA
<213> Artificial Sequence

<220>
<223> Description of the artificial sequence: base sequence of IgG1114_AA_AAA_D265E CH

<400> 49
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggaggtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 50
<211> 330
<212> PRT
<213> Artificial Sequence

<220>
<223> Description of the artificial sequence: amino acid sequence of IgG1114_AA_AAA_S239R CH

<400> 50

Ala Ser Thr Lys Gly Pro Ser Val Phe Pro Leu Ala Pro Ser Ser Lys

```
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Ala Pro Pro Ala
            100                 105                 110

Pro Ala Pro Glu Leu Leu Gly Gly Pro Arg Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255
```

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 51
<211> 993
<212> DNA
<213> Artificial Sequence

<220>
<223> Description of the artificial sequence: base sequence of IgG1114_AA_AAA_D265N CH

<400> 51
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt gaacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 52
<211> 330
<212> PRT
<213> Artificial Sequence

<220>
<223> Description of the artificial sequence: amino acid sequence of IgG1114_AA_AAA_D265N CH

<400> 52

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asn Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

```
Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330


<210>  53
<211>  993
<212>  DNA
<213>  Artificial Sequence

<220>
<223>  Description of the artificial sequence: base sequence of
       IgG1114_AA_AAA_D265E CH

<400>  53
gctagcacca agggcccatc ggtcttcccc ctggcaccct cctccaagag cacctctggg       60

ggcacagcgg ccctgggctg cctggtcaag gactacttcc ccgaaccggt gacggtgtcg      120

tggaactcag gcgccctgac cagcggcgtg cacaccttcc cggctgtcct acagtcctca      180

ggactctact ccctcagcag cgtggtgacc gtgccctcca gcagcttggg cacccagacc      240

tacatctgca acgtgaatca caagcccagc aacaccaagg tggacaagaa agttgagccc      300

aaatcttgtg acaaaactca cacagcgcca ccggcgccag cacctgaact cctgggggga      360

ccgtcagtct tcctcttccc cccaaaaccc aaggacaccc tcatgatctc ccggacccct      420

gaggtcacat gcgtggtggt ggaggtgagc cacgaagacc ctgaggtcaa gttcaactgg      480

tacgtggacg gcgtggaggt gcataatgcc aagacaaagc cgcgggagga gcagtacaac      540

gccacgtacc gtgtggtcag cgtcctcacc gtcctgcacc aggactggct gaatggcaag      600
```

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 54
<211> 330
<212> PRT
<213> Artificial Sequence

<220>
<223> Description of the artificial sequence: amino acid sequence of IgG1114_AA_AAA_D265E CH

<400> 54

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

```
Val Val Val Glu Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330
```

<210> 55
<211> 993
<212> DNA
<213> Artificial Sequence

<220>
<223> Description of the artificial sequence: base sequence of IgG1114_AA_AAA_S267K CH

<400> 55

```
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180
```

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgaag cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 56
<211> 330
<212> PRT
<213> Artificial Sequence

<220>
<223> Description of the artificial sequence: amino acid sequence of IgG1114_AA_AAA_S267K CH

<400> 56

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Lys His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 57

<211> 993
<212> DNA
<213> Artificial Sequence

<220>
<223> Description of the artificial sequence: base sequence of IgG1114_AA_AAA_E269P CH

<400> 57
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacccagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 58
<211> 330
<212> PRT
<213> Artificial Sequence

<220>
<223> Description of the artificial sequence: amino acid sequence of IgG1114_AA_AAA_E269P CH

<400> 58

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Ala Pro Pro Ala
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Pro Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285
```

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330

<210>  59
<211>  993
<212>  DNA
<213>  Artificial Sequence

<220>
<223>  Description of the artificial sequence: base sequence of
       IgG1114_AA_AAA_Y296P CH

<400>  59
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacagcgcca ccggcgccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagccaaac     540

gccacgtacc gtgtggtcag cgtcctcacc gtcctgcacc aggactggct gaatggcaag     600

gagtacaagt gcaaggtctc caacaaagcc ctcccagccc ccatcgcggc aaccatctcc     660

aaagccaaag ggcagccccg agagccacag gtgtacaccc tgcccccatc ccaggaggag     720

atgaccaaga accaggtcag cctgacctgc ctggtcaaag gcttctaccc cagcgacatc     780

gccgtggagt gggagagcaa tgggcagccg gagaacaact acaagaccac gcctcccgtg     840

ctggactccg acggctcctt cttcctctac agcaggctaa ccgtggacaa gagcaggtgg     900

caggagggga atgtcttctc atgctccgtg atgcatgagg ctctgcacaa ccactacaca     960

cagaagagcc tctccctgtc tctgggtaaa tga                                  993

<210>  60
<211>  330
<212>  PRT
<213>  Artificial Sequence

<220>
<223> Description of the artificial sequence: amino acid sequence of IgG1114_AA_AAA_Y296P CH

<400> 60

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Ala Pro Pro Ala
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Pro Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu

225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 61
<211> 993
<212> DNA
<213> Artificial Sequence

<220>
<223> Description of the artificial sequence: base sequence of IgG1114_AA_AAA_S298E CH

<400> 61
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gagacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 62
<211> 330
<212> PRT
<213> Artificial Sequence

<220>
<223> Description of the artificial sequence: amino acid sequence of IgG1114_AA_AAA_S298E CH

<400> 62

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Glu Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 63
<211> 993
<212> DNA
<213> Artificial Sequence

<220>
<223> Description of the artificial sequence: base sequence of IgG1114_AA_AAA_T299A CH

<400> 63
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccgcatacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 64
<211> 330
<212> PRT
<213> Artificial Sequence

<220>
<223> Description of the artificial sequence: amino acid sequence of IgG1114_AA_AAA_T299A CH

<400> 64

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro

```
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Ala Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330


<210>  65
<211>  993
<212>  DNA
<213>  Artificial Sequence

<220>
<223>  Description of the artificial sequence: base sequence of
       IgG1114_AA_AAA_L235R CH
```

<400> 65
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact ccggggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 66
<211> 330
<212> PRT
<213> Artificial Sequence

<220>
<223> IgG1114_AA_AAA_L235R CH

<400> 66

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

```
Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Ala Pro Pro Ala
            100                 105                 110

Pro Ala Pro Glu Leu Arg Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320
```

Gln Lys Ser Leu Ser Leu Ser Leu Gly Lys
325 330

<210> 67
<211> 993
<212> DNA
<213> Artificial Sequence

<220>
<223> Description of the artificial sequence: base sequence of IgG1114_AA_AAA_A327I CH

<400> 67
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaaatc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 68
<211> 330
<212> PRT
<213> Artificial Sequence

<220>
<223> Description of the artificial sequence: amino acid sequence of IgG1114_AA_AAA_A327I CH

<400> 68

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ile Leu Pro Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn

260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 69
<211> 993
<212> DNA
<213> Artificial Sequence

<220>
<223> Description of the artificial sequence: base sequence of IgG1114_AA_AAA_K326G CH

<400> 69
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacggagcc ctcccagccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 70
<211> 330
<212> PRT
<213> Artificial Sequence

<220>
<223> Description of the artificial sequence: amino acid sequence of IgG1114_AA_AAA_K326G CH

<400> 70

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Ala Pro Pro Ala
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205
```

Gly Ala Leu Pro Ala Pro Ile Ala Ala Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330

<210> 71
<211> 993
<212> DNA
<213> Artificial Sequence

<220>
<223> Description of the artificial sequence: base sequence of IgG1114_AA_AAA_L328R CH

<400> 71
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

```
gagtacaagt gcaaggtctc caacaaagcc cgaccagccc ccatcgcggc aaccatctcc      660

aaagccaaag ggcagccccg agagccacag gtgtacaccc tgcccccatc ccaggaggag      720

atgaccaaga accaggtcag cctgacctgc ctggtcaaag gcttctaccc cagcgacatc      780

gccgtggagt gggagagcaa tgggcagccg gagaacaact acaagaccac gcctcccgtg      840

ctggactccg acggctcctt cttcctctac agcaggctaa ccgtggacaa gagcaggtgg      900

caggagggga atgtcttctc atgctccgtg atgcatgagg ctctgcacaa ccactacaca      960

cagaagagcc tctccctgtc tctgggtaaa tga                                   993
```

<210> 72
<211> 330
<212> PRT
<213> Artificial Sequence

<220>
<223> Description of the artificial sequence: amino acid sequence of IgG1114_AA_AAA_L328R CH

<400> 72

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Ala Pro Pro Ala
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
```

145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Arg Pro Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 73
<211> 993
<212> DNA
<213> Artificial Sequence

<220>
<223> Description of the artificial sequence: base sequence of IgG1114_AA_AAA_P329K CH

<400> 73
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcaaggccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 74
<211> 330
<212> PRT
<213> Artificial Sequence

<220>
<223> Description of the artificial sequence: amino acid sequence of IgG1114_AA_AAA_P329K CH

<400> 74

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Lys Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Arg Leu Thr Val Asp Lys Ser Arg Trp Gln Glu Gly Asn
290 295 300

Val Phe Ser Cys Ser Val Met His Glu Ala Leu His Asn His Tyr Thr
305 310 315 320

Gln Lys Ser Leu Ser Leu Ser Leu Gly Lys
325 330

<210> 75
<211> 993

<212> DNA
<213> Artificial Sequence

<220>
<223> Description of the artificial sequence: base sequence of IgG1114_AA_AAA_P329W CH

<400> 75
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacagcgcca ccggcgccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctctgggccc ccatcgcggc aaccatctcc 660

aaagccaaag ggcagccccg agagccacag gtgtacaccc tgcccccatc ccaggaggag 720

atgaccaaga accaggtcag cctgacctgc ctggtcaaag gcttctaccc cagcgacatc 780

gccgtggagt gggagagcaa tgggcagccg gagaacaact acaagaccac gcctcccgtg 840

ctggactccg acggctcctt cttcctctac agcaggctaa ccgtggacaa gagcaggtgg 900

caggagggga atgtcttctc atgctccgtg atgcatgagg ctctgcacaa ccactacaca 960

cagaagagcc tctccctgtc tctgggtaaa tga 993

<210> 76
<211> 330
<212> PRT
<213> Artificial Sequence

<220>
<223> Description of the artificial sequence: amino acid sequence of IgG1114_AA_AAA_P329W CH

<400> 76

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser

35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Trp Ala Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

```
Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330
```

<210> 77
<211> 993
<212> DNA
<213> Artificial Sequence

<220>
<223> Description of the artificial sequence: base sequence of IgG1114_AA_AAA_A330P CH

<400> 77

```
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacagcgcca ccggcgccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

gccacgtacc gtgtggtcag cgtcctcacc gtcctgcacc aggactggct gaatggcaag     600

gagtacaagt gcaaggtctc caacaaagcc ctcccacccc ccatcgcggc aaccatctcc     660

aaagccaaag ggcagccccg agagccacag gtgtacaccc tgcccccatc ccaggaggag     720

atgaccaaga accaggtcag cctgacctgc ctggtcaaag gcttctaccc cagcgacatc     780

gccgtggagt gggagagcaa tgggcagccg gagaacaact acaagaccac gcctcccgtg     840

ctggactccg acggctcctt cttcctctac agcaggctaa ccgtggacaa gagcaggtgg     900

caggagggga atgtcttctc atgctccgtg atgcatgagg ctctgcacaa ccactacaca     960

cagaagagcc tctccctgtc tctgggtaaa tga                                  993
```

<210> 78
<211> 330
<212> PRT
<213> Artificial Sequence

<220>

<223> Description of the artificial sequence: amino acid sequence of IgG1114_AA_AAA_A330P CH

<400> 78

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Ala Pro Pro Ala
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ala Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Pro Pro Ile Ala Ala Thr Ile Ser Lys Ala Lys Gly
210 215 220

Gln Pro Arg Glu Pro Gln Val Tyr Thr Leu Pro Pro Ser Gln Glu Glu
225 230 235 240

```
Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330
```

**Description**

TECHNICAL FIELD

The present invention relates to an antibody composition and a method for producing the same, an IgG half-molecule, and a kit including the IgG half-molecule.

BACKGROUND ART

It is known that antibody preparations approved so far have various action mechanisms (Non-Patent Literature 1). Representative examples thereof include effector functions possessed by an IgG class antibody molecule such as a neutralization activity of inhibiting binding of a ligand of a growth factor or the like and a receptor, an agonistic activity of activating a receptor to which it binds, an antibody-dependent cellular cytotoxicity (hereinafter ADCC) activity, and a complement-dependent cytotoxicity (hereinafter CDC) activity. Among these, from a biomarker analysis of a clinical study of rituximab or trastuzumab, the ADCC activity is suggested to be a clinically important action mechanism of an antibody preparation (Non-Patent Literatures 2 and 3).

An antibody is a tetrameric protein of about 150 kDa composed of a total of four molecules of polypeptide chains: two molecules of heavy chain (H chain) and two molecules of light chain (L chain). As shown in Fig. 1, an antibody is divided into a variable region including a complementarity-determining region (CDR) that is directly involved in antigen binding and has a different amino acid sequence for each antibody clone, and a constant region including an Fc region (hereinafter also abbreviated as Fc) that controls the above-mentioned effector functions or a blood half-life. The Fc includes CH1 to CH3 domains and a hinge domain.

A human antibody is classified into 5 classes: IgG, IgA, IgM, IgD, and IgE having different functions according to the sequence of the H chain constant region. Further, a human IgG class is divided into 4 subclasses from IgG1 to IgG4.

Among these, an antibody of the IgG1 subclass is known to have the highest ADCC activity and CDC activity (Non-Patent Literature 4), and many antibody preparations including rituximab and trastuzumab are IgG1.

On the other hand, the IgG4 subclass is known to have characteristics such that the effector function is low, and further it has an amino acid sequence of an intrinsic hinge domain, and reversible association and dissociation of two H chains in the body called "Fab arm exchange" occurs due to a weak interaction between two CH3 domains as compared with other subclasses (Non-Patent Literature 5 and 6).

The ADCC activity is a mechanism of cytotoxicity caused through the expression of a molecule such as perforin, granzyme, or Fas as a result of the activation of a natural killer cell (hereinafter NK cell) or the like by recognizing Fc of an IgG-type antibody bound to a membrane antigen on a cancer cell surface via a type of an Fc receptor, FcγRIIIa (hereinafter also abbreviated as CD16a) (Non-Patent Literature 1).

By X-ray crystallography, the binding mode between CD16a and human IgG1 has been revealed (Non-Patent Literatures 7 and 8). The CD16a-binding domain on Fc of IgG1 is present at two sites due to the point symmetry of the structure of Fc, and in fact, Fc and IgG bind at a number ratio (stoichiometry) of 1:1. Then, in the two CH2 domains constituting Fc, contact is made in regions (hereinafter CD16a-binding domains) that are different from each other.

Specifically, CD16a interacts with L235, G236, G237, P238, S239, D265, V266, S267, H268, E269, E294, Q295, Y296, N297, S298, T299, R301, N325, A327, 1332, or the like on one of the CH2 domains of IgG1. On the other hand, CD16a simultaneously interacts with L235, G236, G237, K326, A327, L328, P329, A330, or the like on the other CH2 domain of IgG1 (the number represents the position of an amino acid on the CH2 domain according to EU numbering) (Non-Patent Literatures 7, 8, 9, and 10).

It is possible to enhance an ADCC activity by artificially altering the CH2 domain of an antibody preparation to increase the binding ability to CD16a. In fact, there are many known examples of enhancing an ADCC activity by altering an amino acid of the CH2 domain (Non-Patent Literature 11).

Further, it is also known that an ADCC activity can be enhanced by altering a sugar chain of an N-linked complex sugar chain that is bound to Fc (Non-Patent Literature 12). Particularly, the technique for enhancing an ADCC activity by a sugar chain alteration is applied to approved antibody preparations such as mogamulizumab (Non-Patent Literature 13) and obinutuzumab (Non-Patent Literature 14).

A bispecific antibody is an artificially altered antibody molecule configured to be able to bind to two different types of antigens unlike natural antibodies, and many molecular forms have been reported (Non-Patent Literature 15).

A schematic diagram of a structure of a bispecific antibody is shown in Fig. 2A. As an application of the bispecific antibody to a medicine, for example, damage to a cancer cell by binding to the cancer cell and CD3 of a T cell surface (hereinafter, a CD3 bispecific antibody) so as to crosslink both, and enhancement of a drug efficacy by neutralizing two types of functional molecules are exemplified (Non-Patent Literature 15).

In a treatment of cancer or an autoimmune disease, in order to remove a cancer cell that is an etiologic cell or a self-reactive lymphocyte, an antibody preparation exhibiting an effector function of an IgG-type antibody or a T cell

recruiting function of a CD3 bispecific antibody is used.

However, such an etiologic cell is originally derived from a normal cell, and it is generally rare that an etiologic cell and a normal cell can be accurately distinguished from each other by a single surface marker molecule. Therefore, the removal of an etiologic cell utilizing an effector function or the like also attacks a normal cell exhibiting the same antigen molecule, and may result in adverse effects in many cases.

For example, CD20 that is a target antigen of rituximab to be used in a treatment of lymphomas or various autoimmune diseases is expressed in a normal B cell, and HER2 that is a target antigen of trastuzumab to be used in a treatment of breast cancer is expressed in a cardiomyocyte. Therefore, there are concerns about adverse effects by disrupting a normal cell with such an antibody preparation.

On the other hand, Mazor et al. have reported a case where a bispecific antibody having reduced affinity for individual target antigens relatively strongly exhibits an effector activity against a double-positive cell (Non-Patent Literature 16).

CITATION LIST

NON-PATENT LITERATURE

Non-Patent Literature 1: Carter P. Nat Rev Cancer 2001; 1: 118-29
Non-Patent Literature 2: Cartron G, Dacheux L, Salles G, et al. Blood 2002; 99: 754-8
Non-Patent Literature 3: Weng WK, Levy R. J Clin Oncol 2003; 21: 3940-7
Non-Patent Literature 4: Birch, J. R., Lennox, E. S. (Eds.), Monoclonal Antibodies: Principles and Applications, Wiley-Liss, Inc., New York, 1995; p. 45
Non-Patent Literature 5: Aalberse RC and Schuurman J, Immunology 2002; 105: 9-10
Non-Patent Literature 6: Labrijn AF, Nat Biotechnol 2009; 27: 767-71
Non-Patent Literature 7: Sondermann P, Nature 2000; 406: 267-73
Non-Patent Literature 8: Radaev S, J Biol Chem 2001; 276: 16469-77
Non-Patent Literature 9: Ferrara C, Proc Natl Acad Sci 2011; 108; 12660-74
Non-Patent Literature 10: Mizushima T, Genes Cells 2011; 16: 1071-80
Non-Patent Literature 11: Strohl WR, Curr Opin Biotechnol 2009; 20: 685-91
Non-Patent Literature 12: Niwa R, J Pharm Sci 2015; 930-41
Non-Patent Literature 13: Beck A, mAbs 2012; 4: 419-25
Non-Patent Literature 14: Goede V, N Engl J Med 2014; 370: 1101-10
Non-Patent Literature 15: Byrne H, Trends Biotechnol 2013; 31: 621-32
Non-Patent Literature 16: Mazor Y, MAbs 2015; 7: 377-89

SUMMARY OF INVENTION

TECHNICAL PROBLEM

As one method for solving the above-mentioned adverse effects, a technique using a bispecific antibody that exhibits an effector function only when it recognizes two different types of antigens for removing an etiologic cell with higher selectivity is conceivable.

However, as shown in Fig. 2B, when a target cell is attacked using an effector function by a normal bispecific antibody, there is a concern that the bispecific antibody may bind to and attack not only a target cell coexpressing two types of antigens (hereinafter also abbreviated as double-positive cell), but also a cell expressing only one type of target antigen (hereinafter also abbreviated as single-positive cell). Further, a technique for an antibody that selectively exhibits an effector function against a double-positive cell and damages the cell regardless of affinity for individual target antigens has not been known.

Therefore, an object of the present inventors is to provide an antibody composition which more selectively exhibits an effector function against a target cell coexpressing two types of antigens that are different from each other and damages the target cell.

SOLUTION TO PROBLEM

The present inventors conceived that the above problem can be solved by an antibody composition having the following elements [1] to [3] that are different from a normal human IgG1 antibody in a constant region of an antibody

molecule as shown in Fig. 3.

[1] It is a mixture of antibody half-molecules (first and second IgG half-molecules) having antigen binding sites for a first antigen (antigen molecule X) and a second antigen (antigen molecule Y) that are different from each other. That is, it is a mixture of "HL molecules" in which a covalent bond formed by an inter-H chain disulfide bond is not present between the first IgG half-molecule and the second IgG half-molecule.
[2] The HL molecules against each of the antigen molecules X and Y bind to the surface of a target cell (X/Y double-positive cell) expressing both the antigen molecules X and Y and thereafter are associated with each other to form an H2L2 molecule in the same manner as normal IgG and form a CD16a-binding domain, and thus cause an antibody activity.
[3] A CD16a-binding domain is not formed even if the HL molecules against the antigen molecule X or Y are associated with each other on a cell surface to form a homo assembly so that an antibody activity is not caused against a cell expressing only a single antigen molecule.

Based on the above conception, the present inventors found the following (1) and (2) and thus completed the present invention.

(1) With respect to the above [1], as shown in Fig. 4, a hinge domain of an IgG half-molecule is altered so as not to form a disulfide bond between H chains in the hinge domain by substitution or deletion of a part or the whole or modification.
(2) As shown in Fig. 5, CD16a comes into contact with two CH2 domains (in Fig. 5, CH2-A and CH2-B) in Fc at different sites (a region 1 of CH2-A and a region 2 of CH2-B), respectively.

Therefore, when each of the region 2 of CH2-A and the region 1 of CH2-B that are not used for binding are "disrupted" by alteration or the like of an amino acid to attenuate the binding affinity, in a homo assembly of HL molecules having such altered CH2-A, only the region 2 of CH2-A or in a homo assembly of HL molecules having such altered CH2-B, only the region 1 of CH2-B is not present. Therefore, to such a homo assembly, CD16a cannot bind with sufficient affinity.
On the other hand, according to a combination of HL molecules having such altered CH2-A and altered CH2-B, respectively, as constituent elements (hetero assembly), an antibody composition satisfying the above [2] and [3] conditions is obtained.
That is, the present invention relates to the following.

1. An antibody composition, which is an antibody composition against a first antigen and a second antigen that are different from each other, comprising a first IgG half-molecule and a second IgG half-molecule, wherein
each of the first IgG half-molecule and the second IgG half-molecule is composed of one immunoglobulin light chain (hereinafter abbreviated as L chain) and one immunoglobulin heavy chain (hereinafter abbreviated as H chain), the H chain includes an H chain variable region, a hinge domain altered so as not to form a disulfide bond by substitution or deletion of a part or the whole or modification, and CH1 to CH3 domains, and has alteration in either of a first Fcγ receptor IIIA (hereinafter CD16a)-binding domain and a second CD16a-binding domain that are different from each other in the CH2 domain,
the first IgG half-molecule includes an antigen-binding domain that binds to the first antigen, and a CD16a-binding activity in the first CD16a-binding domain is attenuated by the alteration,
the second IgG half-molecule includes an antigen-binding domain that binds to the second antigen, and a CD16a-binding activity in the second CD16a-binding domain is attenuated by the alteration, and
an inter-H chain disulfide bond is not formed between the first IgG half-molecule and the second IgG half-molecule.
2. The antibody composition according to the above 1, wherein the antibody composition exhibits an effector function only for a target cell coexpressing the first antigen and the second antigen and damages the target cell.
3. The antibody composition according to the above 1 or 2, wherein the alteration in the first CD16a-binding domain and the second CD16a-binding domain is substitution, deletion or addition, or modification of an amino acid.
4. The antibody composition according to any one of the above 1 to 3, wherein the first CD16a-binding domain includes at least one selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index.
5. The antibody composition according to the above 4, wherein the alteration in the first CD16a-binding domain is substitution of at least one amino acid residue selected from amino acid residues at position 235, position 236, position 237, position 238, position 239, position 265, position 266, position 267, position 268, position 269, position

294, position 295, position 296, position 297, position 298, position 299, position 301, position 325, position 327, and position 332 numbered according to the EU index.

6. The antibody composition according to the above 5, wherein the alteration in the first CD16a-binding domain is substitution of at least one amino acid residue selected from amino acid residues at position 235, position 238, position 239, position 265, position 267, position 269, position 296, position 298, position 299, and position 327 numbered according to the EU index.

7. The antibody composition according to the above 6, wherein the alteration in the first CD16a-binding domain is substitution of at least one amino acid residue selected from Leu at position 235, Pro at position 238, Ser at position 239, Asp at position 265, Ser at position 267, Glu at position 269, Tyr at position 296, Ser at position 298, Thr at position 299, and Ala at position 327 numbered according to the EU index.

8. The antibody composition according to the above 7, wherein the alteration in the first CD16a-binding domain is at least one amino acid residue substitution selected from L235R, P238A, S239R, D265A, D265N, D265E, S267L, S267K, E269P, Y296P, S298E, T299A, and A327I numbered according to the EU index.

9. The antibody composition according to any one of the above 1 to 8, wherein the second CD16a-binding domain includes at least one selected from amino acid residues at position 235, position 236, position 237, position 326, position 327, position 328, position 329, and position 330 numbered according to the EU index.

10. The antibody composition according to the above 9, wherein the alteration in the second CD16a-binding domain is substitution of at least one amino acid residue selected from amino acid residues at position 235, position 236, position 237, position 326, position 327, position 328, position 329, and position 330 numbered according to the EU index.

11. The antibody composition according to the above 10, wherein the alteration in the second CD16a-binding domain is substitution of at least one amino acid residue selected from at least one selected from amino acid residues at position 326, position 328, position 329, and position 330 numbered according to the EU index.

12. The antibody composition according to the above 11, wherein the alteration in the second CD16a-binding domain is substitution of at least one amino acid residue selected from Lys at position 326, Leu at position 328, Pro at position 329, and Ala at position 330 numbered according to the EU index.

13. The antibody composition according to the above 12, wherein the alteration in the second CD16a-binding domain is at least one amino acid residue substitution selected from K326W, K326G, L328V, L328R, P329Y, P329K, P329W, and A330P numbered according to the EU index.

14. The antibody composition according to any one of the above 1 to 13, wherein the alteration in the first CD16a-binding domain is substitution of at least one amino acid residue selected from amino acid residues at position 235, position 238, position 239, position 265, position 267, position 269, position 296, position 298, position 299, and position 327 numbered according to the EU index, and the alteration in the second CD16a-binding domain is substitution of at least one amino acid residue selected from amino acid residues at position 326, position 328, position 329, and position 330 numbered according to the EU index.

15. The antibody composition according to the above 14, wherein the alteration in the first CD16a-binding domain is substitution of at least one amino acid residue selected from Leu at position 235, Pro at position 238, Ser at position 239, Asp at position 265, Ser at position 267, Glu at position 269, Tyr at position 296, Ser at position 298, Thr at position 299, and Ala at position 327 numbered according to the EU index, and the alteration in the second CD16a-binding domain is substitution of at least one amino acid residue selected from Lys at position 326, Leu at position 328, Pro at position 329, and Ala at position 330 numbered according to the EU index.

16. The antibody composition according to the above 15, wherein the alteration in the first CD16a-binding domain is at least one amino acid residue substitution selected from L235R, P238A, S239R, D265A, D265N, D265E, S267L, S267K, E269P, Y296P, S298E, T299A, and A327I numbered according to the EU index, and the alteration in the second CD16a-binding domain is at least one amino acid residue substitution selected from K326W, K326G, L328V, L328R, P329Y, P329K, P329W, and A330P numbered according to the EU index.

17. The antibody composition according to the above 4, wherein the alteration in the first CD16a-binding domain is substitution of at least one amino acid residue selected from amino acid residues at position 238, position 239, position 265, position 266, position 267, position 268, position 269, position 294, position 295, position 296, position 297, position 298, position 299, position 301, position 325, and position 332 numbered according to the EU index.

18. The antibody composition according to any one of the above 1 to 17, wherein the alteration in the first CD16a-binding domain is substitution of at least one amino acid residue selected from amino acid residues at position 238, position 265, and position 267 numbered according to the EU index.

19. The antibody composition according to the above 18, wherein the alteration in the first CD16a-binding domain is substitution of at least one amino acid residue selected from Pro at position 238, Asp at position 265, and Ser at position 267 numbered according to the EU index.

20. The antibody composition according to the above 19, wherein the alteration in the first CD16a-binding domain is at least one amino acid residue substitution selected from P238A, D265A, and S267L numbered according to the

EU index.

21. The antibody composition according to any one of the above 11 to 20, wherein the alteration in the second CD16a-binding domain is substitution of at least one amino acid residue selected from amino acid residues at position 326, position 328, and position 329 numbered according to the EU index.

22. The antibody composition according to the above 21, wherein the alteration in the second CD16a-binding domain is substitution of at least one amino acid residue selected from Lys at position 326, Leu at position 328, and Pro at position 329 numbered according to the EU index.

23. The antibody composition according to the above 22, wherein the alteration in the second CD16a-binding domain is at least one amino acid residue substitution selected from K326W, L328V, and P329Y numbered according to the EU index.

24. The antibody composition according to any one of the above 1 to 23, wherein the first IgG half-molecule and the second IgG half-molecule include a hinge domain in which at least one amino acid residue of amino acid residues at position 226 and position 229 numbered according to the EU index is substituted.

25. The antibody composition according to any one of the above 1 to 24, wherein immunoglobulin subclasses of the L chain, and the H chain variable region, the CH1 domain, and the CH2 domain of the H chain in the first IgG half-molecule and the second IgG half-molecule are IgG1.

26. The antibody composition according to the above 25, wherein the CH3 domain of the H chain in the first IgG half-molecule and the second IgG half-molecule has a weaker inter-CH3 domain interaction than a CH3 domain of the IgG1 subclass.

27. The antibody composition according to the above 26, wherein immunoglobulin subclass of the CH3 domain of the H chain in the first IgG half-molecule and the second IgG half-molecule is IgG4.

28. The antibody composition according to any one of the above 1 to 27, wherein the antibody composition binds to CD16a through the second CD16a-binding domain in the first IgG half-molecule and the first CD16a-binding domain in the second IgG half-molecule.

29. The antibody composition according to any one of the above 1 to 28, wherein a ratio of sugar chains in which fucose is not bound to N-acetylglucosamine at a reducing end of the sugar chain among the total N-glycoside-linked type sugar chains bound to an Fc region in the first IgG half-molecule and the second IgG half-molecule is 20% or more.

30. The antibody composition according to any one of the above 1 to 29, wherein the first IgG half-molecule and the second IgG half-molecule include at least one amino acid residue substitution for further enhancing the CD16a-binding activity in the CH2 domain.

31. The antibody composition according to the above 30, wherein the first IgG half-molecule and the second IgG half-molecule include at least one amino acid residue substitution selected from S298A, E333A, and K334A numbered according to the EU index in the CH2 domain.

32. A first IgG half-molecule, which is a first IgG half-molecule to be used in combination with a second IgG half-molecule, wherein each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,

the first IgG half-molecule includes an antigen-binding domain that binds to a first antigen, and a CD16a-binding activity in the first CD16a-binding domain is attenuated by the alteration,

the second IgG half-molecule includes an antigen-binding domain that binds to a second antigen different from the first antigen, and a CD16a-binding activity in the second CD16a-binding domain is attenuated by the alteration, and an inter-H chain disulfide bond is not formed between the first IgG half-molecule and the second IgG half-molecule.

33. A first IgG half-molecule, which is a first IgG half-molecule to be used for producing an antibody composition against a first antigen and a second antigen that are different from each other, the antibody composition composed of the first IgG half-molecule and a second IgG half-molecule, wherein

each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,

the first IgG half-molecule includes an antigen-binding domain that binds to the first antigen, and a CD16a-binding activity in the first CD16a-binding domain is attenuated by the alteration,

the second IgG half-molecule includes an antigen-binding domain that binds to the second antigen, and a CD16a-binding activity in the second CD16a-binding domain is attenuated by the alteration, and

an inter-H chain disulfide bond is not formed between the first IgG half-molecule and the second IgG half-molecule.

34. The first IgG half-molecule according to the above 32 or 33, wherein the first CD16a-binding domain includes at least one selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index.

35. The first IgG half-molecule according to the above 34, wherein the alteration in the first CD16a-binding domain is substitution of at least one amino acid residue selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index.

36. The first IgG half-molecule according to the above 35, wherein the alteration in the first CD16a-binding domain is substitution of at least one amino acid residue selected from amino acid residues at position 238, position 239, position 265, position 266, position 267, position 268, position 269, position 294, position 295, position 296, position 297, position 298, position 299, position 301, position 325, and position 332 numbered according to the EU index.

37. The first IgG half-molecule according to the above 36, wherein the alteration in the first CD16a-binding domain is substitution of at least one amino acid residue selected from amino acid residues at position 235, at position 238, position 239, position 265, position 267, position 269, position 296, position 298, position 299, and position 327 numbered according to the EU index.

38. The first IgG half-molecule according to the above 37, wherein the alteration in the first CD16a-binding domain is substitution of at least one amino acid residue selected from Leu at position 235, Pro at position 238, Ser at position 239, Asp at position 265, Ser at position 267, Glu at position 269, Tyr at position 296, Ser at position 298, Thr at position 299, and Ala at position 327 numbered according to the EU index.

39. The first IgG half-molecule according to the above 38, wherein the alteration in the first CD16a-binding domain is at least one amino acid residue substitution selected from L235R, P238A, S239R, D265A, D265N, D265E, S267L, S267K, E269P, Y296P, S298E, T299A, and A327I numbered according to the EU index.

40. A second IgG half-molecule, which is a second IgG half-molecule to be used in combination with a first IgG half-molecule, wherein each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,

the first IgG half-molecule includes an antigen-binding domain that binds to a first antigen, and a CD16a-binding activity in the first CD16a-binding domain is attenuated by the alteration,

the second IgG half-molecule includes an antigen-binding domain that binds to a second antigen different from the first antigen, and a CD16a-binding activity in the second CD16a-binding domain is attenuated by the alteration, and

an inter-H chain disulfide bond is not formed between the first IgG half-molecule and the second IgG half-molecule.

41. A second IgG half-molecule, which is a second IgG half-molecule to be used for producing an antibody composition against a first antigen and a second antigen that are different from each other, the antibody composition composed of a first IgG half-molecule and the second IgG half-molecule, wherein

each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,

the first IgG half-molecule includes an antigen-binding domain that binds to the first antigen, and a CD16a-binding activity in the first CD16a-binding domain is attenuated by the alteration,

the second IgG half-molecule includes an antigen-binding domain that binds to the second antigen, and a CD16a-binding activity in the second CD16a-binding domain is attenuated by the alteration, and

an inter-H chain disulfide bond is not formed between the first IgG half-molecule and the second IgG half-molecule.

42. The second IgG half-molecule according to the above 40 or 41, wherein the second CD16a-binding domain includes at least one selected from amino acid residues at position 235, position 236, position 237, position 326, position 327, position 328, position 329, and position 330 numbered according to the EU index.

43. The second IgG half-molecule according to the above 42, wherein the alteration in the second CD16a-binding domain is substitution of at least one amino acid residue selected from amino acid residues at position 235, position 236, position 237, position 326, position 327, position 328, position 329, and position 330 numbered according to the EU index.

44. The second IgG half-molecule according to the above 43, wherein the alteration in the second CD16a-binding domain is substitution of at least one amino acid residue selected from amino acid residues at position 326, position

328, position 329, and position 330 numbered according to the EU index.

45. The second IgG half-molecule according to the above 44, wherein the alteration in the second CD16a-binding domain is substitution of at least one amino acid residue selected from Lys at position 326, Leu at position 328, Pro at position 329, and Ala at position 330 numbered according to the EU index.

46. The second IgG half-molecule according to the above 45, wherein the alteration in the second CD16a-binding domain is at least one amino acid residue substitution selected from K326W, K326G, L328V, L328R, P329Y, P329K, P329W, and A330P numbered according to the EU index.

47. A DNA, which is the following DNA a) or b):

a) a DNA encoding an amino acid sequence of the first IgG half-molecule according to any one of the above 32 to 39; or

b) a DNA encoding an amino acid sequence of the second IgG half-molecule according to any one of the above 40 to 46.

48. A recombinant vector, comprising at least one of the DNAs a) and b) according to the above 47.

49. A transformant, in which the recombinant vector according to the above 48 is introduced.

50. A method for producing an antibody composition, comprising the following steps of: culturing the transformant according to the above 49 in a culture medium, accumulating at least one of the first IgG half-molecule according to any one of the above 32 to 39 and the second IgG half-molecule according to any one of the above 40 to 46 in a culture and; collecting at least one of the first IgG half-molecule and the second IgG half-molecule from the culture.

51. A kit comprising a first IgG half-molecule and a second IgG half-molecule, wherein

each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,

the first IgG half-molecule includes an antigen-binding domain that binds to a first antigen, and a CD16a-binding activity in the first CD16a-binding domain is attenuated by the alteration,

the second IgG half-molecule includes an antigen-binding domain that binds to a second antigen, and a CD16a-binding activity in the second CD16a-binding domain is attenuated by the alteration, and

an inter-H chain disulfide bond is not formed between the first IgG half-molecule and the second IgG half-molecule.

52. A method for inducing an effector function only for a target cell coexpressing a first antigen and a second antigen using the antibody composition according to any one of the above 1 to 31.

ADVANTAGEOUS EFFECTS OF INVENTION

The antibody composition of the present invention is composed of first and second IgG half-molecules that are two types of IgG half-molecules, which have antigen-binding domains for two types of antigens that are different from each other, and in which a CD16a-binding activity in CD16a-binding domains that are different from each other is attenuated. Accordingly, even if an antibody structure of a homo assembly is constituted by the first IgG half-molecules or the second IgG half-molecules, it cannot bind to CD16a, and therefore cannot exhibit an activity of an antibody. On the other hand, when an antibody structure of a hetero assembly is constituted by the first and second IgG half-molecules, it can bind to CD16a through the second CD16a-binding domain in the first IgG half-molecule and the first CD16a-binding domain in the second IgG half-molecule.

Further, a hinge domain in the first and second IgG half-molecules is altered so as not to form a disulfide bond. Accordingly, an inter-H chain disulfide bond is not formed between the first IgG half-molecule and the second IgG half-molecule. Therefore, when the first and second IgG half-molecules are mixed, it becomes possible to make the first and second IgG half-molecules exist in an equilibrium state of an assembly or half-molecules. Accordingly, the antibody composition of the present invention can more selectively exhibit an effector function against a double-positive cell and damage the cell as compared with a single-positive cell.

BRIEF DESCRIPTION OF DRAWINGS

[Fig. 1] Fig. 1 is a schematic diagram showing the structures of an antibody, VHH-Fc, and scFv-Fc.

[Fig. 2A] Fig. 2A shows a schematic diagram of the structure of a general bispecific antibody.

[Fig. 2B] Fig. 2B shows a schematic diagram of a binding mode by a general bispecific antibody.

[Fig. 3] Fig. 3 shows a schematic diagram of an embodiment of a binding mode by an antibody composition of the present invention.

[Fig. 4] Fig. 4 shows a schematic diagram of an embodiment of the structure of the antibody composition of the present invention.

[Fig. 5] Fig. 5 shows a schematic diagram of a binding mode between normal human IgG1 and CD16a.

[Fig. 6] Fig. 6 shows a schematic diagram of a binding mode between the antibody composition of the present invention and CD16a.

[Fig. 7] Fig. 7 is a schematic diagram showing a candidate site of an amino acid alteration on a format of normal human IgG1.

[Fig. 8] Fig. 8 is a view showing the ADCC activities of human IgG1 anti-CCR6 antibodies in which a CD16a-binding domain was "disrupted".

[Fig. 9] Fig. 9 is a schematic diagram of a monovalent antibody for evaluating an ADCC activity by asymmetrically introducing the alteration into only each of CH2-A and CH2-B.

[Fig. 10] Fig. 10 is a view showing the ADCC activities of CD16a-binding asymmetrically altered monovalent antibodies.

[Fig. 11] Fig. 11 is a schematic diagram of an IgG1 1 14_AA_AAA_D265A (/P329Y)-type IgG half-molecule used in Example 3.

[Fig. 12] Fig. 12 shows the results of evaluating the purification degree by SDS-PAGE of anti-CD4 and CD70 half-molecules.

[Fig. 13] Fig. 13 shows the results of measuring the expression of CD4 antigen and CD70 antigen in CD4 single-positive cells, CD70 single-positive cells, and CD4/CD70 double-positive cells.

[Fig. 14] Fig. 14 is a view showing the ADCC activities of anti-CD4 and CD70 IgG1 and half-molecules against CD4 single-positive cells, CD70 single-positive cells, and CD4/CD70 double-positive cells.

[Fig. 15A] Fig. 15A is a view showing the ADCC activities when adding each half-molecule against CD4/CD70 double-positive cells (TL-Om1), CD70 single-positive cells (MT-1), and CD4 single-positive cells (CD4/EL4).

[Fig. 15B] Fig. 15B is a view showing the ADCC activities when adding each half-molecule against CD4/CD70 double-positive cells (TL-Om1), CD70 single-positive cells (MT-1), and CD4 single-positive cells (CD4/EL4).

[Fig. 15C] Fig. 15C is a view showing the ADCC activities when adding each half-molecule against CD4/CD70 double-positive cells (TL-Om1), CD70 single-positive cells (MT-1), and CD4 single-positive cells (CD4/EL4).

[Fig. 15D] Fig. 15D is a view showing the ADCC activities when adding each half-molecule against CD4/CD70 double-positive cells (TL-Om1), CD70 single-positive cells (MT-1), and CD4 single-positive cells (CD4/EL4).

[Fig. 15E] Fig. 15E is a view showing the ADCC activities when adding each half-molecule against CD4/CD70 double-positive cells (TL-Om1), CD70 single-positive cells (MT-1), and CD4 single-positive cells (CD4/EL4).

[Fig. 15F] Fig. 15F is a view showing the ADCC activities when adding each half-molecule against CD4/CD70 double-positive cells (TL-Om1), CD70 single-positive cells (MT-1), and CD4 single-positive cells (CD4/EL4).

[Fig. 15G] Fig. 15G is a view showing the ADCC activities when adding each half-molecule against CD4/CD70 double-positive cells (TL-Om1), CD70 single-positive cells (MT-1), and CD4 single-positive cells (CD4/EL4).

[Fig. 15H] Fig. 15H is a view showing the ADCC activities when adding each half-molecule, anti-CD4 or CD70 IgG1 against CD4/CD70 double-positive cells (TL-Om1), CD70 single-positive cells (MT-1), and CD4 single-positive cells (CD4/EL4).

## DESCRIPTION OF EMBODIMENTS

### 1. Structure of Antibody Composition

In the present invention, an antibody molecule is also referred to as an immunoglobulin (hereinafter referred to as Ig), and a human antibody is classified into isotypes of IgA1, IgA2, IgD, IgE, IgG1, IgG2, IgG3, IgG4, and IgM according to a difference in molecular structure. IgG1, IgG2, IgG3, and IgG4 having relatively high homology of an amino acid sequence are also collectively referred to as IgG.

An antibody molecule is constituted by polypeptides called heavy chain (hereinafter, referred to as H chain) and light chain (hereinafter, referred to as L chain). An antibody is a tetrameric protein composed of two H chains and two L chains.

Further, the H chain is constituted by each region of an H chain variable region (also referred to as VH) and an H chain constant region (also referred to as CH) from the N terminal side and the L chain is constituted by each region of an L chain variable region (also referred to as VL) and an L chain constant region (also referred to as CL) from the N terminal side. In the CH, $\alpha$, $\delta$, $\varepsilon$, $\gamma$ and $\mu$ chains are known for each subclass. In the CL, $\lambda$ and $\kappa$ are known.

A domain is a functional structural unit that constitutes each polypeptide of an antibody molecule. Further, an Fc region (Fc) in the present invention refers to a partial sequence and a partial structure of an H chain constant region

composed of a hinge domain, a CH2 domain, and a CH3 domain.

The CH is further constituted by each domain of a CH1 domain, a hinge domain, a CH2 domain, and a CH3 domain from the N terminal side. The CH1 domain, the hinge domain, the CH2 domain, the CH3 domain, and the Fc in the present invention can be specified by the amino acid residue number from the N terminus according to the EU index [Kabat et al., Sequences of Proteins of Immunological Interest, US Dept. Health and Human Services (1991)].

Specifically, CH1 is specified as an amino acid sequence at positions 118 to 215 according to the EU index, the hinge is specified as an amino acid sequence at positions 216 to 230 according to the EU index, CH2 is specified as an amino acid sequence at positions 231 to 340 according to the EU index, and CH3 is specified as an amino acid sequence at positions 341 to 447 according to the EU index.

As the IgG in the present invention, an artificial subspecies of an altered molecule that includes at least an antigen-binding domain and Fc and has a similar function to IgG is also included. Specifically, an altered molecule obtained by substitution, deletion or addition, or modification of an amino acid of IgG, and further an adduct of a polypeptide or a domain, and the like are included. Further, those obtained by substitution of a part or the whole of an antigen-binding site composed of VH, VL, or Fab of IgG with another antigen-binding domain are also included. Specifically, single-chain Fv (scFv)-Fc and VHH-Fc shown in Fig. 1, and the like are also included (Brinkmann U et al., MABS 2017, 9: 182-212; Fernandes CFC et al., Frontiers in Immunology 2017, 8: 653).

As the antibody in the present invention, other than a monoclonal antibody obtained from a hybridoma, a recombinant antibody produced by a genetic engineering technique is also included. As the recombinant antibody, a chimeric antibody obtained by binding a human antibody constant region to a non-human antibody variable region, a humanized antibody, and a human antibody produced using a human antibody-producing animal, or the like are included.

The chimeric antibody can be produced by obtaining cDNAs encoding VH and VL from a hybridoma that produces a monoclonal antibody and is derived from a non-human animal cell, inserting each of the cDNAs into an expression vector for an animal cell having DNAs encoding CH and CL of a human antibody thereby constructing a human chimeric antibody expression vector, and introducing the vector into an animal cell and expressing the antibody.

The humanized antibody refers to an antibody produced by inserting complementarity determining regions (hereinafter abbreviated as CDRs) of the H chain and the L chain of a variable region of a non-human antibody into a framework region (hereinafter abbreviated as FR) of a human antibody variable region.

The humanized antibody (or a CDR-grafted antibody) can be produced by the following method. A cDNA that encodes the amino acid sequence of a CDR of VH of a non-human animal antibody and the amino acid sequence of VH composed of the amino acid sequence of FR of VH of an arbitrary human antibody, and a cDNA that encodes the amino acid sequence of a CDR of VL of a non-human animal antibody and the amino acid sequence of VL composed of the amino acid sequence of FR of VL of an arbitrary human antibody are constructed. Each of the cDNAs is inserted into an expression vector for an animal cell having DNAs that encode CH and CL of a human antibody, thereby constructing a humanized antibody expression vector, and introducing the vector into an animal cell and expressing the antibody.

The human antibody originally refers to an antibody that can be naturally present in the human body or an antibody composed of an amino acid sequence encoded by a human gene, but also includes antibodies that are obtained from human antibody phage libraries, cloning of immortalized human peripheral blood lymphocytes, or human antibody-producing transgenic animals, which are produced by recent advancement of genetic engineering, cellular engineering, and developmental engineering technologies, and the like.

The human antibody can be obtained by immunizing a mouse having a human immunoglobulin gene (Tomizuka K. et al., Proc Natl Acad Sci USA. 97, 722-7, 2000) with a desired antigen. Further, the human antibody can be obtained without immunization by selecting a human antibody having a desired binding activity using a phage display library obtained by amplifying antibody genes from human-derived B cells (Winter G. et al., Annu Rev Immunol. 12: 433-55, 1994).

Further, the human antibody can be obtained by producing cells that produce a human antibody having a desired binding activity by immortalizing human B cells using EB virus (Rosen A. et al., Nature 267, 52-54, 1977).

As for the antibody that is present in the human body, for example, lymphocytes isolated from human peripheral blood are infected with EB virus or the like so as to be immortalized, followed by cloning, whereby lymphocytes that produce the antibody can be cultured, and the antibody can be purified from the culture.

The human antibody phage library is a library of phages in which an antibody fragment such as Fab or scFv is expressed on the surface thereof by inserting an antibody gene prepared from human B cells into a phage gene. It is possible to collect phages that express an antibody fragment having a desired antigen-binding activity from the library using a binding activity to a substrate onto which an antigen is immobilized as an index. The antibody fragment can further also be converted into a human antibody molecule composed of two complete H chains and two complete L chains using a genetic engineering technique.

The human antibody-producing transgenic animal refers to an animal in which a human antibody gene is incorporated into the chromosome of a host animal. Specifically, the human antibody-producing transgenic animal can be produced by introducing a human antibody gene into mouse ES cells, implanting the ES cells to an early embryo of another mouse and then allowing the embryo to develop.

As for a method for producing a human antibody from a human antibody-producing transgenic animal, a human antibody-producing hybridoma is obtained by a general method for producing a hybridoma performed for mammals other than humans, and cultured, whereby a human antibody can be produced and accumulated in the culture.

The amino acid sequences of VH and VL may be any of the amino acid sequences of VH and VL of a human antibody, the amino acid sequences of VH and VL of a non-human animal antibody, the amino acid sequences of a humanized antibody in which a CDR of a non-human animal antibody is implanted to a framework of a human antibody, and the amino acid sequences of VH and VL derived from a human antibody.

Specifically, the amino acid sequences of VH and VL of a non-human animal antibody, a humanized antibody, and a human antibody that a hybridoma or an antibody-producing cell produces, and the like are exemplified.

The amino acid sequence of CL may be either of the amino acid sequence of a human antibody, the amino acid sequence of a non-human animal antibody, but is preferably the amino acid sequence of Cκ or Cλ of a human antibody.

The CH may be any as long as it belongs to an immunoglobulin, but preferably, any of yl (IgG1), γ2 (IgG2), y3 (IgG3), and γ4 (IgG4) subclasses belonging to human IgG class can be used.

The "antigen-binding domain" may be a binding protein recombined by using a binding domain of a known binding molecule such as an antibody, a ligand, a receptor, or the like, and specific examples include a recombinant protein including a CDR of an antibody that binds to each antigen, an antibody variable region including a CDR, a recombinant protein including an antibody variable region and a binding domain of a ligand that binds to each antigen, and the like. Among these, the antigen-binding domain is preferably an antibody variable region in the present invention.

The antibody composition of the present invention is an antibody composition against a first antigen and a second antigen that are different from each other, composed of a first IgG half-molecule and a second IgG half-molecule, and has the following properties 1) to 4).

1) Each of the first and second IgG half-molecules is composed of one L chain and one H chain, the H chain includes an H chain variable region, a hinge domain altered so as not to form a disulfide bond by substitution or deletion of a part or the whole or modification, and CH1 to CH3 domains, and has alteration in either of first and second CD16a-binding domains that are different from each other in the CH2 domain.

2) The first IgG half-molecule includes an antigen-binding domain that binds to the first antigen, and a CD 16a-binding activity in the first CD 16a-binding domain is attenuated by the alteration.

3) The second IgG half-molecule includes an antigen-binding domain that binds to the second antigen, and a CD16a-binding activity in the second CD16a-binding domain is attenuated by the alteration.

4) An inter-H chain disulfide bond is not formed between the first IgG half-molecule and the second IgG half-molecule.

The "antibody composition against a first antigen and a second antigen that are different from each other" denotes a composition containing an IgG half-molecule against a first antigen and an IgG half-molecule against a second antigen.

As a possible state of the IgG half-molecule in the antibody composition of the present invention, for example, a half-molecule and a dimer are exemplified. Examples of the half-molecule include the first IgG half-molecule and the second IgG half-molecule. Examples of the dimer include a dimer of the first IgG half-molecule, a dimer of the second IgG half-molecule, and a dimer of the first IgG half-molecule and the second IgG half-molecule.

The "antibody composition against a first antigen and a second antigen that are different from each other" of the present invention also includes an antibody composition including an IgG half-molecule against a first antigenic determinant (epitope) and an IgG half-molecule against a second antigenic determinant (epitope) in the same antigen.

The "IgG half-molecule" is a dimeric protein composed of one L chain and one H chain, and the H chain includes an H chain variable region and CH1 to CH3 domains. In the CH2 domain of the H chain of the IgG half-molecule, two CD16a-binding domains (first and second CD16a-binding domains) that are different from each other are present.

Further, the "IgG half-molecule" also includes a half-molecule of an artificial subspecies of altered molecule that includes an antigen-binding domain and Fc and has a similar function to IgG. Specifically, a half-molecule of an altered molecule obtained by substitution, deletion or addition, or modification of an amino acid of IgG, and further a half-molecule of an adduct of a polypeptide or a domain, and the like are included. Further, half-molecules of those obtained by substitution of a part or the whole of an antigen-binding site composed of VH, VL, or Fab of IgG with another antigen-binding domain, specifically, half-molecules of single-chain Fv (scFv)-Fc and VHH-Fc shown in Fig. 1, and the like are also included.

The "CD16a-binding domain" refers to a domain that is present in Fc of IgG and binds to CD16a. The CD16a-binding domain on Fc of IgG1 is present at two sites due to the point symmetry of the structure of Fc, and in two CH2 domains constituting Fc, contact with CD16a is made in regions that are different from each other.

Examples of the first CD16a-binding domain include a domain including at least one selected from amino acid residues at position 235, position 236, position 237, position 238, position 239, position 265, position 266, position 267,

position 268, position 269, position 294, position 295, position 296, position 297, position 298, position 299, position 301, position 325, position 327, and position 332 numbered according to the EU index.

When the immunoglobulin subclass of the CH2 domain is IgG1, examples of the first CD16a-binding domain include a domain including at least one selected from amino acid residues of Leu at position 235, Gly at position 236, Gly at position 237, Pro at position 238, Ser at position 239, Asp at position 265, Val at position 266, Ser at position 267, His at position 268, Glu at position 269, Glu at position 294, Gln at position 295, Tyr at position 296, Asn at position 297, Ser at position 298, Thr at position 299, Arg at position 301, Asn at position 325, Ala at position 327, and Ile at position 332 numbered according to the EU index.

Examples of the second CD16a-binding domain include a domain including at least one selected from amino acid residues at position 235, position 236, position 237, position 326, position 327, position 328, position 329, and position 330 numbered according to the EU index.

When the immunoglobulin subclass of the CH2 domain is IgG1, examples of the second CD16a-binding domain include a domain including at least one selected from amino acid residues of Leu at position 235, Gly at position 236, Gly at position 237, Lys at position 326, Ala at position 327, Leu at position 328, Pro at position 329, and Ala at position 330 numbered according to the EU index.

The "CD16a-binding activity" refers to an activity of binding of Fc of IgG to CD16a. The CD16a-binding activity of the IgG half-molecule can be confirmed by combining two molecules of the IgG half-molecule to form IgG, and producing a recombinant CD16a protein, and measuring the binding activity (US Patent Application Publication No. 2004/0259150). Further, the confirmation can also be performed by imparting the below-mentioned effector activity (ADCC activity or the like) to Fc of the IgG half-molecule, and forming IgG in which two molecules of the IgG half-molecule are combined, and then measuring the effector activity of the IgG.

The state where "a CD16a-binding activity in the CD16a-binding domain is attenuated" refers to a state where the effector activity (ADCC activity or the like) of IgG obtained by combining two molecules of the IgG half-molecule to which alteration for attenuating a CD16a-binding activity in the first or second CD16a-binding domain in Fc is added is decreased as compared with the effector activity of IgG obtained by combining two molecules of the IgG half-molecule before the alteration.

A CD16a-binding activity in the CD16a-binding domain is attenuated by alteration in the CD16a-binding domain. Examples of the alteration include substitution, deletion or addition, or modification of an amino acid residue.

Examples of the alteration for attenuating a CD16a-binding activity in the first CD16a-binding domain include substitution of at least one amino acid residue selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index, and preferred is substitution of at least one amino acid residue selected from amino acid residues at position 235, position 238, position 239, position 265, position 266, position 267, position 268, position 269, position 294, position 295, position 296, position 297, position 298, position 299, position 301, position 325, and position 332, and more preferred is substitution of at least one amino acid residue selected from amino acid residues at position 235, position 238, position 239, position 265, position 267, position 269, position 296, position 298, position 299, and position 327. As a combination of amino acid residue substitutions, for example, amino acid residue substitutions at a combination of position 238 and position 265 (hereinafter such a combination is represented by position 238/position 265, or the like), at position 238/position 267, at position 265/position 267, or at position 238/position 265/position 267 is exemplified.

When the immunoglobulin subclass of the CH2 domain is IgG1, examples of the alteration for attenuating a CD16a-binding activity in the first CD16a-binding domain include substitution of at least one amino acid residue selected from Leu at position 235, Gly at position 236, Gly at position 237, Pro at position 238, Ser at position 239, Asp at position 265, Val at position 266, Ser at position 267, His at position 268, Glu at position 269, Glu at position 294, Gln at position 295, Tyr at position 296, Asn at position 297, Ser at position 298, Thr at position 299, Arg at position 301, Asn at position 325, Ala at position 327, and Ile at position 332 numbered according to the EU index, and preferred is substitution of at least one amino acid residue selected from Leu at position 235, Pro at position 238, Ser at position 239, Asp at position 265, Val at position 266, Ser at position 267, His at position 268, Glu at position 269, Glu at position 294, Gln at position 295, Tyr at position 296, Asn at position 297, Ser at position 298, Thr at position 299, Arg at position 301, Asn at position 325, and Ile at position 332, and more preferred is substitution of at least one amino acid residue selected from Leu at position 235, Pro at position 238, Ser at position 239, Asp at position 265, Ser at position 267, Glu at position 269, Tyr at position 296, Ser at position 298, Thr at position 299, and Ala at position 327. Specifically, for example, at least one amino acid residue substitution selected from L235R, P238A, S239R, D265A, D265N, D265E, S267L, S267K, E269P, Y296P, S298E, T299A, and A327I is exemplified.

Examples of the alteration for attenuating a CD16a-binding activity in the second CD16a-binding domain include substitution of at least one amino acid residue selected from at least one selected from amino acid residues at position 235, position 236, position 237, position 326, position 327, position 328, position 329, and position 330 numbered

according to the EU index, and preferred is substitution of at least one amino acid residue selected from amino acid residues at position 326, position 328, position 329, and position 330, and more preferred is substitution of at least one amino acid residue selected from amino acid residues at position 326, position 328, and position 329. As a combination of amino acid residue substitutions, for example, amino acid residue substitutions at position 326/position 328, at position 326/position 329, at position 328/position 329, and at position 326/position 328/position 329 are exemplified.

When the immunoglobulin subclass of the CH2 domain is IgG1, examples of the alteration for attenuating a CD16a-binding activity in the second CD16a-binding domain include substitution of at least one amino acid residue selected from Leu at position 235, Gly at position 236, Gly at position 237, Lys at position 326, Ala at position 327, Leu at position 328, Pro at position 329, and Ala at position 330 numbered according to the EU index, and preferred is substitution of at least one amino acid residue selected from Lys at position 326, Leu at position 328, Pro at position 329, and Ala at position 330, and more preferred is substitution of at least one amino acid residue selected from Lys at position 326, Leu at position 328, and Pro at position 329. Specifically, for example, at least one amino acid residue substitution selected from K326W, K326G, L328V, L328R, P329Y, P329K, P329W, and A330P is exemplified.

A combination of the alteration for attenuating a CD16a-binding activity in the CD16a-binding domain of the first IgG half-molecule and the alteration for attenuating a CD16a-binding activity in the CD16a-binding domain of the second IgG half-molecule is not particularly limited as long as asymmetric alteration is brought about, and the above-mentioned alterations can be appropriately combined. Specific examples include the following combinations of amino acid residue substitutions.

- amino acid residue substitutions at position 265 in the first IgG half-molecule and at position 329 in the second IgG half-molecule
- amino acid residue substitutions at position 329 in the first IgG half-molecule and at position 265 in the second IgG half-molecule
- amino acid residue substitutions at position 238/position 267 in the first IgG half-molecule and at position 329 in the second IgG half-molecule
- amino acid residue substitutions at position 329 in the first IgG half-molecule and at position 238/position 267 in the second IgG half-molecule

The combination of the alteration for attenuating a CD16a-binding activity in the CD16a-binding domain of the first IgG half-molecule (first alteration) and the alteration for attenuating a CD16a-binding activity in the CD16a-binding domain of the second IgG half-molecule (second alteration) is preferably combinations shown in the following Table 1, and more preferably a combination of the first alteration and the second alteration as follows: S267K and P329Y, Y296P and P329Y, S298E and P329Y, D265A and P329Y, or S239R and P329Y.

[Table 1]

| First alteration | Second alteration |
|---|---|
| D265A | P329Y |
| P238A/S267L | P329Y |
| S239R | K326G |
| S239R | L328R |
| D265A | L328R |
| D265E | L328R |
| S267K | L328R |
| E269P | L328R |
| Y296P | L328R |
| S298E | L328R |
| T299A | L328R |
| S239R | P329Y |
| D265E | P329Y |
| S267K | P329Y |

(continued)

| First alteration | Second alteration |
|---|---|
| Y296P | P329Y |
| S298E | P329Y |
| T299A | P329Y |
| L235R | P329Y |
| A327I | P329Y |
| S239R | P329Y |
| D265A | P329Y |
| D265E | P329Y |
| S267K | P329Y |
| Y296P | P329Y |
| S298E | P329Y |
| T299A | P329Y |
| S239R | P329W |
| D265A | P329W |
| D265N | P329W |
| D265E | P329W |
| S267K | P329W |
| E269P | P329W |
| Y296P | P329W |
| S298E | P329W |
| T299A | P329W |
| L235R | P329W |
| A327I | P329W |
| S239R | A330P |
| D265A | A330P |
| D265E | A330P |
| S267K | A330P |
| E269P | A330P |
| Y296P | A330P |
| S298E | A330P |
| T299A | A330P |
| L235R | A330P |
| A327I | A330P |

An amino acid residue after substitution described above may be a mutually substitutable amino acid. Hereinafter, examples of the mutually substitutable amino acid are shown. Amino acids included in the same group can be mutually substitutable.

group A: leucine, isoleucine, norleucine, valine, norvaline, alanine, 2-aminobutanoic acid, methionine, O-methylserine, t-butyl glycine, t-butyl alanine, and cyclohexylalanine

group B: aspartic acid, glutamic acid, isoaspartic acid, isoglutamic acid, 2-aminoadipic acid, and 2-aminosuberic acid
group C: asparagine and glutamine
group D: lysine, arginine, ornithine, 2,4-diaminobutanoic acid, and 2,3-diaminopropionic acid
group E: proline, 3-hydroxyproline, and 4-hydroxyproline
group F: serine, threonine, and homoserine
group G: phenylalanine and tyrosine

The above-mentioned amino acids to be substituted may be either a natural type or an unnatural type. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine, and the like. Examples of the unnatural amino acid include various amino acids having an amino group and a carboxyl group, however, preferably, derivatives of various types of natural amino acids are desired. Many unnatural amino acids are available from respective reagent companies (Sigma-Aldrich Co. LLC, TCI Co., Ltd.). With respect to the unnatural amino acids, there are many disclosures in literature (Chem. Today 2003, 65; Curr Opin Chem Biol. 2000, 6, 645).

In the first IgG half-molecule, a CD16a-binding activity in the first CD16a-binding domain is attenuated by the alteration, and also in the second IgG half-molecule, a CD16a-binding activity in the second CD16a-binding domain is attenuated by the alteration. Accordingly, even if an antibody structure of a homo assembly is constituted by the first IgG half-molecules or the second IgG half-molecules, it cannot bind to CD16a, and therefore cannot exhibit an activity of an antibody.

On the other hand, when an antibody structure of a hetero assembly is constituted by the first and second IgG half-molecules, it can bind to CD16a through the second CD16a-binding domain in the first IgG half-molecule and the first CD16a-binding domain in the second IgG half-molecule.

The first and second IgG half-molecules have antigen-binding domains that are different from each other. Therefore, by constituting an antibody structure of a hetero assembly composed of the first and second IgG half-molecules, the assembly becomes an antibody composition which CD16a binds to and thus can selectively exhibit an ADCC activity only when it binds to a target cell expressing two types of antigens that are different from each other on the same cell.

A hinge domain in the first and second IgG half-molecules is altered so as not to form a disulfide bond by substitution or deletion of a part or the whole thereof or modification. Accordingly, an inter-H chain disulfide bond is not formed between the first IgG half-molecule and the second IgG half-molecule. Therefore, by the coexistence of the first and second IgG half-molecules, it becomes possible to make the first and second IgG half-molecules exist in an equilibrium state of an assembly or half-molecules, so that high selectivity for a double-positive cell can be exhibited.

As the substitution of a part of a hinge domain so as not to form an inter-H chain disulfide bond between the first IgG half-molecule and the second IgG half-molecule, for example, substitution of at least one of the amino acid residues at position 226 and position 229 numbered according to the EU index is exemplified.

In order to make the first and second IgG half-molecules exist in the above-mentioned equilibrium state, it is preferred that the CH3 domain of the H chain has a weaker interaction between CH3 domains as compared with an interaction between CH3 domains belonging to an immunoglobulin subclass of the L chain, the H chain variable region and the CH1 domain and the CH2 domain of the H chain in the first and second IgG half-molecules.

Specifically, for example, it is preferred that the CH3 domain of the H chain in the first and second IgG half-molecules has a weaker inter-CH3 domain interaction than an inter-CH3 domain interaction of the IgG1 subclass.

Examples of the CH3 domain having a weaker inter-CH3 domain interaction than an inter-CH3 domain interaction of the IgG1 subclass include a CH3 domain of IgG1 and a CH3 domain of IgG4, in which the inter-CH3 domain interaction has been attenuated by alteration of an amino acid (substitution, deletion or addition, or modification or the like of an amino acid).

2. Control of Effector Activity of Antibody Composition

The antibody composition of the present invention can also impart an effector activity dependent on Fc in the first and second IgG half-molecules. The effector activity of the antibody composition can be controlled by various methods.

For example, it is preferred to further enhance a CD16a-binding activity by further including at least one amino acid residue substitution for enhancing a CD16a-binding activity in the CH2 domain in the first and second IgG half-molecules. Accordingly, the effector activity of the antibody composition can be enhanced.

The amino acid residue substitution for enhancing the CD16a-binding activity of the antibody composition is preferably an amino acid residue substitution in a region different from a region altered for attenuating a CD16a-binding activity in the first and second IgG half-molecules.

The effector activity refers to an antibody-dependent activity caused via Fc of an antibody, and an ADCC activity,

a CDC activity, or an antibody-dependent cellular phagocytosis activity (ADCP activity) by phagocytes such as macrophages or dendritic cells, and the like are known. In the present invention, an ADCC activity and a CDC activity can be measured using a known measuring method [Cancer Immunol. Immunother., 36, 373 (1993)].

The ADCC activity refers to an activity in which an antibody binding to an antigen on a target cell binds to an Fc receptor of an immunocyte via Fc of the antibody so as to activate the immunocyte (a natural killer cell or the like) and damage the target cell.

The Fc receptor (hereinafter sometimes referred to as FcR) is a receptor that binds to Fc of an antibody, and the binding of the antibody induces various effector activities.

The FcR corresponds to the subclass of an antibody, and IgG, IgE, IgA, and IgM bind specifically to FcγR, FcεR, FcαR, and FcμR, respectively. Further, in the FcγR, there are subtypes of FcyRI (CD64), FcyRII (CD32), and FcyRIII(CD16), and the subtypes have isoforms of FcγRIA, FcyRIB, FcγRIC, FcyRIIA, FcyRIIB, FcyRIIC, FcγRIIIA (CD16a), and FcγRIIIB, respectively. The different types of FcyRs are present on different cells [Annu. Rev. Immunol. 9: 457-492 (1991)].

In humans, FcyRIIIB is expressed specifically in neutrophils, and FcyRIIIA is expressed in monocytes, natural killer cells (NK cells), and some T cells. Binding of the antibody via FcyRIIIA induces an NK cell-dependent ADCC activity.

The CDC activity refers to an activity in which an antibody binding to an antigen on a target cell activates a series of cascades (complement activation pathways) composed of complement-related protein groups in the blood, and therefore damages the target cell. In addition, a protein fragment generated by the activation of the complement can induce the migration and activation of an immunocyte.

The cascade of CDC activity starts by forming a C1 complex through binding of C1q having a binding domain for Fc of an antibody to Fc, and its binding to C1r and C1s that are two serine proteases.

Examples of a method for controlling the effector activity of the antibody composition of the present invention include a method as described below.

For example, the effector activity of the antibody composition can be controlled by a method for controlling the amount of fucose (also referred to as core fucose) that is α1,6-linked to N-acetylglucosamine (GlcNAc) present at the reducing end of an N-linked complex sugar chain (hereinafter sometimes simply abbreviated as complex sugar chain) that is bound to Asn at position 297 according to the EU index using the amino acid sequence of Fc of the IgG1 subclass in the first and second IgG half-molecules (WO 2005/035586, WO 2002/31140, or WO 00/61739), or by substituting an amino acid residue of Fc of the antibody.

1) Control of Effector Activity by Sugar Chain Alteration

The effector activity of the antibody composition can be increased or decreased by controlling the content of fucose to be added to N-acetylglucosamine at the reducing end of the complex sugar chain bound to Fc of the first and second IgG half-molecules.

As for a method for decreasing the content of fucose to be added to the N-linked complex sugar chain bound to Fc of the IgG half-molecule, the IgG half-molecule in which fucose is not bound can be obtained by expressing the IgG half-molecule using an α1,6-fucosyltransferase (FUT8) gene-deficient CHO cell. The antibody composition composed of the IgG half-molecule in which fucose is not bound has a high ADCC activity.

On the other hand, as for a method for increasing the content of fucose to be added to the N-linked complex sugar chain bound to Fc of the IgG half-molecule, the IgG half-molecule in which fucose is bound can be obtained by expressing the IgG half-molecule using a host cell into which an α1,6-fucosyltransferase gene has been introduced. The antibody composition composed of the IgG half-molecule in which fucose is bound has a lower ADCC activity than the antibody composition composed of the IgG half-molecule in which fucose is not bound.

In Fc of the IgG half-molecule, the N-linked sugar chain is bound to the Asn residue at position 297 according to the EU index, however, it is not known that the sugar chain is bound to other Asn residues of Fc. Therefore, generally, two N-glycoside-linked sugar chains are bound per molecule of the antibody composition.

As the N-linked sugar chain, a high mannose type, a complex type, and a hybrid type are known, and a high ADCC activity can be obtained by any N-linked sugar chain as long as it is a sugar chain to which fucose is not bound as compared with a sugar chain to which fucose is bound.

As the complex sugar chain to be bound to Fc of the IgG half-molecule, a sugar chain in which one or more N-acetylglucosamine (GlcNAc) residues or galactose-N-acetylglucosamine (hereinafter referred to as Gal-GlcNAc) residues are α1,2-linked or α1,4-linked to mannose (Man) at the non-reducing end side of a core structure (trimannosyl core structure) is exemplified.

Further, a complex type sugar chain having sialic acid, bisecting N-acetylglucosamine (hereinafter referred to as bisecting GlcNAc), or the like at the non-reducing end side of Gal-GlcNAc can be exemplified.

In the present invention, the core fucose or the α1,6-fucose refers to a sugar chain structure in which the 6-position of N-acetylglucosamine (hereinafter sometimes referred to as GlcNAc) at the reducing end of the N-glycoside-linked complex sugar chain and the 1-position of fucose (hereinafter sometimes referred to as Fuc) are α-linked. Further, the sugar chain in which fucose is not bound to N-acetylglucosamine at the reducing end of the N-glycoside-linked complex sugar chain is simply referred to as a sugar chain having no fucose or no core fucose.

Further, in the present invention, the core structure or the trimannosyl core structure refers to a Manα1-6(Manα1-3)Manβ1-4GlcNAcβ1-4G1cNAc structure.

As the sugar chain bound to the IgG half-molecule, a double-stranded N-glycoside-linked complex sugar chain (also referred to as a biantennary complex sugar chain) is represented by the following chemical formula.

[Chem. 1]

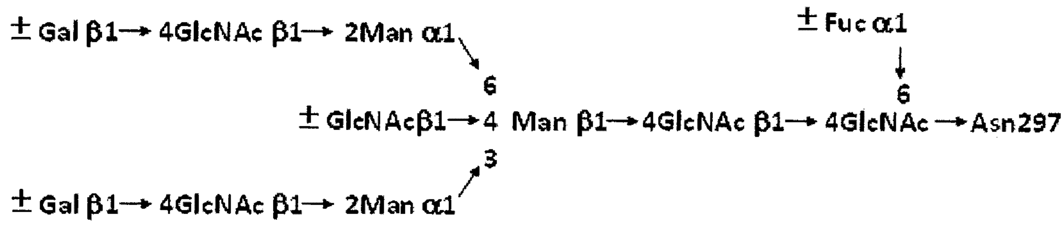

The first and second IgG half-molecules preferably have Fc in which the complex type sugar chain is bound to Asn at position 297 according to the EU index. They may have a single sugar chain structure or a plurality of different sugar chain structures as long as they have the above-mentioned sugar chain structure. Specifically, an antibody composition in which the ratio of the sugar chain in which fucose is not bound to N-acetylglucosamine at the reducing end of the sugar chain (sugar chain having no core fucose) among all the N-glycoside-linked sugar chains bound to Fc in the first and second IgG half-molecules is 20% or more is exemplified.

The ratio of the sugar chain having no core fucose includes any ratio as long as the ADCC activity of the antibody composition is increased, but a ratio of preferably 20% or more, more preferably 51% to 100%, further more preferably 80% to 100%, particularly preferably 90%, 91%, 92%, 93%, 94%, 95%, 96%, 97%, 98%, or 99%, and most preferably 100% can be exemplified.

The ratio of the sugar chain having no core fucose being 50% includes, for example, both an antibody composition containing a molecule in which fucose is not bound to one sugar chain of the N-glycoside-linked sugar chains bound to the first and second IgG half-molecules at 100%, and an antibody composition containing a molecule in which fucose is not bound to both sugar chains of the N-glycoside-linked sugar chains bound to the first and second IgG half-molecules at 50% and also containing a molecule in which fucose is bound to both sugar chains of the N-glycoside-linked sugar chains bound to the first and second IgG half-molecules at 50%.

In the present invention, as the sugar chain having no fucose, the structure of the sugar chain at the non-reducing end may be any as long as fucose is not bound to N-acetylglucosamine on the reducing end side in the chemical formula shown above.

In the present invention, the state where fucose is not bound to N-acetylglucosamine at the reducing end of the sugar chain (having no core fucose) refers to a state where fucose is not substantially bound thereto. The IgG half-molecule in which fucose is not substantially bound specifically refers to a case where it is an IgG half-molecule to such an extent that fucose cannot be substantially detected in the below-mentioned sugar chain analysis. The extent that fucose cannot be substantially detected refers to that it is the detection limit or less in the measurement. The antibody composition composed of the first and second IgG half-molecules having no core fucose in all the sugar chains has the highest ADCC activity.

The ratio of the IgG half-molecule including a sugar chain having no fucose in the IgG half-molecule having Fc to which the N-glycoside-linked complex sugar chain is bound can be determined by releasing the sugar chains using a known method such as hydrazinolysis or enzyme digestion [Biochemical Experimentation Methods 23-Method for Studying Glycoprotein Sugar Chain (Japan Scientific Societies Press) edited by Reiko Takahashi (1989)] from the IgG half-molecule, subjecting the released sugar chains to fluorescence labeling or radioisotope labeling, and separating the labeled sugar chains by chromatography.

In addition, the ratio of the IgG half-molecule in which a sugar chain having no fucose is bound included in the IgG half-molecule containing Fc in which the complex sugar chain is bound can be determined by analyzing the released sugar chains using an HPAED-PAD method (J. Liq. Chromatogr., 6, 1577, 1983).

2) Control of Effector Activity by Amino Acid Residue Substitution

The antibody composition of the present invention can increase or decrease an ADCC activity, an ADCP activity, and a CDC activity by changing the antibody subclass of Fc or by amino acid residue substitution in Fc in the first IgG half-molecule and the second IgG half-molecule.

The IgG1 subclass antibody is known to have the highest ADCC activity and CDC activity in the IgG subclasses, and the immunoglobulin subclass of the CH2 domain is preferably IgG1.

As the amino acid residue substitution in Fc, for example, by using the amino acid sequence of Fc described in US Patent Application Publication No. 2007/0148165, the CDC activity of the antibody can be increased. Further, by performing an amino acid residue substitution described in US Patent No. 6,737,056, US Patent No. 7,297,775 and US Patent No. 7,317,091, the ADCC activity or the CDC activity of the antibody composition can be increased or decreased.

Specific examples of the amino acid residue substitution for enhancing the ADCC activity include P247I, A339D, F243L, R292P, Y300L, P396L, T393A, H433P, S239D, S298A, A330L, I332E, E333A, K334A, and the like. On the other hand, specific examples of the amino acid residue substitution for decreasing an ADCC activity include L235E, P238A, N297A, K322A, P331S, and the like.

As a specific amino acid residue substitution for increasing the CDC activity, at least one amino acid residue substitution selected from K326A, S267E, H268F, S324T, K274Q, N276K, Y296F, Y300F, K326W, K326Y, E333A, E333S, A339T, D356E, L358M, N384S, K392N, T394F, T394Y, V397M, and V422I is exemplified.

The CDC activity can also be increased by combining any two or more amino acid residue substitutions, and the number of amino acid residues to be substituted can be increased according to the purpose. As the amino acid residue substitution for increasing the CDC activity, preferably at least one amino acid residue substitution selected from N276K, A339T, T394F, and T394Y, amino acid residue substitutions of N276K and A339T, amino acid residue substitutions of K274Q, N276K, Y296F, Y300F, A339T, D356E, L358M, N384S, V397M, and V422I, and the like are exemplified. On the other hand, as specific amino acid residue substitutions for decreasing the CDC activity, L235E, N297A, K322A, P329A, P331S, and the like are exemplified.

In addition, the blood half-life can be extended by introducing an amino acid mutation such as T250Q, M428L, M252Y, S254T, or T256E into Fc of the human IgG1 subclass. Moreover, cellular cytotoxicity such as an ADCC activity, an ADCP activity, or a CDC activity can be decreased using Fc from which an N-linked sugar chain has been removed, Fc of human IgG2 or IgG4 subclass, chimeric Fc of IgG2 and IgG4, or the like by introducing an amino acid mutation at position N297.

3. Method for Producing Antibody Composition

As the method for producing an antibody composition of the present invention, a production method including the following steps 1 to 3 is exemplified.

Step 1: a step of introducing a recombinant vector containing a DNA encoding the amino acid sequence of the IgG half-molecule (hereinafter also abbreviated as a recombinant vector for expression of the IgG half-molecule) into a cell, thereby obtaining a transformant
Step 2: a step of culturing the transformant obtained in the step 1 to accumulate the IgG half-molecule in a culture, and collecting the IgG half-molecule from the culture
Step 3: a step of obtaining an antibody composition composed of the IgG half-molecule collected in the step 2

Hereinafter, the respective steps will be described.

[Step 1]

The step 1 is a step of introducing a recombinant vector containing a DNA encoding the amino acid sequence of at least one of the first and second IgG half-molecules into a cell, thereby obtaining a transformant.

The step (1) specifically includes the following steps (1-1) to (1-3).

(1-1) a step of attenuating a CD16a-binding activity in the first CD16a-binding domain in the first IgG half-molecule by alteration
(1-2) a step of attenuating a CD16a-binding activity in the second CD16a-binding domain in the second IgG half-molecule by alteration
(1-3) a step of performing the alteration so as not to form an inter-H chain disulfide bond by performing substitution or deletion of a part or the whole or modification in a hinge domain of the first and second IgG half-molecules.

As for the step (1-1), for example, in the production of a recombinant vector for expression of the first IgG half-molecule, a step of substituting at least one amino acid residue selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index that is appropriately performed according to the subclass of the CH2 domain is exemplified.
As for the step (1-2), for example, in the production of a recombinant vector for expression of the second IgG half-molecule, a step of substituting at least one amino acid residue selected from amino acid residues at position 235, position 236, position 237, position 326, position 327, position 328, position 329, and position 330 numbered according to the EU index that is appropriately performed according to the subclass of the CH2 domain is exemplified.
As for the step (1-3), for example, in the production of a recombinant vector for expression of the IgG half-molecule, a step of adding substitution of at least one of the amino acid residues at position 226 and position 229 numbered according to the EU index that is appropriately performed according to the subclass of the hinge domain is exemplified.
The IgG half-molecule can be obtained, for example, by expressing it in a transformant in the following manner using a method described in Molecular Cloning, Second Edition, Current Protocols in Molecular Biology, Antibodies, A Laboratory Manual, Cold Spring Harbor Laboratory, 1988, Monoclonal Antibodies: principles and practice, Third Edition, Acad. Press, 1993, Antibody Engineering, A Practical Approach, IRL Press at Oxford University Press, 1996, or the like.

(1) Construction of Recombinant Vector for Expression of IgG Half-Molecule

The recombinant vector for expression of the IgG half-molecule is an expression vector for animal cells into which a gene encoding the amino acid sequence of the IgG half-molecule constituting the antibody composition of the present invention is incorporated.
The recombinant vector can be constructed by cloning a DNA encoding the amino acid sequence of the IgG half-molecule into an expression vector for animal cells.
As for the DNA, the total DNA may be synthesized or synthesis by a polymerase chain reaction (PCR method) is also possible (Molecular Cloning, Second Edition). Further, it is also possible to produce the gene encoding the IgG half-molecule by combining a plurality of such methods.
When an animal cell is used as a host, as the expression vector, any vector can be used as long as it can exhibit its function in the animal cell. Examples thereof include pcDNAI, pCDM8 (manufactured by Funakoshi Co., Ltd.), pAGE107 [JP-A-H3-22979; and Cytotechnology, 3, 133 (1990)], pAS3-3 (JP-A-H2-227075), pCDM8 [Nature, 329, 840 (1987)], pcDNAI/Amp (manufactured by Invitrogen, Inc.), pcDNA3.1 (manufactured by Invitrogen, Inc.), pREP4 (manufactured by Invitrogen, Inc.), pAGE103 [J. Biochemistry, 101, 1307 (1987)], pAGE210, pME18SFL3, pKANTEX93 (WO 97/10354), N5KG1val (US Patent No. 6,001,358), Tol2 transposon vector (WO 2010/143698), and the like.
As a promoter, any promoter can be used as long as it can exhibit its functions in an animal cell. Examples thereof include a cytomegalovirus (CMV) immediate early (IE) gene promoter, an SV40 early promoter, a retrovirus promoter, a metallothionein promoter, a heat-shock promoter, an SRα promoter, and a Moloney murine leukemia virus promoter or enhancer. In addition, a human CMV IE gene enhancer may be used together with the promoter.
As the expression vector, either of a type in which the H chain and the L chain of the antibody exist on separate vectors or a type in which both the H chain and the L chain of the antibody exist on the same vector (hereinafter, referred to as tandem type) can be used.

(2) Acquisition of cDNA Encoding Variable Region

cDNAs encoding VH and VL of an arbitrary antibody can be obtained in the following manner. The cDNAs are synthesized using mRNA extracted from a hybridoma cell that produces the arbitrary antibody as a template. The synthesized cDNAs are inserted into a vector such as a phage or a plasmid, thereby producing a cDNA library.
A recombinant phage or a recombinant plasmid having a cDNA encoding VH and a recombinant phage or a recombinant plasmid having a cDNA encoding an L chain variable region are each isolated from the library using a DNA encoding a constant region or a variable region of an existing antibody as a probe. The entire base sequences of VH and VL of the target antibody on the recombinant phage or the recombinant plasmid are determined, and then the entire amino acid sequences of VH or VL are deduced from the base sequences.
The hybridoma cell that produces an arbitrary non-human animal antibody can be obtained as follows. A non-human animal is immunized with an antigen to which the antibody binds, and a hybridoma is produced from an antibody-producing cell of the immunized animal and a myeloma cell according to a well-known method [Molecular Cloning, Second Edition, Current Protocols in Molecular Biology, Antibodies, A Laboratory Manual, Cold Spring Harbor Laboratory, 1988, Monoclonal Antibodies: principles and practice, Third Edition, Acad. Press, 1993, Antibody Engineering, A Practical

Approach, IRL Press at Oxford University Press, 1996]. Subsequently, a single-cell cloned hybridoma is selected and cultured, followed by purification from the culture supernatant.

As the non-human animal, any animal such as a mouse, a rat, a hamster, or a rabbit can be used as long as it can produce a hybridoma cell.

Examples of the method for preparing total RNA from the hybridoma cell include a guanidine thiocyanate-cesium trifluoroacetate method [Methods in Enzymol., 154, 3 (1987)], and an RNeasy Kit (manufactured by QIAGEN, Inc.), and also examples of the method for preparing mRNA from total RNA include an oligo (dT) immobilized cellulose column method [Molecular Cloning: A Laboratory Manual, Cold Spring Harbor Lab. Press New York, 1989] and the like.

Further, examples of a kit for preparing mRNA from the hybridoma cell include Fast Track mRNA Isolation Kit (manufactured by Invitrogen, Inc.), Quick Prep mRNA Purification Kit (manufactured by Pharmacia Company), and the like.

Examples of the methods for synthesizing a cDNA and preparing a cDNA library include conventional methods (Molecular Cloning: A Laboratory Manual, Cold Spring Harbor Lab. Press New York, 1989; and Current Protocols in Molecular Biology, Supplement 1-34), and a method using a commercially available kit. Examples of the commercially available kit include Super Script (registered trademark) Plasmid System for cDNA Synthesis and Plasmid Cloning (manufactured by GIBCO BRL, Inc.) and ZAP-cDNA Synthesis Kit (manufactured by Stratagene, Inc.).

When the cDNA library is produced, as the vector into which a cDNA synthesized using mRNA extracted from a hybridoma cell as a template is incorporated, any vector can be used as long as it can incorporate the cDNA.

For example, ZAP Express (Strategies, 5, 58, 1992), pBluescript II SK(+) (Nucleic Acids Research, 17, 9494, 1989), λZAP II (manufactured by Stratagene, Inc.), λgt 10 and λgt 11 (DNA Cloning: A Practical Approach, I, 49, 1985), Lambda BlueMid (manufactured by Clontech Laboratories, Inc.), λExCell, pT7T3 18U (manufactured by Pharmacia, Inc.), pcD2 (Mol. Cell. Biol., 3, 280, 1983), pUC18 (Gene, 33, 103, 1985), or the like can be used.

As Escherichia coli into which a cDNA library constructed by a phage or plasmid vector is introduced, any Escherichia coli can be used as long as it can introduce, express, and maintain the cDNA library.

For example, XL1-Blue MRF (Strategies, 5, 81, 1992), C600 (Genetics, 39, 440, 1954), Y1088, Y1090 (Science, 222, 778, 1983), NM522 Journal of Molecular Biology (J. Mol. Biol., 166, 1, 1983), K802 (J. Mol. Biol., 16, 118, 1966), JM105 (Gene, 38, 275, 1985), or the like is used.

As for the method for selecting cDNA clones encoding VH and VL of the non-human animal antibody from the cDNA library, the selection can be performed by a colony hybridization method using a probe labeled with an isotope, fluoresce, or the like, or a plaque hybridization method (Molecular Cloning: A Laboratory Manual, Cold Spring Harbor Lab. Press New York, 1989).

Further, it is also possible to prepare cDNAs encoding VH and VL by preparing primers and performing PCR (Molecular Cloning: A Laboratory Manual, Cold Spring Harbor Lab. Press New York, 1989; and Current Protocols in Molecular Biology, Supplement 1-34) using cDNAs or the cDNA library as a template.

The cDNA selected by the above-mentioned method is cleaved with an appropriate restriction enzyme or the like, and then cloned into a plasmid such as pBluescript II SK(-) (manufactured by Stratagene, Inc.), and the base sequence of the cDNA can be determined by a commonly used base sequence analysis method, for example, by performing a reaction of a dideoxy method by Sanger et al. (Proc. Natl. Acad. Sci. U. S. A., 74, 5463, 1977) or the like, and then performing an analysis using an automatic base sequence analyzer, for example, a base sequence analyzer such as an ABI PRISM 377 DNA sequencer (manufactured by Applied Biosystems, Inc.).

By deducing the entire amino acid sequence of each of VH and VL from the determined base sequence and comparing it with the entire amino acid sequence of each of VH and VL of a known antibody (Sequences of Proteins of Immunological Interest, US Dept. Health and Human Services, 1991), it can be confirmed whether the obtained cDNA encodes the amino acid sequence completely including each of VH and VL of the antibody containing a secretion signal sequence.

Further, when the amino acid sequence of an antibody variable region or the base sequence of a DNA encoding the variable region is already known, it can be produced using the following method.

When the amino acid sequence is known, the DNA can be obtained by designing the base sequence of a DNA encoding the variable region in consideration of the codon usage frequency (Sequences of Proteins of Immunological Interest, US Dept. Health and Human Services, 1991), synthesizing several synthetic DNAs composed of approximately 100 to 150 bases based on the base sequence of the designed DNA, and carrying out the PCR method using them or synthesizing a full-length DNA. When the base sequence is known, the DNA can be obtained in the same manner as described above based on the information.

(3) Analysis of Amino Acid Sequence of Variable Region of Antibody

With respect to the complete amino acid sequences of VH and VL of the antibody containing a secretion signal sequence, by comparing them with the amino acid sequences of VH and VL of a known antibody (Sequences of Proteins

of Immunological Interest, US Dept. Health and Human Services, 1991), the length of the secretion signal sequence and the N-terminal amino acid sequence can be deduced, and further the subgroup to which the antibody belongs can be known. In addition, also the amino acid sequences of the respective CDRs of VH and VL can be found out by a similar method.

(4) Construction of cDNA Encoding Variable Region of Humanized Antibody

The cDNAs encoding VH and VL of a humanized antibody can be constructed in the following manner. First, the amino acid sequences of framework regions (hereinafter, referred to as FRs) of VH and VL of a human antibody for grafting the CDRs of VH and VL of a target non-human animal antibody are selected. As the amino acid sequences of FRs of VH and VL of the human antibody, any can be used as long as they are derived from a human antibody.

Examples thereof include amino acid sequences of FRs of VH and VL of human antibodies registered in a database such as Protein Data Bank, common amino acid sequences in each subgroup of FRs of VH and VL of human antibodies (Sequences of Proteins of Immunological Interest, US Dept. Health and Human Services, 1991), and the like.

Among them, in order to prepare the humanized antibody having a sufficient activity, it is preferred to select amino acid sequences having a homology as high as possible (at least 60% or more) with the amino acid sequences of FRs of VH and VL of the target non-human animal antibody.

Subsequently, the amino acid sequences of CDRs of VH and VL of the target non-human animal antibody are grafted to the selected amino acid sequences of FRs of VH and VL of the human antibody, and the amino acid sequences of VH and VL of the humanized antibody are designed. The designed amino acid sequences are converted into base sequences of DNAs in consideration of the usage frequency of codons found in the base sequences of the antibody genes (Sequences of Proteins of Immunological Interest, US Dept. Health and Human Services, 1991), and the base sequences of DNAs encoding the amino acid sequences of VH and VL of the humanized antibody are designed. The designed base sequences of the DNAs are fully synthesized.

Further, cloning into the recombinant vector for expression of the IgG half-molecule constructed in the above 3(1) can be easily carried out by introducing an appropriate restriction enzyme recognition sequence at the 5' ends of the synthetic DNA located at both ends. After PCR, each of the amplification products is cloned into a plasmid such as pBluescript II SK(-) (manufactured by Stratagene, Inc.) and the base sequences are determined by the method described in the above 3(2), thereby obtaining a plasmid having the base sequences of the DNAs encoding the amino acid sequences of VH and VL of the desired humanized antibody.

(5) Alteration of Amino Acid Sequence of Variable Region of Humanized Antibody

It is known that the antigen-binding activity of a humanized antibody prepared merely by grafting only CDRs of VH and VL of a non-human animal antibody to FRs of VH and VL of a human antibody is decreased as compared with that of the original non-human animal antibody (BIO/TECHNOLOGY, 9, 266, 1991).

The reason for this is probably because in VH and VL of the original non-human animal antibody, not only CDRs but also some of the amino acid residues of FRs are directly or indirectly involved in the antigen-binding activity, and such amino acid residues are changed to different amino acid residues of FRs of VH and VL of the human antibody by grafting the CDRs.

In order to solve this problem, an attempt has been made for a humanized antibody to raise the lowered antigen-binding activity by identifying the amino acid residues directly involved in the binding to an antigen or the amino acid residues indirectly involved in the binding to an antigen through an interaction with the amino acid residues of CDRs to maintain the conformation of the antibody in the amino acid sequences of FRs of VH and VL of the human antibody, and altering them to amino acid residues derived from the original non-human animal antibody (BIO/TECHNOLOGY, 9, 266, 1991).

In the preparation of the humanized antibody, it is most important how efficiently the amino acid residues of FRs involved in the antigen-binding activity are identified, and therefore, the construction and analysis of the conformation of the antibody have been carried out by X-ray crystallography (J. Mol. Biol., 112, 535, 1977), computer modeling (Protein Engineering, 7, 1501, 1994), or the like.

Such information of the conformation of the antibody has provided much useful information for the preparation of humanized antibodies. However, there has not yet been established any method for preparing a humanized antibody adaptable to all types of antibodies. At present, it is still necessary to make various trial-and-error approaches such as preparation of several types of variants for each antibody and examination of the correlation with the antigen-binding activity among the variants.

Alteration of the amino acid residues of FRs of VH and VL of a human antibody can be achieved by the PCR method described in the above 3(4) using synthetic DNAs for alteration. With respect to the amplification product after the PCR, the base sequence is determined by the method described in 3(2) to confirm that the desired alteration has

been carried out.

(6) Expression of IgG Half-Molecule

A transformant that transiently or stably produces at least one of the first and second IgG half-molecules can be obtained by introducing the recombinant vector for expression of the IgG half-molecule of the above 3(1) into an appropriate animal cell.

(6-a) Transient Expression of Antibody Composition

By performing transient expression of antibody compositions using the recombinant vector for expression of the IgG half-molecule obtained in (3) and (6) or a recombinant vector obtained by alteration thereof, the antigen-binding activities of many types of antibody compositions prepared can be efficiently evaluated.

As a host cell into which the recombinant vector for expression of the IgG half-molecule is introduced, any cell can be used as long as it is a host cell capable of expressing at least one of the first and second IgG half-molecules. For example, COS-7 cells [American Type Culture Collection (ATCC) number: CRL1651] are exemplified (Methods in Nucleic Acids Res., CRC press, 283, 1991).

In the introduction of the recombinant vector for expression of the IgG half-molecule into COS-7 cells, a DEAE-dextran method (Methods in Nucleic Acids Res., CRC press, 1991), a lipofection method (Proc. Natl. Acad. Sci. USA, 84, 7413, 1987), or the like is used.

After the introduction of the recombinant vector for expression of the IgG half-molecule, the expression level and the antigen-binding activity of the IgG half-molecule in a culture supernatant are measured using an enzyme immunoassay method [Monoclonal Antibodies-Principles and practice, Third edition, Academic Press (1996), Antibodies-A Laboratory Manual, Cold Spring Harbor Laboratory (1988) and A manual for monoclonal antibody experiments, Kodansha scientific books (1987)] or the like.

(6-b) Stable Expression of IgG Half-Molecule

A transformant that stably expresses the IgG half-molecule can be obtained by introducing the recombinant vector for expression of the IgG half-molecule obtained in (1) into an appropriate host cell.

In the introduction of the recombinant vector into a host cell, any method can be used as long as it is a method for introducing a DNA into the host cell, and examples thereof include an electroporation method (Cytotechnology, 3, 133, 1990), a calcium phosphate method (JP-A-H2-227075), a lipofection method (Proc. Natl. Acad. Sci. U. S. A., 84, 7413, 1987), an injection method [Manipulating the Mouse Embryo A Laboratory Manual], a method using a particle gun (gene gun) (Japanese Patent No. 2606856 and Japanese Patent No. 2517813), a DEAE-dextran method [Biomanual Series 4-Methods of Gene Transfer, Expression and Analysis (Yodosha), edited by Takashi Yokota and Kenichi Arai (1994)], a virus vector method (Manipulating Mouse Embryo, Second Edition), and the like.

As the host cells into which the recombinant vector is introduced, any cell can be used as long as it is a host cell capable of expressing at least one of the first and second IgG half-molecules. Examples thereof include human leukemia cell Namalwa cells, monkey COS cells, CHO cells that are Chinese hamster cells, HBT5637 (JP-A-S63-299), rat myeloma cells, mouse myeloma cells, cells derived from Syrian hamster kidney, embryonic stem cells, fertilized egg cells, and the like.

Specific examples thereof include PER.C6, CHO-K1 (ATCC CCL-61), DUKXB11 (ATCC CCL-9096), Pro-5 (ATCC CCL-1781), CHO-S (Life Technologies, Cat #11619), Lec13 cells, rat myeloma cells YB2/3HL.P2.G11.16Ag.20 (ATCC NO: CRL1662, or also called YB2/0), mouse myeloma cells NS0, mouse myeloma cells SP2/0-Ag14 (ATCC NO: CRL1581), mouse P3X63-Ag8.653 cells (ATCC NO: CRL1580), dihydrofolate reductase gene (dihydroforate reductase, hereinafter, referred to as dhfr)-deficient CHO cells (CHO/DG44) [Proc. Natl. Acad. Sci. USA, 77, 4216 (1980)], Syrian Hamster cells BHK, HBT563 cells, cells of substrains of the above-mentioned cell lines and cells prepared by acclimating the above-mentioned cell lines under serum-free culture, cells prepared by acclimating the above-mentioned cell lines under non-adhesion culture conditions, and the like.

As a cell used for the production of the IgG half-molecule, a cell for reducing or deleting the amount of core fucose of the sugar chain bound to Asn at position 297 according to the EU index in Fc can also be used. Specifically, a cell in which an enzyme involved in the synthesis of GDP-L-fucose or transport thereof to the Golgi body, or an enzyme involved in the binding of core fucose has been reduced or deleted is selected, or a cell obtained by any of various artificial methods can also be used as the host cell.

Specifically, a cell in which core fucose is controlled can be produced by a method for reducing or deleting an enzyme activity involved in the sugar chain modification of core fucose, a method for increasing an activity of a core fucose cleavage enzyme, or the like.

Examples of the enzyme involved in the sugar chain modification of core fucose include an enzyme involved in the synthesis or transport of GDP-L-fucose, and an enzyme involved in the binding of core fucose to an N-glycoside-linked complex sugar chain.

Specific examples of the enzyme involved in the synthesis of GDP-L-fucose or the transport thereof to the Golgi body include GDP-mannose 4,6-dehydratase (hereinafter referred to as GMD), GDP-4-keto-6-deoxy-D-mannose-3,5-epimerase (hereinafter referred to as Fx), GDP-beta-L-fucose pyrophosphorylase (GFPP), fucokinase, GDP-L-fucose transporter, and the like.

Examples of the enzyme involved in the binding of core fucose include α1,6-fucosyltransferase (hereinafter referred to as FUT8), and the like.

In the cell that produces the IgG half-molecule, one of the above-mentioned enzyme activities may be reduced or deleted or a plurality of enzyme activities may be combined and reduced or deleted.

Examples of the method for reducing or deleting the above-mentioned enzyme activities include (a) a method for gene disruption targeting the gene of the enzyme; (b) a method for introducing a dominant-negative mutant of the gene of the enzyme; (c) a method for introducing a mutation into the enzyme; (d) a method for suppressing transcription or translation of the gene of the enzyme; (e) a method for selecting a cell line resistant to a lectin that recognizes a sugar chain structure in which the 6-position of N-acetylglucosamine at the reducing end of the N-glycoside-linked sugar chain and the 1-position of fucose are α-linked; and the like.

Examples of the lectin include a lectin that is bound to α1,6-fucose such as lentil lectin LCA (lentil agglutinin derived from Lens culinaris), pea lectin PSA (pea lectin derived from Pisum sativum), broad bean lectin VFA (agglutinin derived from Vicia faba), and Aleuria aurantia lectin AAL (lectin derived from Aleuria aurantia).

Specific examples of the cell include FUT8 gene-deficient CHO cells (WO 2005/035586, WO 2002/31140, and WO 2000/061739), Lec13 that has acquired lectin resistance (Somatic Cell and Molecular genetics, 12, 55, 1986), GDP-fucose transporter gene-deficient cells (WO 2003/085102), GDP-mannose 4,6-dehydratase (GMD) gene-deficient cells (WO 2002/31140), WGA lectin resistant cells, LCA lectin resistant cells (WO 2002/31140), and the like.

In addition to the above-mentioned methods, the IgG half-molecule in which high mannose-type N-linked sugar chain is bound and the amount of core fucose is reduced can also be expressed by inhibiting an enzyme such as mannosidase I or mannosidase II that is an enzyme involved in an N-linked sugar chain synthesis system.

Further, by using a host cell made to overexpress N-acetylglucosamine transferase III (GnTIII), the IgG half-molecule in which a bisecting GlcNAc-bound complex and a hybrid sugar chain are bound, and the amount of core fucose is reduced can also be produced.

After introduction of the recombinant vector, a transformant that stably expresses the IgG half-molecule is selected by culturing it in a culture medium for animal cell culture containing an agent such as G418 sulfate (hereinafter, referred to as G418), cycloheximide (hereinafter, abbreviated as CHX), or methotrexate (hereinafter, abbreviated as MTX) (JP-A-H2-257891).

Examples of the culture medium for animal cell culture include RPMI 1640 medium (manufactured by Invitrogen, Inc.), GIT medium (manufactured by Nihon Pharmaceutical Co., Ltd.), EX-CELL301 medium, EX-CELL302, EX-CELL325 medium (manufactured by JRH), IMDM medium (manufactured by Invitrogen, Inc.), Hybridoma-SFM medium (manufactured by Invitrogen, Inc.), culture media obtained by adding various additives such as fetal bovine serum (hereinafter abbreviated as FBS) to these culture media, and the like.

The IgG half-molecule is expressed and accumulated in a culture supernatant by culturing the obtained transformant in a culture medium. The expression level and the antigen-binding activity of the IgG half-molecule in the culture supernatant can be measured by an ELISA method or the like. In addition, the expression level of the IgG half-molecule produced by the transformant can be improved using a dhfr gene amplification system (JP-A-H2-257891) or the like.

The method for expressing the IgG half-molecule using an animal cell as the host has been described, however, the IgG half-molecule can be expressed also in yeast, an insect cell, a plant cell, or an animal individual or a plant individual in the same manner as in the animal cell based on a known technique.

When yeast is used as the host cell, a microorganism belonging to the genus Saccharomyces, the genus Schizosaccharomyces, the genus Kluyveromyces, the genus Trichosporon, the genus Schwanniomyces, or the like, for example, Saccharomyces cerevisiae, Schizosaccharomyces pombe, Kluyveromyces lactis, Trichosporon pullulans, Schwanniomyces alluvius or the like can be exemplified.

As a method for introducing the recombinant vector, any method can be used as long as it is a method for introducing a DNA into yeast, and for example, methods described in an electroporation method (Methods Enzymol., 194, 182, 1990), a spheroplast method (Proc. Natl. Acad. Sci. U. S. A., 84, 1929, 1978), a lithium acetate method (J. Bacteriology, 153, 163, 1983, Proc. Natl. Acad. Sci. U. S. A., 75, 1929, 1978), and the like are exemplified.

When an insect cell is used as the host, the IgG half-molecule can be expressed by, for example, a method described in current Protocols in Molecular Biology (Baculovirus Expression Vectors, A Laboratory Manual, W. H. Freeman and Company, New York, 1992), Bio/Technology, 6, 47, 1988, or the like.

[Step 2]

The step 2 is a step of culturing the transformant obtained in the step 1 to produce and accumulate the IgG half-molecule in a culture, and collecting the IgG half-molecule from the culture and purifying the IgG half-molecule.

The first and second IgG half-molecules may be collected from the same transformant, or may be collected from transformants individually expressing each of the first and second IgG half-molecules. Generally, the first and second IgG half-molecules are collected from transformants individually expressing each of the first and second IgG half-molecules, and mixed after purification, whereby the antibody composition is prepared.

When the host cell prepared in the step 1 has an ability to express the IgG half-molecule, after the IgG half-molecule is introduced into the host cell described below, the cell is cultured, and the target IgG half-molecule can be collected from the culture.

Further, an animal individual into which a gene is introduced (non-human transgenic animal) or a plant individual into which a gene is introduced (transgenic plant) is prepared by redifferentiation of an animal or plant cell into which a gene is introduced, and the IgG half-molecule may be collected using such an individual.

When the transformant is an animal individual or a plant individual, the individual is raised or cultivated according to a conventional method to produce and accumulate the IgG half-molecule, and the IgG half-molecule can be collected from the animal individual or the plant individual.

Examples of the method for producing the IgG half-molecule using an animal individual include a method for producing the target IgG half-molecule in an animal prepared by introducing a gene according to a known method (American Journal of Clinical Nutrition, 63, 639S, 1996; American Journal of Clinical Nutrition, 63, 627S, 1996; Bio/Technology, 9, 830, 1991).

In the case of an animal individual, for example, a non-human transgenic animal into which a DNA encoding the IgG half-molecule is introduced is raised to produce and accumulate the IgG half-molecule in the animal, and the IgG half-molecule can be collected from the inside of the animal.

As a place of production and accumulation in the animal, for example, milk (JP-A-S63-309192), egg, or the like of the animal can be exemplified. As the promoter used at that time, any promoter can be used as long as it enables expression in an animal. For example, an α-casein promoter, a β-casein promoter, a β-lactoglobulin promoter, and a whey acidic protein promoter that are mammary gland cell-specific promoters, and the like are preferably used.

Examples of the method for producing the IgG half-molecule using a plant individual include a method for cultivating a transgenic plant into which a DNA encoding the IgG half-molecule is introduced according to a known method [Tissue Culture, 20 (1994); Tissue Culture, 21 (1995); and Trends in Biotechnology, 15, 45 (1997)] to produce and accumulate the IgG half-molecule in the plant, and collecting the IgG half-molecule from the inside of the plant.

The IgG half-molecule can be purified in the following manner. When the IgG half-molecule produced by the transformant into which the gene encoding the IgG half-molecule is introduced is, for example, expressed as a soluble protein in the cells, after culture is completed, the cells are collected by centrifugation and suspended in an aqueous buffer solution, and thereafter, the cells are homogenized using an ultrasonic homogenizer, a French press, a Manton Gaulin homogenizer, a dynomill, or the like, whereby a cell-free extract is obtained.

From the supernatant obtained by centrifugation of the cell-free extract, the IgG half-molecule can be purified by a general enzyme isolation and purification method, that is, by using methods such as a solvent extraction method, a salting-out method using ammonium sulfate or the like, a desalting method, a precipitation method using an organic solvent, anion exchange chromatography using a resin such as diethylaminoethyl (DEAE)-sepharose or DIAION HPA-75 (manufactured by Mitsubishi Chemical Corporation), cation exchange chromatography using a resin such as S-Sepharose FF (Pharmacia, Inc.), hydrophobic chromatography using a resin such as butyl sepharose or phenyl sepharose, a gel filtration method using a molecular sieve, affinity chromatography, a chromatofocusing method, and electrophoresis such as isoelectric focusing electrophoresis alone or in combination.

In the present invention, as the affinity chromatography, affinity chromatography using a CH-binding body or an Fc-binding body is used (Monoclonal Antibodies-Principles and practice, Third edition, Academic Press, 1996, Antibodies-A Laboratory Manual, Cold Spring Harbor Laboratory, 1988).

Further, when the IgG half-molecule is expressed within the cells by forming an insoluble body, the cells are collected and homogenized in the same manner, followed by centrifugation, whereby the insoluble body of the IgG half-molecule is collected as a precipitated fraction. The collected insoluble body of the IgG half-molecule is solubilized with a protein denaturing agent. The IgG half-molecule is returned to a normal conformation by diluting or dialyzing the solubilized solution, and thereafter, the IgG half-molecule can be purified by the same isolation and purification method as described above.

When the IgG half-molecule is secreted extracellularly, the IgG half-molecule or a derivative thereof can be recovered in the culture supernatant. That is, the culture supernatant is obtained by treating the culture by a method such as centrifugation in the same manner as described above, and the IgG half-molecule can be purified from the culture supernatant by the same isolation and purification method as described above.

Specifically, the CH-binding body or the Fc-binding body may be any material such as a protein or a resin as long as it binds to CH or Fc, and examples thereof include an Fc-binding protein, an antibody that binds to an H chain constant region (CH) of an antibody, and the like.

Example of the Fc-binding protein include Protein A derived from Staphylococcus Aureus, Protein G derived from hemolytic Streptococcus, an Fc receptor and the subclasses thereof (FcyRI, IIA, IIB, IIIA, and IIIB) and binding portion fragments thereof, and the like.

Examples of the antibody that binds to CH include antibodies that bind to a CH1 domain, a hinge domain, a CH2 domain, or a CH3 domain.

In the present invention, more preferred examples of the CH-binding body include Protein A, Protein G, an anti-CHI antibody, and binding portion fragments thereof.

As the method for purifying the IgG half-molecule, for example, the culture supernatant obtained by culturing using the transformant described above in [step 1] (6) is loaded onto a Protein A column or a Protein G column, and thereafter the column is washed with a phosphate buffer (phosphate buffer saline, hereinafter, abbreviated as PBS).

Thereafter, the IgG half-molecule is eluted from the column using a citric acid buffer at low pH (pH 2.0 to 6.0) or the like, and the eluate is neutralized with an alkaline Tris buffer or the like. The neutralized eluate is subjected to dialysis using a sufficient amount of PBS or the like, whereby the purified IgG half-molecule can be obtained.

The molecular weight of the purified IgG half-molecule can be measured using polyacrylamide gel electrophoresis [Nature, 227, 680 (1970)], Western blotting [Monoclonal Antibodies-Principles and practice, Third edition, Academic Press (1996), Antibodies-A Laboratory Manual, Cold Spring Harbor Laboratory (1988)], or the like.

[Step 3]

The step 3 is a step of mixing the first and second IgG half-molecules collected and purified in the step 2, thereby obtaining the antibody composition. It is preferred that the mixing ratio of the first and second IgG half-molecules is appropriately set according to a binding activity to an antigen, a binding activity to an antigen-positive cultured cell line, the strength of a CD16a-binding activity in each CD16a-binding domain, an interaction between the CH3 of the first IgG half-molecule and the CH3 of the second IgG half-molecule, or the like.

4. Evaluation of Activity of Antibody Composition

As a method for measuring the protein amount of the purified IgG half-molecule, an FcR-binding activity, a C1q-binding activity, an antigen-binding activity, or cellular cytotoxicity such as an ADCC activity or a CDC activity of the antibody composition constituted by the IgG half-molecule, known methods described in, for example, Molecular Cloning 2nd Edition, Current Protocols in Molecular Biology; Antibodies, A Laboratory manual, Cold Spring Harbor Laboratory, 1988; Monoclonal Antibodies: principles and practice, Third Edition, Acad. Press, 1993; Antibody Engineering, A Practical Approach, IRL Press at Oxford University Press, 1996, and the like are exemplified.

As a specific example, the binding activity of the antibody composition to an antigen or the binding activity thereof to an antigen-positive cultured cell line can be measured by an ELISA method, a fluorescent antibody method (Cancer Immunol. Immunother, 36, 373, 1993), or the like. The cellular cytotoxicity against an antigen-positive cultured cell line can be evaluated by measuring a CDC activity, an ADCC activity, or the like (Cancer Immunol. Immunother, 36, 373, 1993; US Patent Application Publication No. 2004/0259150).

Whether or not the antibody composition has a binding activity to CD16a can be confirmed by producing a recombinant CD16a protein and then measuring a binding activity (US Patent Application Publication No. 2004/0259150).

Example of the method for measuring an ADCC activity include a method in which a target cell labeled with a radioisotope, a fluorescent substance, a dye, or the like, the antibody composition, and an effector cell are brought into contact with one another, and then the activity of the labeling substance released from the damaged target cell or the biological activity or the like of an enzyme released therefrom is measured, and the like.

Example of the method for measuring a CDC activity include a method in which a target cell labeled with a radioisotope, a fluorescent substance, a dye, or the like, the antibody composition, and a biological sample such as serum containing a complement factor are brought into contact with one another, and then the activity of the labeling substance released from the damaged target cell or the biological activity of an enzyme released therefrom is measured, and the like.

5. Analysis of Sugar Chain Structure

The sugar chain structure of the IgG half-molecule expressed in various types of cells can be analyzed according to a general analysis of a sugar chain structure of a glycoprotein.

For example, a sugar chain bound to the IgG half-molecule is constituted by a neutral sugar such as galactose

(Gal), mannose (Man), or fucose (Fuc), an amino sugar such as N-acetylglucosamine (GlcNAc), or an acidic sugar such as sialic acid (Sial), and can be analyzed using a technique such as a sugar chain structure analysis using a sugar composition analysis and a two-dimensional sugar chain mapping method.

(1) Neutral Sugar and Amino Sugar Composition Analysis

A composition analysis of a sugar chain of the IgG half-molecule can be carried out by performing acid hydrolysis of a sugar chain with trifluoroacetic acid or the like to release a neutral sugar or an amino sugar and analyzing the composition ratio thereof.

Specific examples of the method include a method using a sugar composition analyzer manufactured by Dionex Corporation. BioLC is a device for analyzing a sugar composition by an HPAEC-PAD (high performance anion-exchange chromatography-pulsed amperometric detection) method (J. Liq. Chromatogr., 6, 1577, 1983).

Further, a composition ratio can be analyzed also by a fluorescence labeling method using 2-aminopyridine. Specifically, a sample that is acid-hydrolyzed according to a known method [Agric. Biol. Chem., 55(1), 283-284, 1991] is fluorescently labeled by 2-aminopyridylation, and a composition ratio can be calculated by performing an HPLC analysis.

(2) Sugar Chain Structure Analysis

The structure analysis of a sugar chain in the IgG half-molecule can be carried out by a two-dimensional sugar chain mapping method (Anal. Biochem., 171, 73, 1988; Biochemical Experimentation Methods 23-Methods of Studies on Glycoprotein Sugar Chains, Japan Scientific Societies Press, edited by Reiko Takahashi, 1989). The two-dimensional sugar chain mapping method is, for example, a method for deducing a sugar chain structure by plotting the retention time or elution position of a sugar chain by reverse-phase chromatography on the X axis and the retention time or elution position of the sugar chain by normal-phase chromatography on the Y axis, respectively, and comparing them with results of those of known sugar chains.

Specifically, a sugar chain is released from the IgG half-molecule by hydrazinolysis of the IgG half-molecule and fluorescence labeling of the sugar chain with 2-aminopyridine (hereinafter, abbreviated as PA) (J. Biochem., 95, 197, 1984) is performed, and thereafter, the sugar chain is separated from an excess amount of a PA-treating reagent by gel filtration, followed by reverse-phase chromatography. Then, each peak of the fractionated sugar chain is subjected to normal-phase chromatography. The sugar chain structure can be deduced by plotting on a two-dimensional sugar chain map based on these results and comparing them with the spots of a sugar chain standard (manufactured by TaKaRa, Inc.) or those in literature (Anal. Biochem., 171, 73, 1988).

Further, mass spectrometry such as MALDI-TOF-MS of each sugar chain is performed, and the structure deduced by the two-dimensional sugar chain mapping method can be confirmed.

Which portion of Fc of the IgG half-molecule a sugar chain is bound to can be confirmed by treating the IgG half-molecule subjected to reductive alkylation with an endoprotease such as trypsin, pepsin, Lys-C, or Asp-N, and separating the resultant by reverse-phase chromatography (LC), followed by an analysis using a mass spectrometer (MS) or the like.

In other words, whether or not a sugar chain is actually bound can be confirmed by whether or not the molecular weight of a peptide that can be produced by a protease treatment and the molecular weight of a peptide to which the sugar chain is bound matches the MS analytical values based on the amino acid sequence of the Fc of the target IgG half-molecule.

6. Method for Identifying Sugar Chain Structure

The ratio of sugar chains having no core fucose among all the N-glycoside-linked complex sugar chains bound to Fc in the IgG half-molecule can be identified using the method for analyzing a sugar chain structure described in the above 5. In addition, it can also be identified by an immunological quantitative method using a lectin.

The identification of a sugar chain structure in the IgG half-molecule by an immunological quantitative method using a lectin can be carried out according to an immunological quantitative method such as Western staining, RIA (radioimmunoassay), VIA (viroimmunoassay), EIA (enzymoimmunoassay), FIA (fluoroimmunoassay), or MIA (metalloimmunoassay) described in literature [Monoclonal Antibodies: Principles and Applications, Wiley-Liss, Inc. (1995); Enzyme Immunoassay, 3rd Ed., IGAKU-SHOIN Ltd. (1987); Enzyme Antibody Technique, Revised Edition, Gakusai Kikaku (1985)], or the like, for example, in the following manner.

A lectin that recognizes the sugar chain structure of the IgG half-molecule is labeled, and the labeled lectin and the antibody composition that is a sample are reacted with each other. Subsequently, the amount of a complex of the labeled lectin with the antibody composition is measured.

Examples of the lectin used for identification of the sugar chain structure of the IgG half-molecule include WGA

(wheat-germ agglutinin derived from T. vulgaris), ConA (concanavalin A derived from C. ensiformis), RIC (a toxin derived from R. communis), L-PHA (leucoagglutinin derived from P. vulgaris), LCA (lentil agglutinin derived from L. culinaris), PSA (pea lectin derived from P. sativum), AAL (Aleuria aurantia Lectin), ACL (Amaranthus caudatus Lectin), BPL (Bauhinia purpurea Lectin), DSL (Datura stramonium Lectin), DBA (Dolichos biflorus Agglutinin), EBL (Elderberry Balk Lectin), ECL (Erythrina cristagalli Lectin), EEL (Euonymus europaeus Lectin), GNL (Galanthus nivalis Lectin), GSL (Griffonia simplicifolia Lectin), HPA (Helix pomatia Agglutinin), HHL (Hippeastrum Hybrid Lectin), Jacalin, LTL (Lotus tetragonolobus Lectin), LEL (Lycopersicon esculentum Lectin), MAL (Maackia amurensis Lectin), MPL (Maclura pomifera Lectin), NPL (Narcissus pseudonarcissus Lectin), PNA (Peanut Agglutinin), E-PHA (Phaseolus vulgaris Erythroagglutinin), PTL (Psophocarpus tetragonolobus Lectin), RCA (Ricinus communis Agglutinin), STL (Solanum tuberosum Lectin), SJA (Sophora japonica Agglutinin), SBA (Soybean Agglutinin), UEA (Ulex europaeus Agglutinin), VVL (Vicia villosa Lectin), and WFA (Wisteria floribunda Agglutinin).

A lectin that specifically recognizes core fucose is preferably used, and specific examples thereof include lentil lectin LCA (lentil agglutinin derived from Lens culinaris), pea lectin PSA (pea lectin derived from Pisum sativum), broad bean lectin VFA (agglutinin derived from Vicia faba), and Aleuria aurantia lectin AAL (lectin derived from Aleuria aurantia).

7. Use of Antibody Composition of the Present Invention

The antibody composition of the present invention can recognize two types of antigens that are different from each other by being composed of the first and second IgG half-molecules having antigen-binding domains for antigens that are different from each other.

Therefore, the antibody composition of the present invention can take a molecular form in accordance with two different types of target antigens, and thus becomes an antibody composition medical product having high selectivity for a double-positive cell that expresses the two types of antigens.

The antigen to which the antibody composition of the present invention binds may be any antigen, and preferably, an antigen molecule associated with cancer, an immune disease, an allergic disease, a cardiovascular disease, or the like is exemplified. Examples thereof include a cytokine, a chemokine, a growth factor and a receptor therefor, a CD antigen, and the like.

Examples of the receptor for a cytokine or a growth factor include receptors for interferon (hereinafter referred to as IFN)-α, IFN-β, IFN-γ, interleukin (hereinafter referred to as IL)-2, IL-3, IL-4, IL- 5, IL-6, IL-7, IL-8, IL-9, IL-10, IL-12, IL-13, IL-15, IL-17, IL-18, IL-21, IL-23, IL-27, a granulocyte colony-stimulating factor (G-CSF), a granulocyte/macrophage colony-stimulating factor (GM-CSF), or a macrophage colony-stimulating factor (M-CSF), and the like.

Examples of the receptor for a chemokine include receptors for SLC, ELC, 1-309, TARC, MDC, MIP-3α, or CTACK.

Examples of the receptor for a growth factor include receptors for epidermal growth factor (EGF), vascular endothelial growth factor (VEGF), angiopoietin, fibroblast growth factor (FGF), hepatocyte growth factor (HGF), platelet-derived growth factor (PDGF), insulin-like growth factor (IGF), erythropoietin (EPO), TGFβ, Iephrin, angiopoietin, Frizzled ligand, or SDF-1.

Examples of the cluster of differentiation (hereinafter referred to as CD) antigen include CD1a, CD1c (BDCA1), CD1d, CD2, CD3, CD4, CD5, CD6, CD7, CD8, CD10, CD11a, CD11b, CD11c, CD14, CD16, CD18, CD19, CD20, CD21, CD22, CD23, CD24, CD25, CD26 (DPP-4), CD27, CD28, CD30, CD32, CD34, CD37, CD38, CD39, CD40, CD43, CD44, CD45, CD47, CD49, CD51, CD52, CD53, CD54, CD55, CD56, CD57, CD59, CD62E, CD62L, CD62P, CD64, CD66a (CEACAM1), CD66b (NCA-95), CD66c (NCA-50/90), CD66d (CGM1), CD66e (CEA), CD66f(PSG), CD68, CD69, CD70, CD72, CD73, CD74, CD75, CD76, CD77, CD78, CD79a, CD79b, CD80 (B7.1), CD81, CD82, CD83, CD84 (SLAMF5), CD85a (ILT-5), CD85b (ILT8), CD85c (LIR8), CD85d (ILT4), CD85f(ILT11), CD85g (ILT7), CD85h (ILT1), CD85i (LIR6a), CD85j (ILT2), CD85k (ILT3), CD85m (ILT10), CD86 (B7.2), CD87, CD89, CD94 (NKG2), CD95 (Fas), CD98, CD103, CD107a (LAMP1), CD114 (G-CSFR), CD115 (M-CSFR), CD116 (GM-CSFR), CD117 (SCF-R), CD119 (IFNGR1), CD121a (IL-1R1), CD122 (IL-2Rb), CD123 (IL-3Ra), CD124 (IL-4Ra), CD125 (IL-5Ra), CD126 (IL-6Ra), CD127 (IL-7Ra), CD134 (OX40), CD135 (FLT3), CD137 (4-1BB), CD138 (Syndecan-1), CD140 (PDGFR), CD146 (MUC18), CD147 (EMMRRIN), CD152 (CTLA-4), CD158a (KIR2DL1), CD158b1 (KIR2DL2), CD158b2 (KIR2DL3), CD158c (KIR2DS6), CD158d (KIR2DL4), CD158el (KIR3DL1), CD158e2 (KIR3DS1), CD158f (KIR2DL5), CD158g (KIR2DS5), CD158h (KIR2DS1), CD158i (KIR2DS4), CD158j (KIR2DS2), CD158k (KIR3DL2), CD159a (NKG2A), CD159c (NKG2C), CD161 (NKRP1A), CD162 (PSGL-1), CD163, CD169 (SIGLEC1), CD178 (FasL), CD183 (CXCR3), CD184 (CXCR4), CD185 (CXCR5), CD191 (CCR1), CD193 (CCR3), CD194 (CCR4), CD195 (CCR5), CD196 (CCR6), CD197 (CCR7), CD198 (CCR8), CD199 (CCR9), CD200 (OX2), CD206 (MMR), CD207 (Langerin), CD209 (DC-SIGN), CD212 (IL-12Rβ1), CD213al (IL-13Ra1), CD213a2 (IL-13Ra2), CD215 (IL-15RA), CD217 (IL-17R), CD218a (IL-18Ra), CD218b (IL-18Rβ), CD223 (LAG3), CD226 (DNAM-1), CD229 (SLAMF3), CD252 (OX40L), CD269 (BCMA), CD272 (BTLA), CD274 (PD-L1), CD276 (B7H3), CD278 (ICOS), CD279 (PD-1), CD281 (TLR1), CD282 (TLR2), CD283 (TLR3), CD284 (TLR4), CD286 (TLR6), CD288 (TLR8), CD289 (TLR9), CD294 (CRTH2), CD301 (MGL), CD302 (DCL1), CD303 (BDCA2),

CD304 (BDCA4), CD317 (BST2), CD324 (E-cadherin), CD326 (EpCAM), CD357 (GITR), CD358 (DR6), CD360 (IL-21R), CD365 (TIM-1), CD366 (TIM-3), CD369 (DECTIN-1), CD370 (CLEC9A), human leukocyte antigen (HLA)-Class II, HLA-I, and the like.

Further, examples of an antigen involved in pathogenesis of tumors or an antigen for an antibody, which regulates an immunological function, include ganglioside GM1, GM2, GD2, GD3, Lewis X, Lewis Y, CD3, CD4, CD40, a CD40 ligand, a B7 family molecule (for example, CD80, CD86, CD274, B7-DC, B7-H2, B7-H3, or B7-H4), a ligand for a B7 family molecule (for example, CD28, CTLA-4, ICOS, PD-1, or BTLA), OX-40, an OX-40 ligand, CD137, a tumor necrosis factor (TNF) receptor family molecule (for example, DR3, DR4, DR5, BAFFR, LIGHT, TNFR1, or TNFR2), a TNF-related apoptosis-inducing ligand receptor (TRAIL) family molecule, a receptor family of a TRAIL family molecule (for example, TRAIL-R1, TRAIL-R2, TRAIL-R3, or TRAIL-R4), a receptor activator of a nuclear factor kappa B ligand (RANK), an RANK ligand, CD25, a folate receptor, Mesothelin, Siglec-8, a cytokine/chemokine receptor [for example, IL-1RII, IL-12R$\beta$2, IL-17RB, IL-23R, IL-27R$\alpha$, IL-31R, IL-33R$\alpha$, IL-36R, transforming growth factor (TGF) $\beta$RII, CCR2, CCR10, CXCR1, or CXCR2], an NK cell receptor (for example, NKG2D, E4BP4, NKp30, NKp44, NKp46, or AhR), a T cell receptor (for example, TCR$\alpha/\beta$, TCR V$\beta$11, TCR$\gamma/\delta$, TSLPR, SLAM, SLAMF6, LAP,GARP, SR-A1, CD200R, DCR3, or TIGIT), a B cell receptor (for example, BLYS, APRIL, or TSLPR), a dendritic cell receptor (for example, FCER1A, TLR7, CADM1, XCR1, BTLA, SIRPA, DCIR, TROP2, AXL, SIGLEC6, SIGLEC15, CX3CR1, S100A8, S100A9, or ASGR1), and the like.

Examples of a receptor tyrosine kinase include an EGF receptor, an insulin receptor, an IGF-1 receptor, an NGF receptor, a PDGF receptor, an M-CSF receptor, an FGF receptor, a VEGF receptor, an Eph receptor, and the like. Examples of a tyrosine kinase-associated receptor include a cytokine receptor, an Fc receptor, and the like. Further, examples of a cell adhesion molecule include cadherin, integrin, and the like. Examples of a G protein-coupled receptor include an adenosine receptor, a glucagon receptor, and the like.

Specific examples of the receptor tyrosine kinase include Epidermal Growth Factor Receptor (EGFR), V-ERB-B2 Avian Erythroblastic Leukemia Viral Oncogene Homolog 2 (HER2), V-ERB-B2 Avian Erythroblastic Leukemia Viral Oncogene Homolog 3 (HER3), V-ERB-B2 Avian Erythroblastic Leukemia Viral Oncogene Homolog 4 (HER4), Insulin Receptor (INSR), Insulin-like Growth Factor I Receptor (IGF1R), Nerve Growth Factor Receptor (NGFR), Platelet-derived Growth Factor Receptor, Alpha (PDGFRA), Platelet-derived Growth Factor Receptor, Beta (PDGFRB), Colony-stimulating Factor Receptor (CSF1R), Colony-stimulating Factor 2 Receptor, Alpha (CSF2RA), Colony-stimulating Factor 3 Receptor, Granulocyte (CSF3R), Fibroblast Growth Factor Receptor 1 (FGFR1), Fibroblast Growth Factor Receptor 2 (FGFR2), Fibroblast Growth Factor Receptor 3 (FGFR3), Fibroblast Growth Factor Receptor 4 (FGFR4), Kinase Insert Domain Receptor (KDR), Ephrin Receptor EphA1 (EPHA1), Ephrin Receptor EphA2 (EPHA2), Ephrin Receptor EphA3 (EPHA3), and the like.

Further, examples of the tyrosine kinase-associated receptor include interleukin-1 receptor 1 (IL-1R1), interleukin-1 receptor accessory protein (IL-1RAP), hepatocyte growth factor receptor (c-Met), macrophage stimulating 1 receptor (RON), platelet-derived growth factor receptor (PDGFR), junctional adhesion molecule-like (JAML), nectin-like protein 5 (Necl-5), tumor necrosis factor receptor 1 (TNF-R1), tumor necrosis factor receptor 2 (TNF-R2), TNF-related apoptosis-inducing ligand receptor 1 (TRAIL-R1), TNF-related apoptosis-inducing ligand receptor 2 (TRAIL-R2), death receptor 3 (DR3), death receptor 6 (DR6), receptor activator of NF-kB (RANK), nerve growth factor receptor (NGFR), lymphotoxin-beta receptor (LT$\beta$R), OX40 (TNFRSF4), Fas (TNFRSF6), 4-1BB (TNFRSF9), Fn14 (TNFRSF12A), TACI (TNFRSF13B), BAFF-R (TNFRSF13C), HVEM (TNFRSF14), BCMA (TNFRSF17), GITR (TNFRSF18), TROY (TNFRSF19), ectodysplasin A1 receptor (EDAR), ectodysplasin A2 receptor (XEDAR), a receptor expressed in lymphoid tissues (RELT), CD3, CD27, CD30, CD40, FcaRI, Fc$\gamma$RIII and Fc$\varepsilon$RI, Fc Fragment of IgG, Receptor Transpoter, Alpha (FCGRT), and the like.

Further, examples of the cell adhesion molecule include Integrin, Alpha-9 (ITGA9), P-selectin Glycoprotein Ligand-1 (PSGL-1), Cadherin-11 (CDH11), Mucosal Vascular Addression Cell Adhesion Molecule 1 (MADCAM1), Integrin, Alpha-4 (ITGA4), Integrin, and Beta-4 (ITGB4), and examples of the G protein-coupled receptor include Adenosine A2A Receptor (ADORA2A), Adenosine A2B Receptor (ADORA2B), Repulsive Guidance Molecule A (RGMA), Glucagon Receptor (GCGR), Prolactin Receptor (PRLR), Glucagon-like Peptide-1 Receptor (GLP1R), and the like.

A medicine containing the antibody composition of the present invention can be administered alone as a therapeutic agent, however, it is preferably provided as a pharmaceutical preparation produced by generally mixing it together with one or more pharmaceutically acceptable carriers using an arbitrary method well known in the technical field of pharmaceutics.

As the route of administration, it is preferred to use the most effective route in the treatment. For example, oral administration or parenteral administration such as intraoral, intra-airway, intrarectal, subcutaneous, intramuscular, and intravenous administration can be exemplified, and in the case of an antibody composition preparation, preferably, intravenous administration can be exemplified.

Examples of a dosage form include a spray, a capsule, a tablet, a granule, a syrup, an emulsion, a suppository, an injection, an ointment, a tape, and the like.

Examples of the pharmaceutical preparation suitable for oral administration include an emulsion, a syrup, a capsule, a tablet, a powder, a granule, and the like.

A liquid preparation such as an emulsion or a syrup can be produced using, as an additive, water, a saccharide such as sucrose, sorbitol, or fructose, a glycol such as polyethylene glycol or propylene glycol, an oil such as sesame oil, olive oil, or soybean oil, a preservative such as a p-hydroxybenzoate ester, a flavor such as strawberry flavor or peppermint, or the like.

A capsule, a tablet, a powder, a granule, or the like can be produced using, as an additive, an excipient such as lactose, glucose, sucrose, or mannitol, a disintegrating agent such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, or the like.

Examples of the pharmaceutical preparation suitable for parenteral administration include an injection, a suppository, a spray, or the like.

An injection is prepared using a carrier composed of a salt solution or a glucose solution, or a mixture thereof, or the like. Alternatively, it is also possible to prepare a powder injection by freeze-drying the antibody composition according to a conventional method and adding sodium chloride thereto.

A suppository is prepared using a carrier such as cacao butter, a hydrogenated fat, or carboxylic acid.

Further, a spray is prepared using the antibody composition itself, or using a carrier which does not stimulate the buccal or airway mucous membrane of a recipient and facilitates absorption of the antibody composition by dispersing it as fine particles, or the like.

Specific examples of the carrier include lactose, glycerin, and the like. A pharmaceutical preparation such as an aerosol or a dry powder is possible according to the properties of the antibody composition and the carrier to be used. Further, also for these parenteral preparations, components exemplified as additives for the oral preparations can be added.

A dose or administration frequency varies depending on a desired therapeutic effect, an administration method, a treatment duration, an age, a body weight, or the like, however, a dose of the active ingredient is generally 10 μg/kg to 20 mg/kg per day for an adult.

Further, as a method for studying an anti-tumor effect of the antibody composition on various tumor cells, a CDC activity measurement method, an ADCC activity measurement method, and the like are exemplified as an in vitro experiment, and an anti-tumor experiment, and the like using a tumor system in an experimental animal such as a mouse are exemplified as an in vivo experiment.

As one aspect of the present invention, a kit including the first and second IgG half-molecules is exemplified. In the kit, other materials including an appropriate container (for example, a bottle, a vial, or a test tube), a label showing explanation or the like, a filter, a needle, a syringe, and an instruction manual may be optionally included.

In the above kit, in addition to the IgG half-molecule as the active ingredient, for example, sterile water, physiological saline, a vegetable oil, a surfactant, a lipid, a solubilizing agent, a buffer, a protein stabilizer (for example, BSA, gelatin, or the like), a preservative, a blocking solution, a reaction solution, a reaction stopping solution, a reagent for treating a sample, or the like may be mixed as needed.

Examples of the mode of usage of the kit include (1) a method in which the first IgG half-molecule and the second IgG half-molecule are mixed in advance and then administered, and (2) a method in which the first IgG half-molecule and the second IgG half-molecule are separately administered. Examples of the method (2) in which the first IgG half-molecule and the second IgG half-molecule are separately administered include a method in which the first IgG half-molecule and the second IgG half-molecule are administered simultaneously or sequentially. The amounts and ratios of the first IgG half-molecule and the second IgG half-molecule can be appropriately adjusted.

Further, as one aspect of the present invention, a first IgG half-molecule to be used for producing an antibody composition against a first antigen and a second antigen that are different from each other, the antibody composition composed of the first IgG half-molecule and a second IgG half-molecule is exemplified.

As another aspect of the present invention, a second IgG half-molecule to be used for producing an antibody composition against a first antigen and a second antigen that are different from each other, the antibody composition composed of a first IgG half-molecule and the second IgG half-molecule is exemplified.

As one aspect of the present invention, a first IgG half-molecule to be used in combination with a second IgG half-molecule is exemplified. Further, as one aspect of the present invention, a second IgG half-molecule to be used in combination with a first IgG half-molecule is exemplified. Here, the use of first IgG half-molecule and the second IgG half-molecule "in combination" means that the first IgG half-molecule and the second IgG half-molecule are administered simultaneously or sequentially so as to form an antibody composition against a first antigen and a second antigen that are different from each other, the antibody composition composed of the first IgG half-molecule and the second IgG half-molecule.

Hereinafter, the present invention will be more specifically described by way of Examples, but the present invention is not limited to the following Examples.

EXAMPLES

The present inventors conceived that in a constant region of an antibody molecule of the present invention, the following elements that are different from a normal human IgG1 antibody are needed.

1) As shown in Fig. 4, it is a mixture of antibody half-molecules against different antigen molecules X and Y, that is, it is an "HL molecule" in which a covalent bond formed by an inter-H chain disulfide bond is not present in a hinge domain.
2) As shown in Fig. 3, the HL molecules against each of the antigen molecules X and Y bind to the surface of a target cell expressing both X and Y and thereafter are associated with each other to form an H2L2 molecule in the same manner as normal IgG and constitute a CD16a-binding domain to cause an antibody activity (for example, an ADCC activity).
3) As shown in Fig. 3, a CD16a-binding domain is not constituted even if the HL molecules against X or Y are associated with each other on a cell surface so that an antibody activity is not caused against a cell expressing only a single antigen molecule.

In this Example, with respect to the above 1), an attempt was made by substituting cysteine of a hinge domain with alanine.

It is known by X-ray crystallography that Fc of human IgG1 and CD16a show an asymmetric binding mode (Nature 2000; 406: 267-73, J Biol Chem 2001; 276: 16469-77, Mizushima T, Genes Cells 2011; 16: 1071-80). Therefore, the present inventors conceived that the below-mentioned amino acid alteration utilizing such an asymmetric binding mode is introduced into the CH2 domain of the above-mentioned HL molecules for achieving the above 2) and 3).

As shown in Fig. 5, CD16a comes into contact with two CH2 domains in Fc at different sites, respectively. The two CH2 domains are tentatively named CH2-A and CH2-B, and an interacting site with CD16a on CH2-A is defined as a region 1, and an interacting site with CD16a on CH2-B is defined as a region 2.

As the region 1, L235, G236, G237, P238, S239, D265, V266, S267, H268, E269, E294, Q295, Y296, N297, S298, T299, R301, N325, A327, 1332, and the like are known. Further, as the region 2, L235, G236, G237, K326, A327, L328, P329, A330, and the like are known.

As shown in Fig. 5, due to the symmetry of the structure of Fc, on the opposite side to the actual binding domain where Fc and CD16a are bound, another binding domain that is composed of the region 2 of CH2-A and the region 1 of CH2-B, and is not actually used for binding to CD16a is present.

As shown in Fig. 6, the region 2 of CH2-A and the region 1 of CH2-B that are not used for binding to the CD16a are "disrupted" by introducing an amino acid alteration into each region. In that case, when HL molecules (X) having such altered CH2-A or HL molecules (Y) having such altered CH2-B are homo-associated to form an H2L2 molecule (XX or YY), only the region 1 or only the region 2 is present. Therefore, it is considered that to such a homo assembly, CD16a may not be able to bind with sufficient affinity. On the other hand, it is considered that an H2L2 molecule (XY) in which the HL molecules having such altered CH2-A and altered CH2-B, respectively, as a constituent element are hetero-associated may satisfy the above conditions 2) and 3).

[Example 1]

Production of human IgG1 anti-CCR6 antibody in which CD16a binding was "disrupted"

In order to specify a site on CH2 capable of attenuating the binding of CD16a, an antibody in which the above-mentioned region 1 or region 2 was "disrupted" by an amino acid alteration was produced. That is, as shown in Fig. 7, an amino acid residue at a candidate site was altered on a format of normal human IgG1.

In that case, for expressing human IgG1 as a recombinant antibody, two H chains are encoded by the same gene on an expression vector, and an amino acid residue is simultaneously altered in both without distinction between CH2-A and CH2-B, and the CD16a-binding site is simultaneously disrupted at two sites on both sides, resulting in searching for an altered site for decreasing an ADCC activity.

Incidentally, in this Example, unless otherwise stated, all the antibody molecules were produced such that $\alpha$1,6-fucose of the N-linked sugar chain that is bound to asparagine at position 297 of the H chain is completely removed so as to enhance an ADCC activity. Therefore, as the host cells for expressing the antibody, fucosyltransferase (FUT8) knockout CHO cells (WO 2005/035586 and WO 02/31140) were used.

(1) Production of expression vector for human IgG1 anti-CCR6 antibody in which CD16a binding was "disrupted"

In Figs. 5 and 6, a schematic diagram of the "region 1" and the "region 2" on the CH2 domain is shown. An expression vector for a human IgG1 anti-human CCR6 antibody (WO 2013/005649) in which an amino acid alteration

was introduced with respect to amino acid sites P238, P265, and S267 binding to CD16a in the "region 1" on the CH2 domain and amino acid sites K326, L328, and P329 binding to CD16a in the "region 2" on the CH2 domain for "disrupting" the binding to CD16a as shown in Fig. 6 was constructed. The gene sequences and the amino acid sequences of the antibodies used for the construction are shown in Table 2.

[Table 2]

| Sequence name | Gene sequence | Amino acid sequence |
|---|---|---|
| CCR6 (KG1684) VL | SEQ ID NO: 1 | SEQ ID NO: 2 |
| CCR6 (KG1684) VH | SEQ ID NO: 3 | SEQ ID NO: 4 |
| IgG1_CK | SEQ ID NO: 5 | SEQ ID NO: 6 |
| IgG1_CH | SEQ ID NO: 7 | SEQ ID NO: 8 |
| IgG1_P238A CH | SEQ ID NO: 9 | SEQ ID NO: 10 |
| IgG1_D265A CH | SEQ ID NO: 11 | SEQ ID NO: 12 |
| IgG1_S267L CH | SEQ ID NO: 13 | SEQ ID NO: 14 |
| IgG1_P238A/D265A CH | SEQ ID NO: 15 | SEQ ID NO: 16 |
| IgG1_P238A/S267L CH | SEQ ID NO: 17 | SEQ ID NO: 18 |
| IgG1_D265A/S267L CH | SEQ ID NO: 19 | SEQ ID NO: 20 |
| IgG1_P238A/D265A/S267L CH | SEQ ID NO: 21 | SEQ ID NO: 22 |
| IgG1_K326W CH | SEQ ID NO: 23 | SEQ ID NO: 24 |
| IgG1_L328V CH | SEQ ID NO: 25 | SEQ ID NO: 26 |
| IgG1_P329Y CH | SEQ ID NO: 27 | SEQ ID NO: 28 |
| IgG1_K326W/L328V CH | SEQ ID NO: 29 | SEQ ID NO: 30 |
| IgG1_K326W/P329Y CH | SEQ ID NO: 31 | SEQ ID NO: 32 |
| IgG1_L328V/P329Y CH | SEQ ID NO: 33 | SEQ ID NO: 34 |
| IgG1_K326W/L328V/P329Y CH | SEQ ID NO: 35 | SEQ ID NO: 36 |

A PCR reaction was carried out in accordance with a package insert of PrimeSTAR Max DNA Polymerase (Takara Bio) using a human IgG1 anti-human CCR6 antibody expression vector pCI-IgG1_KG1684 composed of a gene sequence represented by SEQ ID NO: 1, 3, 5, or 7 as a template, and also using PrimeSTAR Max DNA Polymerase and primers (Sigma-oligo) into which an altered site was introduced.

In the PCR reaction, GeneAmp PCR System 9700 (Applied Biosystems) was used, and after thermal denaturation at 98°C for 1 minute, a reaction at 98°C for 10 seconds, at 58°C for 5 seconds, and at 72°C for 5 seconds was performed 30 cycles. The reaction solution was subjected to electrophoresis using 0.8% agarose gel, and an amplified fragment was recovered using QIAquick Gel Extraction Kit (Qiagen). A ligation reaction with a plasmid pCI vector (Promega) was carried out using In-Fusion HD Cloning Kit (Clontech), and Escherichia coli DH5α competent cells (Takara Bio) were transformed using the reaction solution.

Each plasmid DNA was prepared from the thus obtained transformant clones and allowed to react using Big Dye Terminator Cycle Sequencing Kit v3.1 (Applied Biosystems) in accordance with the instruction attached thereto, and then the base sequence of the DNA inserted into the plasmid was analyzed by a DNA sequencer ABI PRISM 3700 DNA Analyzer of the same company.

(2) Expression of human IgG1 anti-CCR6 antibody in which CD16a binding was "disrupted"

The expression vector produced in (1) was introduced into host cells by the following method. As the host cells, FUT8 knockout CHO cells (WO 2005/035586 and WO 02/31140) were used. A method for introducing a plasmid was carried out in accordance with the instruction attached thereto.

The amount of the culture solution was set to 200 mL, and the culture was carried out for 5 days under the set conditions of 37°C, 5% $CO_2$, and 125 rpm. After culture, the cell suspension was centrifuged, and passed through a 0.2 μm filter (Thermo Scientific), whereby the culture supernatant containing an altered antibody was recovered.

The name of a purified antibody sample of each antibody is shown in Table 3.

[Table 3]

| Purified antibody (name) | Region 1 of CH2 | Region 2 of CH2 |
|---|---|---|
| IgG1 | - | - |
| IgG1_P238A | P238A | P238A |
| IgG1_D265A | D265A | D265A |
| IgG1_S267L | S267L | S267L |
| IgG1_P238A/D265A | P238A/D265A | |
| IgG1_P238A/S267L | P238A/S267L | |
| IgG1_D265A/S267L | D265A/S267L | |
| IgG1_P238A/D265A/S267L | P238A/D265A/S267L | - |
| IgG1_K326W | - | K326W |
| IgG1_L328V | - | L328V |
| IgG1_P329Y | - | P329Y |
| IgG1_K326W/L328V | - | K326W/L328V |
| IgG1_K326W/P329Y | - | K326W/P329Y |
| IgG1_L328V/P329Y | - | L328V/P329Y |
| IgG1_K326W/L328V/P329Y | - | K326W/L328V/P329Y |

(3) Purification of human IgG1 anti-CCR6 antibody in which CD16a binding was "disrupted"

The altered antibody was purified by affinity purification using MabSelect SuRe (GE Healthcare) shown below. After a resin was equilibrated with PBS, the culture supernatant obtained in (2) was loaded, followed by washing twice with PBS. After washing, the antibody was eluted using an elution buffer (100 mM citric acid, pH 3.5), followed by neutralization by adding 1/10 amount of a neutralization buffer (2 M Tris-HCl, pH 8.0).

Subsequently, concentration and replacement with buffer (10 mM citric acid, 150 mM NaCl, pH 6.0) by ultrafiltration were carried out using Amicon Ultra-4 Centerifugal Filter Units (Millipore), and an absorbance (A280) at 280 nM was measured using NanoDrop 8000 (Thermo Scientific), whereby the concentration measurement and preparation of an antibody solution were carried out.

(4) Evaluation of purification degree by SDS-PAGE of human IgG1 anti-CCR6 antibody in which CD16a binding was "disrupted"

In order to evaluate the purification degree of the purified samples of various types of CD16-binding-deficient human IgG1 anti-CCR6 antibodies, by using about 1 μg of each antibody purified sample, SDS denaturing polyacrylamide gel electrophoresis (hereinafter referred to as SDS-PAGE) was carried out according to a known method [Nature, 227, 680 (1970)].

As a result, under reducing conditions, in the various types of CD16-binding-deficient human IgG1 anti-CCR6 antibodies, in the same manner as the normal IgG1 type, a band was observed in the vicinity of about 50 kilodaltons (hereinafter referred to as kDa) for the H chain and in the vicinity of about 25 kDa for the L chain. Further, under non-reducing conditions, a band was observed in the vicinity of about 150 kDa, and therefore, it was confirmed that the produced anti-CCR6 domain-exchanged antibodies are constituted by the target H chain and L chain.

From the above results, it was confirmed that in the purified samples of various types of CD16-binding-deficient human IgG1 anti-CCR6 antibodies obtained in the section 3 of this Example, target IgG molecules each constituted by the H chain and the L chain are contained at a sufficient ratio.

(5) ADCC activity of human IgG1 anti-CCR6 antibody in which CD16a-binding domain was "disrupted"

As an effector cell, an NK-92/CD16 transfectant obtained by transfecting NK-92 (ATCC) that is an NK cell line

with human CD16a (Val type) to stably express it was used, and as a target cell, a human CCR6/CHO transfectant was used. The respective cultured cells were recovered and counted, and then prepared using RPMI medium (RPMI 1640 medium that was supplemented with 5% FBS/1% PS, and did not contain phenol red) at a cell density of $8 \times 10^5$ cells/mL and $2 \times 10^5$ cells/mL, respectively.

After the antibody solution was dispensed into a 96-well plate at 50 μL/well with a continuous dispenser, and thereafter, the target cells were dispensed at 50 μL/well. The effector cells were dispensed at 50 μL/well, followed by centrifugation at 1800 rpm for 2 minutes, and it was confirmed that the cells were evenly seeded.

After the plate was left to stand at 37°C for 3 hours and 15 minutes in a $CO_2$ incubator, 10/1 amount of a solubilizing solution was dispensed into a Total control, and the plate was left to stand at 37°C for 45 minutes. After centrifugation at 1800 rpm for 2 minutes, 50 μL of the supernatant was dispensed into an ELISA plate. A substrate (powder) was dissolved in 12 mL of a suspension buffer to prepare a coloring solution, and the coloring solution was applied at 50 μL/well to cause a reaction. A stopping solution was added at 50 μL/well, and an absorbance (A450) was measured using a plate reader.

Note that as a kit for ADCC measurement, CytoTox 96(R) Non-Radioactive Cytotoxicity Assay (Promega) was used. An ADCC activity (%) was calculated using the following formula.

$$\text{ADCC activity (\%)} = 100 \times (S - E - T) / (Max - T)$$

S = absorbance of sample reaction well - absorbance of culture medium well
E = absorbance of effector well - absorbance of culture medium well
T = absorbance of target well - absorbance of culture medium well
Max = 100% reaction well - 100% reaction control well

The results are shown in Fig. 8. As shown in Fig. 8, it was confirmed that in the region 1 of CH2, in variants composed of IgG1_D265A, IgG1_P238A/S267L, or IgG1_D265A/S267L, the ADCC activity is significantly decreased against the target cells.

Further, it was confirmed that in the region 2 of CH2, in variants composed of IgG1_P329Y, IgG1_K326W/P329Y, IgG1_L328V/P329Y, or IgG1_K326W/L328V/P329Y, the ADCC activity is significantly decreased, and it was found that P329Y alteration is included in any of the variants.

From the above results, it was revealed that the CD16a-binding site can be disrupted by introducing an amino acid alteration of D265A or P238A/S267L in the "region 1" on the CH2 domain, and by introducing an amino acid alteration of at least P329Y in the "region 2" on the CH2 domain.

[Example 2]

Production of human IgG1 anti-CCR6 monovalent antibody into which CD16a-binding asymmetric alteration was introduced

As an example of an amino acid sequence capable of disrupting an asymmetric binding site of CD16a to Fc in Example 1, D265A or P238A/S267L derived from the "region 1" was selected in CH2-A, and P329Y derived from the "region 2" was selected in CH2-B.

In this section, in order to evaluate an ADCC activity by asymmetrically introducing the above-mentioned alterations in only CH2-A and CH2-B, respectively, "a monovalent antibody" in which two molecules of H chain are encoded by individual genes and expressed, and these can be hetero-associated (WO 2011/108502) was used as a basic skeleton. In Fig. 9, a schematic diagram of one embodiment of such a monovalent antibody is shown. In Fig. 9, the above-mentioned hetero assembly is "asymmetric variant #1".

(1) Production of CD16a-binding asymmetrically altered monovalent antibody expression vector

An expression vector for a human IgG1 anti-CCR6 monovalent antibody into which CH2-A (D265A or P238A/S267L) and CH2-B (P329Y) were asymmetrically or symmetrically introduced (WO 2011/108502) was constructed.

A PCR reaction was carried out in accordance with a package insert of PrimeSTAR Max DNA Polymerase (Takara Bio) using the human IgG1 anti-CCR6 monovalent antibody expression vector pCI-mvG1_KG1684 as a template, and also using PrimeSTAR Max DNA Polymerase and primers (Sigma-oligo) into which an altered site was introduced.

In the PCR reaction, GeneAmp PCR System 9700 (Applied Biosystems) was used, and after thermal denaturation at 98°C for 1 minute, a reaction at 98°C for 10 seconds, at 58°C for 5 seconds, and at 72°C for 5 seconds was

performed 30 cycles.

The reaction solution was subjected to electrophoresis using 0.8% agarose gel, and an amplified fragment was recovered using QIAquick Gel Extraction Kit (Qiagen). A ligation reaction with a plasmid pCI vector (Promega) was carried out using In-Fusion HD Cloning Kit (Clontech), and Escherichia coli DH5α competent cells (Takara Bio) were transformed using the reaction solution.

Each plasmid DNA was prepared from the thus obtained transformant clones and allowed to react using Big Dye Terminator Cycle Sequencing Kit v3.1 (Applied Biosystems) in accordance with the instruction attached thereto, and then the base sequence of the DNA inserted into the plasmid was analyzed by a DNA sequencer ABI PRISM 3700 DNA Analyzer of the same company.

(2) Expression of CD16a-binding asymmetrically altered monovalent antibody

The expression of the antibody was carried out in the same manner as in the section 2 of Example 1, and the culture supernatant containing the antibody was recovered. Each purified antibody sample and an altered site thereof are shown in Table 4. Note that an amino acid residue substitution at the altered site was carried out in the same manner as in Example 1.

[Table 4]

| Purified antibody (name) | CH2-A (Region 1, Region 2) | CH2-B (Region 1, Region 2) |
|---|---|---|
| wild type | - | - |
| asymmetric variant #1 | D265A | P329Y |
| asymmetric variant #2 | P329Y | D265A |
| asymmetric variant #3 | P238A/S267L | P329Y |
| asymmetric variant #4 | P329Y | P238A/S267L |
| symmetric variant #1 | D265A | D265A |
| symmetric variant #2 | P329Y | P329Y |
| symmetric variant #3 | P238A/S267L | P238A/S267L |
| symmetric variant #4 | D265A, P329Y | D265A, P329Y |
| symmetric variant #5 | P238A/S267L, P329Y | P238A/S267L, P329Y |

(3) Purification of CD16a-binding asymmetrically altered monovalent antibody

The purification of the antibody was carried out in the same manner as in the section 3 of Example 1. As the elution buffer, 100 mM citric acid, pH 3.9 was used. Thereafter, a monomer fraction was fractionated from the antibody solution using AKTA FPLC (GE Healthcare) and Superdex High-performance Column (GE Healthcare). By performing filter sterilization with a membrane filter (Millex-GV, Millipore) with a pore diameter of 0.22 μm of an AKTA system, a purified antibody was obtained. An absorbance (A280) at 280 nm was measured using NanoDrop 8000 (Thermo Scientific).

(4) Evaluation of purification degree by SDS-PAGE of human IgG1 anti-CCR6 monovalent antibody into which CD16a-binding asymmetric alteration was introduced

In order to evaluate the purification degree of the purified samples of human IgG1 anti-CCR6 monovalent antibodies into which various types of CD16a-binding asymmetric alterations were introduced, by using about 1 μg of each antibody purified sample, SDS-PAGE was carried out.

As a result, under reducing conditions, in all the variants, a band was observed in the vicinity of about 50 kD for the H chain and the Fc-fused L-chain in the same manner as the wild-type monovalent antibody. Further, under non-reducing conditions, a band was observed in the vicinity of about 100 kDa, and therefore, it was confirmed that the produced asymmetric variants and symmetric variants are constituted by the target H chain and L chain.

From the above results, it was confirmed that in the purified samples of human IgG1 anti-CCR6 monovalent antibodies into which various types of CD16a-binding asymmetric alterations were introduced obtained in the section 3 of this Example, target monovalent antibody molecules each constituted by the H chain and the Fc-fused L chain are

contained at a sufficient ratio.

(5) ADCC activity of CD16a-binding asymmetrically altered monovalent antibody

An ADCC activity was measured in the same manner as the section 5 of Example 1. The results are shown in Fig. 10. As shown in Fig. 10, it was confirmed that the monovalent antibodies (asymmetric variants #1 to 4) into which a CD16a-binding asymmetric alteration was introduced damage the target cells in an antibody concentration-dependent manner. On the other hand, it was confirmed that the monovalent antibodies (symmetric variants #1 to #5) into which a CD16a-binding symmetric alteration was introduced do not show an ADCC activity against the target cells.
From the above results, it was confirmed that a monovalent antibody into which a CD16a-binding asymmetric alteration was introduced maintains binding affinity for human CD16a and can exhibit an ADCC activity against the target cells.
On the other hand, when the same alteration is introduced into both CH2 domains, an ADCC activity is lost, and therefore, it was confirmed that an ADCC activity is not induced when antibodies having the same altered CH2 are homophilically associated on a cell surface, and altered CH2 domains for constituting an HL antibody that exhibits an ADCC activity only when it is hetero-associated with two antigens on a cell surface aimed at by the present invention were found.

[Example 3]

In this Example, it was verified whether an ADCC activity can be induced selectively for a target cell coexpressing two types of antigens by mounting altered CH2 that were obtained in Examples 1 and 2 and can induce an ADCC activity only when they are hetero-associated on two types of antibody half-molecules (HL molecules) against different antigens.
An interaction between CH3 domains has been reported in J. Immunol. 2011; 187: 3238-3246. The interaction ($K_D$ value) of the CH3 domains of human IgG1 is $3.0 \times 10^{-9}$ M, the $K_D$ value of the CH3 domains of human IgG4 is $4.8 \times 10^{-8}$ M, so that the interaction between CH3 domains of human IgG4 is about 6 to 7 times weaker than that of IgG1.
The production of an antibody half-molecule was attempted by utilizing this property and an amino acid alteration (C226A/C229A: AA) for cleaving an inter-H chain disulfide bond in a hinge domain. A domain-exchanged antibody (hereinafter referred to as IgG1 114_AA type) in which for the H chain constant region of a half-molecule, human IgG1 was used as a basic skeleton, and only the CH3 domain was exchanged to a human IgG4 sequence, and further, an amino acid alteration (AA) was added to the hinge domain was used.
In addition, in order to enhance an ADCC activity, a known amino acid alteration for enhancing an ADCC activity was made in the CH2 domain, and further, $\alpha$1,6-fucose of the N-linked sugar chain that is bound to Asn297 was removed, and finally, a CD16a-binding asymmetric amino acid alteration was introduced. In Fig. 11, a schematic diagram of an IgG1114_AA_AAA_D265A (/P329Y)-type IgG half-molecule is shown. As two types of model antigens, CD4 and CD70 were used.
An anti-CD4 antibody (J. Immunol. 1992; 149: 1779-1787) and an anti-CD70 antibody (WO 2007/03637) in which the above-mentioned alterations were combined were produced according to the following procedure. The anti-CD4 antibody and the anti-CD70 antibody having a heavy chain constant region constituted by an amino acid sequence in which a known amino acid alteration for enhancing an ADCC activity (S298A/E333A/K334A: AAA) alteration and a CD16a-binding asymmetric amino acid alteration (D265A or P329Y) were introduced into the IgG1114_AA type are referred to as an IgG1114_AA_AAA_D265A(/P329Y)-type anti-CD4 half-molecule, and an IgG1114_AA_AAA_D265A (/P329Y)-type anti-CD70 half-molecule, respectively.
A subclass from which each domain of the various types of designed anti-CD4 and CD70 half-molecules is derived, and the correspondence of the amino acid sequence of the heavy chain constant region are shown in Table 5. Further, the gene sequences and the amino acid sequences of antibodies against CD4 and CD70 used are shown in Table 6.

[Table 5]

| Structure name | CH1 | Hinge | CH2 | CH3 | Gene and amino acid sequences |
|---|---|---|---|---|---|
| IgG1114_AA_AAA_D265A | G1 | G1(AA) | G1(AAA_D265A) | G4 | SEQ ID NOS: 37 and 38 |
| IgG1114_AA_AAA_P329Y | G1 | G1(AA) | G1(AAA_P329Y) | G4 | SEQ ID NOS: 39 and 40 |

[Table 6]

| Sequence name | Gene sequence | Amino acid sequence |
|---|---|---|
| CD4 (ibalizumab) VL | SEQ ID NO: 41 | SEQ ID NO: 42 |
| CD4 (ibalizumab) VH | SEQ ID NO: 43 | SEQ ID NO: 44 |
| CD70 (2H5) VL | SEQ ID NO: 45 | SEQ ID NO: 46 |
| CD70 (2H5) VH | SEQ ID NO: 47 | SEQ ID NO: 48 |

(1) Production of expression vector for anti-CD4 half-molecule and anti-CD70 half-molecule

A DNA fragment of about 9 kbp was cut using restriction enzymes NheI and NotI from a human IgG1 anti-CD4 antibody expression vector pCI-IgG1_CD4 (ibalizumab) (SEQ ID NO: 5, 7, 41, or 43) or a human IgG1 anti-CD70 antibody expression vector pCI-IgG1_CD70 (2H5) (SEQ ID NO: 5, 7, 45, or 47), and purified.

A ligation reaction of the purified DNA fragment with an artificial synthetic gene composed of the CH1 domain, the hinge domain (to which a C226A/C229A alteration was added) and the CH2 domain (to which a S298A/K333A/E334A alteration and a D265A or P329Y alteration were added), of a human IgG1 antibody, and the CH3 domain of a human IgG4 antibody was carried out using In-Fusion HD Cloning Kit (Clontech), and Escherichia coli DH5$\alpha$ competent cells (Takara Bio) were transformed using the reaction solution. Each plasmid DNA was prepared from the thus obtained transformant clones, and the base sequence of the DNA was analyzed by Fasmac.

(2) Expression of various types of anti-CD4 IgG1 antibodies and anti-CD70 IgG1 antibodies and half-molecules of these antibodies

The expression of the antibody was carried out in the same manner as the section 2 of Example 1, and the culture supernatant containing the antibody was recovered.

The names of the purified antibody samples are shown in Table 7.

[Table 7]

| Purified antibody (name) | Structure of antibody |
|---|---|
| CD4 antibody | IgG1 |
| CD70 antibody | IgG1 |
| CD4 antibody half-molecule 1 | IgG1114_AA_AAA_D265A |
| CD4 antibody half-molecule 2 | IG1114_AA_AAA_P329Y |
| CD70 antibody half-molecule 1 | IgG1114_AA_AAA_D265A |
| CD70 antibody half-molecule 2 | IgG1114_AA_AAA_P329Y |

(3) Purification of anti-CD4 half-molecule and anti-CD70 half-molecule

The purification of the antibody was carried out in the same manner as in the section 3 of Example 1, and the concentration measurement and preparation of an antibody solution were carried out.

(4) Evaluation of purification degree by SDS-PAGE of anti-CD4 half-molecule and anti-CD70 half-molecule

In order to evaluate the purification degree of the prepared various types of antibody samples, by using about 1 $\mu$g of each antibody purified sample, SDS-PAGE was carried out. The results are shown in Fig. 12.

As shown in Fig. 12, in all the purified antibodies, under reducing conditions, a band was observed in the vicinity of about 50 kDa for the H chain and in the vicinity of about 25 kDa for the L chain. Further, under non-reducing conditions, a band was observed in the vicinity of about 150 kDa for the anti-CD4 IgG1 antibody and the anti-CD70 IgG1 antibody, and in the vicinity of about 75 kDa for the anti-CD4 half-molecule and the anti-CD70 half-molecule, and therefore, it was confirmed that the produced CD4 and CD70 IgG1 antibodies are each an antibody structure constituted by the target H chain and L chain, and the anti-CD4 half-molecule and the anti-CD70 half-molecule are each constituted by the target H chain and L chain, and have an antibody structure that easily form a half-molecule.

From the above results, it was confirmed that in the purified samples of anti-CD4 half-molecule and the anti-CD70 half-molecule obtained in the section 3 of this Example, target antibody molecules are contained at a sufficient ratio.

(5) ADCC activity of anti-CD4 IgG1 antibody and anti-CD70 IgG1, and half-molecules of these antibodies against CD4/CD70 double-positive cells, and CD4 and CD70 single-positive cells

An ADCC activity was measured in the same manner as the section 5 of Example 1. As for the target cell, a CD4/EL-4 transfectant was used as a CD4 single-positive cell, MT-1 was used as a CD70 single-positive cell, and TL-Om1 was used as a CD4/CD70 double-positive cell. The results of measuring the expression levels of CD40 and CD70 in these cells using a flow cytometer are shown in Fig. 13.

As shown in Fig. 13, it was confirmed that the target antigen was expressed in the cells. Further, the results of evaluating the ADCC activity are shown in Fig. 14. As positive control antibodies, normal IgG1-type anti-CD4 and CD70 antibodies were used.

As shown in Fig. 14, it was demonstrated as expected that the anti-CD4 antibody exhibits an ADCC activity against the CD4/EL-4 transfectant, the anti-CD70 antibody exhibits an ADCC activity against MT-1, and the anti-CD4 antibody and the anti-CD70 antibody exhibit an ADCC activity against TL-Om1.

On the other hand, an antibody solution obtained by mixing the anti-CD4 antibody half-molecule 1 and the anti-CD70 antibody half-molecule 2 or an antibody solution obtained by mixing the anti-CD4 antibody half-molecule 2 and the anti-CD70 antibody half-molecule 1 does not exhibit an ADCC activity against the CD4 and CD70 single-positive cells, but specifically exhibits an ADCC activity only against the CD4/CD70 double-positive cells.

[Example 4]

It was verified whether an ADCC activity can be induced selectively for a target cell coexpressing two types of antigens by mounting altered CH2 in which CD16a binding was disrupted by substitution at each of position 235, position 239, position 265, position 267, position 269, position 296, position 298, position 299, and position 327 numbered according to the EU index in the first CD16a-binding domain, and at position 326, position 328, position 329, and position 330 numbered according to the EU index in the second CD16a-binding domain on the half-molecules (HL molecules) of two types of antibodies against different antigens.

Designed various types of anti-CD4 half-molecules and CD70 half-molecules were prepared in the same manner as in Example 3, and an ADCC activity was evaluated. An antibody solution obtained by mixing the anti-CD4 half-molecule and the anti-CD70 half-molecule was added to give 1 μg/mL. Similarly, a normal IgG1-type anti-CD4 antibody and a normal IgG1-type anti-CD70 antibody used as the positive control antibodies were also added to give 1 μg/mL. A subclass derived from each domain of the designed various types of anti-CD4 half-molecules and CD70 half-molecules, and correspondence to the amino acid sequence of a heavy chain constant region are shown in Table 8.

[Table 8]

| Purified antibody (name) | Structure of antibody | SEQID NO | |
|---|---|---|---|
| CD4 antibody half-molecule 3 | IgG1114_AA_AAA_S239R CH | 49 | gene sequence |
| CD4 antibody half-molecule 3 | IgG1114_AA_AAA_S239R CH | 50 | amino acid sequence |
| CD4 antibody half-molecule 4 | IgG1114_AA_AAA_D265N CH | 51 | gene sequence |
| CD4 antibody half-molecule 4 | IgG1114_AA_AAA_D265NCH | 52 | amino acid sequence |
| CD4 antibody half-molecule 5 | IgG1114_AA_AAA_D265E CH | 53 | gene sequence |
| CD4 antibody half-molecule 5 | IgG1114_AA_AAA_D265E CH | 54 | amino acid sequence |
| CD4 antibody half-molecule 6 | IgG1114_AA_AAA_S267K CH | 55 | gene sequence |
| CD4 antibody half-molecule 6 | IgG1114_AA_AAA_S267K CH | 56 | amino acid sequence |
| CD4 antibody half-molecule 7 | IgG1114_AA_AAA_E269P CH | 57 | gene sequence |
| CD4 antibody half-molecule 7 | IgG1114_AA_AAA_E269P CH | 58 | amino acid sequence |
| CD4 antibody half-molecule 8 | IgG1114_AA_AAA_Y296P CH | 59 | gene sequence |
| CD4 antibody half-molecule 8 | IgG1114_AA_AAA_Y296P CH | 60 | amino acid sequence |
| CD4 antibody half-molecule 9 | IgG1114_AA_AAA_S298E CH | 61 | gene sequence |

(continued)

| Purified antibody (name) | Structure of antibody | SEQID NO | |
|---|---|---|---|
| CD4 antibody half-molecule 9 | IgG1114_AA_AAA_S298E CH | 62 | amino acid sequence |
| CD4 antibody half-molecule 10 | IgG1114_AA_AAA_T299A CH | 63 | gene sequence |
| CD4 antibody half-molecule 10 | IgG1114_AA_AAA_T299ACH | 64 | amino acid sequence |
| CD4 antibody half-molecule 11 | IgG1114_AA_AAA_L235R CH | 65 | gene sequence |
| CD4 antibody half-molecule 11 | IgG1114_AA_AAA_L235R CH | 66 | amino acid sequence |
| CD4 antibody half-molecule 12 | IgG1114_AA_AAA_A327I CH | 67 | gene sequence |
| CD4 antibody half-molecule 12 | IgG1114_AA_AAA_A327I CH | 68 | amino acid sequence |
| CD70 antibody half-molecule 3 | IgG1114_AA_AAA_K326G CH | 69 | gene sequence |
| CD70 antibody half-molecule 3 | IgG1114_AA_AAA_K326G CH | 70 | amino acid sequence |
| CD70 antibody half-molecule 4 | IgG1114_AA_AAA_L328R CH | 71 | gene sequence |
| CD70 antibody half-molecule 4 | IgG1114_AA_AAA_L328R CH | 72 | amino acid sequence |
| CD70 antibody half-molecule 5 | IgG1114_AA_AAA_P329K CH | 73 | gene sequence |
| CD70 antibody half-molecule 5 | IgG1114_AA_AAA_P329K CH | 74 | amino acid sequence |
| CD70 antibody half-molecule 6 | IgG1114_AA_AAA_P329W CH | 75 | gene sequence |
| CD70 antibody half-molecule 6 | IgG1114_AA_AAA_P329W CH | 76 | amino acid sequence |
| CD70 antibody half-molecule 7 | IgG1114_AA_AAA_A330P CH | 77 | gene sequence |
| CD70 antibody half-molecule 7 | IgG1114_AA_AAA_A330P CH | 78 | amino acid sequence |

As shown in Figs. 15A to 15H, it was demonstrated that the antibody solution obtained by mixing the anti-CD4 half-molecule mounted with CH2 in which the first CD16a-binding domain was altered and the anti-CD70 half-molecule mounted with CH2 in which the second CD16a-binding domain was altered does not exhibit an ADCC activity against the CD4 and CD70 single-positive cells, but specifically exhibits an ADCC activity only against the CD4/CD70 double-positive cells.

The present invention has been described in detail using the specific aspects, but it is obvious to those skilled in the art that various changes and modifications may be made without departing from the spirit and scope of the present invention. Note that the present application is based on the Japanese patent application (Japanese Patent Application No. 2018-185367) filed on September 28, 2018, which is incorporated by reference in its entirety.

SEQUENCE LISTING FREE TEXT

SEQ ID NO: 1: base sequence of human CCR6 (KG1684) VL
SEQ ID NO: 2: amino acid sequence of human CCR6 (KG1684) VL
SEQ ID NO: 3: base sequence of human CCR6 (KG1684) VH
SEQ ID NO: 4: amino acid sequence of human CCR6 (KG1684) VH
SEQ ID NO: 5: base sequence of human IgG1 CK
SEQ ID NO: 6: amino acid sequence of human IgG1 CK
SEQ ID NO: 7: base sequence of human IgG1 CH
SEQ ID NO: 8: amino acid sequence of human IgG1 CH
SEQ ID NO: 9: base sequence of human IgG1_P238A CH
SEQ ID NO: 10: amino acid sequence of human IgG1_P238A CH
SEQ ID NO: 11: base sequence of human IgG1_D265A CH
SEQ ID NO: 12: amino acid sequence of human IgG1_D265A CH
SEQ ID NO: 13: base sequence of human IgG1_S267L CH
SEQ ID NO: 14: amino acid sequence of human IgG1_S267L CH
SEQ ID NO: 15: base sequence of human IgG1_P238A/D265A CH

SEQ ID NO: 16: amino acid sequence of human IgG1_P238A/D265A CH
SEQ ID NO: 17: base sequence of human IgG1_P238A/S267L CH
SEQ ID NO: 18: amino acid sequence of human IgG1_P238A/S267L CH
SEQ ID NO: 19: base sequence of human IgG1_D265A/S267L CH
SEQ ID NO: 20: amino acid sequence of human IgG1_D265A/S267L CH
SEQ ID NO: 21: base sequence of human IgG1_P238A/D265A/S267L CH
SEQ ID NO: 22: amino acid sequence of human IgG1_P238A/D265A/S267L CH
SEQ ID NO: 23: base sequence of human IgG1_K326W CH
SEQ ID NO: 24: amino acid sequence of human IgG1_K326W CH
SEQ ID NO: 25: base sequence of human IgG1_L328V CH
SEQ ID NO: 26: amino acid sequence of human IgG1_L328V CH
SEQ ID NO: 27: base sequence of human IgG1_P329Y CH
SEQ ID NO: 28: amino acid sequence of human IgG1_P329Y CH
SEQ ID NO: 29: base sequence of human IgG1_K326W/L328V CH
SEQ ID NO: 30: amino acid sequence of human IgG1_K326W/L328V CH
SEQ ID NO: 31: base sequence of human IgG1_K326W/P329Y CH
SEQ ID NO: 32: amino acid sequence of human IgG1_K326W/P329Y CH
SEQ ID NO: 33: base sequence of human IgG1_L328V/P329Y CH
SEQ ID NO: 34: amino acid sequence of human IgG1_L328V/P329Y CH
SEQ ID NO: 35: base sequence of human IgG1_K326W/L328V/P329Y CH
SEQ ID NO: 36: amino acid sequence of human IgG1_K326W/L328V/P329Y CH
SEQ ID NO: 37: base sequence of human IgG1114_AA_AAA_D265A CH
SEQ ID NO: 38: amino acid sequence of human IgG1114_AA_AAA_D265A CH
SEQ ID NO: 39: base sequence of human IgG1114_AA_AAA_P329Y CH
SEQ ID NO: 40: amino acid sequence of human IgG1114_AA_AAA_P329Y CH
SEQ ID NO: 41: base sequence of human CD4 (ibalizumab) VL
SEQ ID NO: 42: amino acid sequence of human CD4 (ibalizumab) VL
SEQ ID NO: 43: base sequence of human CD4 (ibalizumab) VH
SEQ ID NO: 44: amino acid sequence of human CD4 (ibalizumab) VH
SEQ ID NO: 45: base sequence of human CD70 (2H5) VL
SEQ ID NO: 46: amino acid sequence of human CD70 (2H5) VL
SEQ ID NO: 47: base sequence of human CD70 (2H5) VH
SEQ ID NO: 48: amino acid sequence of human CD70 (2H5) VH
SEQ ID NO: 49: base sequence of human IgG1114_ AA_AAA_S239R CH
SEQ ID NO: 50: amino acid sequence of human IgG1114_AA_AAA_S239R CH
SEQ ID NO: 51: base sequence of human IgG1114_AA_AAA_D265N CH
SEQ ID NO: 52: amino acid sequence of human IgG1114_AA_AAA_D265N CH
SEQ ID NO: 53: base sequence of human IgG1114_AA_AAA_D265E CH
SEQ ID NO: 54: amino acid sequence of human IgG1114_AA_AAA_D265E CH
SEQ ID NO: 55: base sequence of human IgG1114_AA_AAA_S267K CH
SEQ ID NO: 56: amino acid sequence of human IgG1114_AA_AAA_S267K CH
SEQ ID NO: 57: base sequence of human IgG1114_AA_AAA_E269P CH
SEQ ID NO: 58: amino acid sequence of human IgG1114_AA_AAA_E269P CH
SEQ ID NO: 59: base sequence of human IgG1114_AA_AAA_Y296P CH
SEQ ID NO: 60: amino acid sequence of human IgG1114_AA_AAA_Y296P CH
SEQ ID NO: 61: base sequence of human IgG1114_AA_AAA_S298E CH
SEQ ID NO: 62: amino acid sequence of human IgG1114_AA_AAA_S298E CH
SEQ ID NO: 63: base sequence of human IgG1114_AA_AAA_T299A CH
SEQ ID NO: 64: amino acid sequence of human IgG1114_AA_AAA_T299A CH
SEQ ID NO: 65: base sequence of human IgG1114_AA_AAA_L235R CH
SEQ ID NO: 66: amino acid sequence of human IgG1114_AA_AAA_L235R CH
SEQ ID NO: 67: base sequence of human IgG1114_AA_AAA_A327I CH
SEQ ID NO: 68: amino acid sequence of human IgG1114_AA_AAA_A327I CH
SEQ ID NO: 69: base sequence of human IgG1114_AA_AAA_K326G CH
SEQ ID NO: 70: amino acid sequence of human IgG1114_AA_AAA_K326G CH
SEQ ID NO: 71: base sequence of human IgG1114_AA_AAA_L328R CH
SEQ ID NO: 72: amino acid sequence of human IgG1114_AA_AAA_L328R CH
SEQ ID NO: 73: base sequence of human IgG1114_AA_AAA_P329K CH

SEQ ID NO: 74: amino acid sequence of human IgG1114_AA_AAA_P329K CH
SEQ ID NO: 75: base sequence of human IgG1114_AA_AAA_P329W CH
SEQ ID NO: 76: amino acid sequence of human IgG1114_AA_AAA_P329W CH
SEQ ID NO: 77: base sequence of human IgG1114_AA_AAA_A330P CH
SEQ ID NO: 78: amino acid sequence of human IgG1114_AA_AAA_A330P CH

## Claims

1. An antibody composition, which is an antibody composition against a first antigen and a second antigen that are different from each other, comprising a first IgG half-molecule and a second IgG half-molecule, wherein
each of the first IgG half-molecule and the second IgG half-molecule is composed of one immunoglobulin light chain (hereinafter abbreviated as L chain) and one immunoglobulin heavy chain (hereinafter abbreviated as H chain), the H chain includes an H chain variable region, a hinge domain altered so as not to form a disulfide bond by substitution or deletion of a part or the whole or modification, and CH1 to CH3 domains, and has alteration in either of a first Fcγ receptor IIIA (hereinafter CD16a)-binding domain and a second CD16a-binding domain that are different from each other in the CH2 domain,
the first IgG half-molecule includes an antigen-binding domain that binds to the first antigen, and a CD16a-binding activity in the first CD16a-binding domain is attenuated by the alteration,
the second IgG half-molecule includes an antigen-binding domain that binds to the second antigen, and a CD16a-binding activity in the second CD16a-binding domain is attenuated by the alteration, and an inter-H chain disulfide bond is not formed between the first IgG half-molecule and the second IgG half-molecule.

2. The antibody composition according to claim 1,
wherein the antibody composition exhibits an effector function only for a target cell coexpressing the first antigen and the second antigen and damages the target cell.

3. The antibody composition according to claim 1 or 2,
wherein the alteration in the first CD16a-binding domain and the second CD16a-binding domain is substitution, deletion or addition, or modification of an amino acid.

4. The antibody composition according to any one of claims 1 to 3,
wherein the first CD16a-binding domain includes at least one selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index.

5. The antibody composition according to claim 4,
wherein the alteration in the first CD16a-binding domain is substitution of at least one amino acid residue selected from amino acid residues at position 235, position 236, position 237, position 238, position 239, position 265, position 266, position 267, position 268, position 269, position 294, position 295, position 296, position 297, position 298, position 299, position 301, position 325, position 327, and position 332 numbered according to the EU index.

6. The antibody composition according to claim 5,
wherein the alteration in the first CD16a-binding domain is substitution of at least one amino acid residue selected from amino acid residues at position 235, position 238, position 239, position 265, position 267, position 269, position 296, position 298, position 299, and position 327 numbered according to the EU index.

7. The antibody composition according to any one of claims 1 to 6,
wherein the second CD16a-binding domain includes at least one selected from amino acid residues at position 235, position 236, position 237, position 326, position 327, position 328, position 329, and position 330 numbered according to the EU index.

8. The antibody composition according to claim 7,
wherein the alteration in the second CD16a-binding domain is substitution of at least one amino acid residue selected from amino acid residues at position 235, position 236, position 237, position 326, position 327, position 328, position 329, and position 330 numbered according to the EU index.

9. The antibody composition according to claim 8,
wherein the second CD16a-binding domain includes at least one selected from amino acid residues at position 326, position 328, position 329, and position 330 numbered according to the EU index.

10. The antibody composition according to any one of claims 1 to 9,
wherein the alteration in the first CD16a-binding domain is substitution of at least one amino acid residue selected from amino acid residues at position 235, position 238, position 239, position 265, position 267, position 269, position 296, position 298, position 299, and position 327 numbered according to the EU index, and
the alteration in the second CD16a-binding domain is substitution of at least one amino acid residue selected from amino acid residues at position 326, position 328, position 329, and position 330 numbered according to the EU index.

11. The antibody composition according to any one of claims 1 to 10,
wherein the alteration in the first CD16a-binding domain is substitution of at least one amino acid residue selected from amino acid residues at position 238, position 265, and position 267 numbered according to the EU index.

12. The antibody composition according to any one of claims 1 to 11,
wherein the alteration in the second CD16a-binding domain is substitution of at least one amino acid residue selected from amino acid residues at position 326, position 328, and position 329 numbered according to the EU index.

13. The antibody composition according to any one of claims 1 to 12,
wherein the first IgG half-molecule and the second IgG half-molecule include a hinge domain in which at least one amino acid residue of amino acid residues at position 226 and position 229 numbered according to the EU index is substituted.

14. The antibody composition according to any one of claims 1 to 13,
wherein immunoglobulin subclasses of the L chain, and the H chain variable region, the CH1 domain, and the CH2 domain of the H chain in the first IgG half-molecule and the second IgG half-molecule are IgG1.

15. The antibody composition according to claim 14,
wherein the CH3 domain of the H chain in the first IgG half-molecule and the second IgG half-molecule has a weaker inter-CH3 domain interaction than a CH3 domain of the IgG1 subclass.

16. The antibody composition according to claim 15,
wherein immunoglobulin subclass of the CH3 domain of the H chain in the first IgG half-molecule and the second IgG half-molecule is IgG4.

17. The antibody composition according to any one of claims 1 to 16,
wherein the antibody composition binds to CD16a through the second CD16a-binding domain in the first IgG half-molecule and the first CD16a-binding domain in the second IgG half-molecule.

18. The antibody composition according to any one of claims 1 to 17,
wherein a ratio of sugar chains in which fucose is not bound to N-acetylglucosamine at a reducing end of the sugar chain among the total N-glycoside-linked type sugar chains bound to an Fc region in the first IgG half-molecule and

the second IgG half-molecule is 20% or more.

**19.** The antibody composition according to any one of claims 1 to 18,
wherein the first IgG half-molecule and the second IgG half-molecule include at least one amino acid residue substitution for further enhancing the CD16a-binding activity in the CH2 domain.

**20.** The antibody composition according to claim 19,
wherein the first IgG half-molecule and the second IgG half-molecule include at least one amino acid residue substitution selected from S298A, E333A, and K334A numbered according to the EU index in the CH2 domain.

**21.** A first IgG half-molecule, which is a first IgG half-molecule to be used in combination with a second IgG half-molecule, wherein each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,
the first IgG half-molecule includes an antigen-binding domain that binds to a first antigen, and a CD16a-binding activity in the first CD16a-binding domain is attenuated by the alteration,
the second IgG half-molecule includes an antigen-binding domain that binds to a second antigen different from the first antigen, and a CD16a-binding activity in the second CD16a-binding domain is attenuated by the alteration, and an inter-H chain disulfide bond is not formed between the first IgG half-molecule and the second IgG half-molecule.

**22.** A first IgG half-molecule, which is a first IgG half-molecule to be used for producing an antibody composition against a first antigen and a second antigen that are different from each other, the antibody composition composed of the first IgG half-molecule and a second IgG half-molecule, wherein
each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,
the first IgG half-molecule includes an antigen-binding domain that binds to the first antigen, and a CD16a-binding activity in the first CD16a-binding domain is attenuated by the alteration,
the second IgG half-molecule includes an antigen-binding domain that binds to the second antigen, and a CD16a-binding activity in the second CD16a-binding domain is attenuated by the alteration, and
an inter-H chain disulfide bond is not formed between the first IgG half-molecule and the second IgG half-molecule.

**23.** A second IgG half-molecule, which is a second IgG half-molecule to be used in combination with a first IgG half-molecule, wherein each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,
the first IgG half-molecule includes an antigen-binding domain that binds to a first antigen, and a CD16a-binding activity in the first CD16a-binding domain is attenuated by the alteration,
the second IgG half-molecule includes an antigen-binding domain that binds to a second antigen different from the first antigen, and a CD16a-binding activity in the second CD 16a-binding domain is attenuated by the alteration, and an inter-H chain disulfide bond is not formed between the first IgG half-molecule and the second IgG half-molecule.

**24.** A second IgG half-molecule, which is a second IgG half-molecule to be used for producing an antibody composition against a first antigen and a second antigen that are different from each other, the antibody composition composed of a first IgG half-molecule and the second IgG half-molecule, wherein
each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CHI to CH3 domains, and has alteration in either of a first CD16a-binding domain and a second CD16a-binding domain that are different from each other in the CH2 domain,
the first IgG half-molecule includes an antigen-binding domain that binds to the first antigen, and a CD16a-binding activity in the first CD16a-binding domain is attenuated by the alteration,

the second IgG half-molecule includes an antigen-binding domain that binds to the second antigen, and a CD16a-binding activity in the second CD16a-binding domain is attenuated by the alteration, and
an inter-H chain disulfide bond is not formed between the first IgG half-molecule and the second IgG half-molecule.

**25.** A DNA, which is the following DNA a) or b):

a) a DNA encoding an amino acid sequence of the first IgG half-molecule according to claim 21 or 22; or
b) a DNA encoding an amino acid sequence of the second IgG half-molecule according to claim 23 or 24.

**26.** A recombinant vector, comprising at least one of the DNAs a) and b) according to claim 25.

**27.** A transformant, in which the recombinant vector according to claim 26 is introduced.

**28.** A method for producing an antibody composition, comprising the following steps of:

culturing the transformant according to claim 27 in a culture medium,
accumulating at least one of the first IgG half-molecule according to claim 21 or 22 and the second IgG half-molecule according to claim 23 or 24 in a culture and;
collecting at least one of the first IgG half-molecule and the second IgG half-molecule from the culture.

**29.** A kit comprising a first IgG half-molecule and a second IgG half-molecule, wherein each of the first IgG half-molecule and the second IgG half-molecule is composed of one L chain and one H chain, the H chain includes an H chain variable region, a hinge domain altered so as not to form a disulfide bond by substitution or deletion of a part or the whole or modification, and CH1 to CH3 domains, and has alteration in either of a first CD16a-binding domain and a second CD16a-binding domain that are different from each other in the CH2 domain,
the first IgG half-molecule includes an antigen-binding domain that binds to a first antigen, and a CD16a-binding activity in the first CD16a-binding domain is attenuated by the alteration,
the second IgG half-molecule includes an antigen-binding domain that binds to a second antigen, and a CD16a-binding activity in the second CD 16a-binding domain is attenuated by the alteration, and
an inter-H chain disulfide bond is not formed between the first IgG half-molecule and the second IgG half-molecule.

**30.** A method for inducing an effector function only for a target cell coexpressing a first antigen and a second antigen using the antibody composition according to any one of claims 1 to 20.

FIG. 1

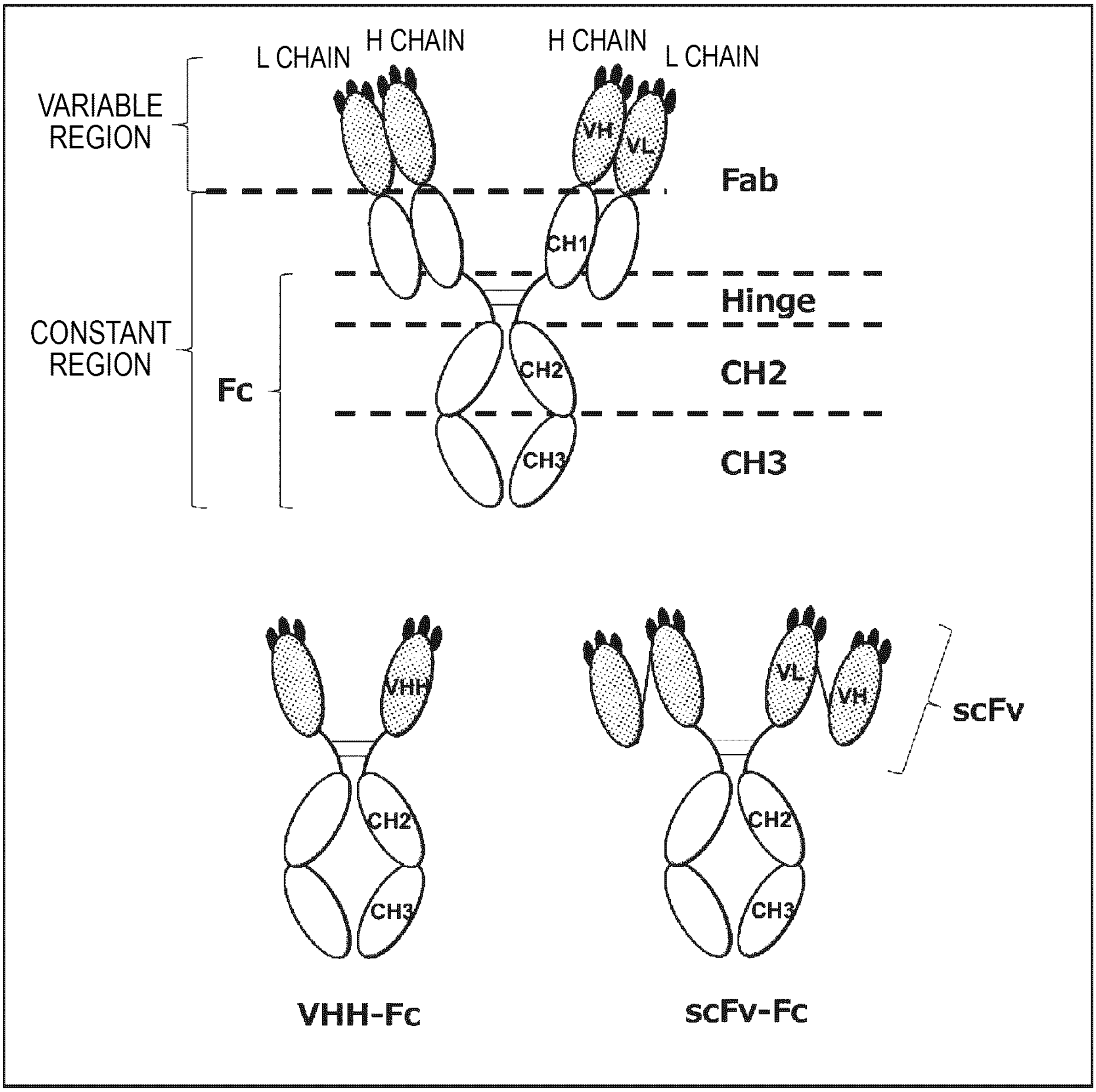
L CHAIN
H CHAIN
H CHAIN
L CHAIN
VARIABLE REGION
VH
VL
Fab
CH1
CONSTANT REGION
Hinge
Fc
CH2
CH2
CH3
CH3
VHH
CH2
CH3
VL
VH
scFv
CH2
CH3
VHH-Fc
scFv-Fc

*FIG. 2A*

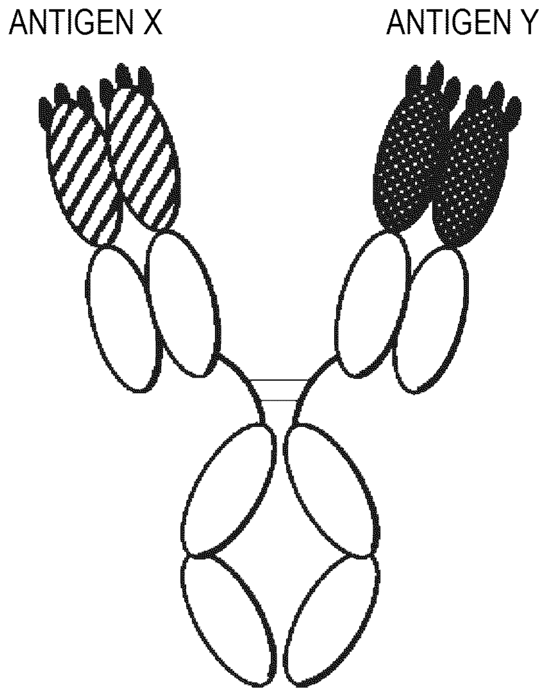
ANTIGEN X
ANTIGEN Y

*FIG. 2B*

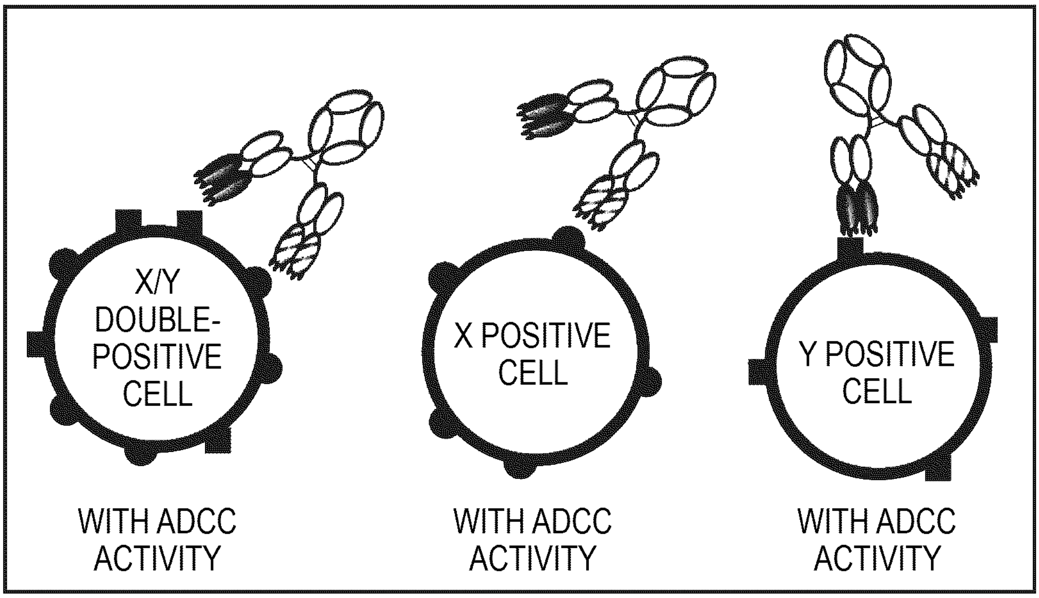
X/Y DOUBLE-POSITIVE CELL
WITH ADCC ACTIVITY
X POSITIVE CELL
WITH ADCC ACTIVITY
Y POSITIVE CELL
WITH ADCC ACTIVITY

FIG. 3

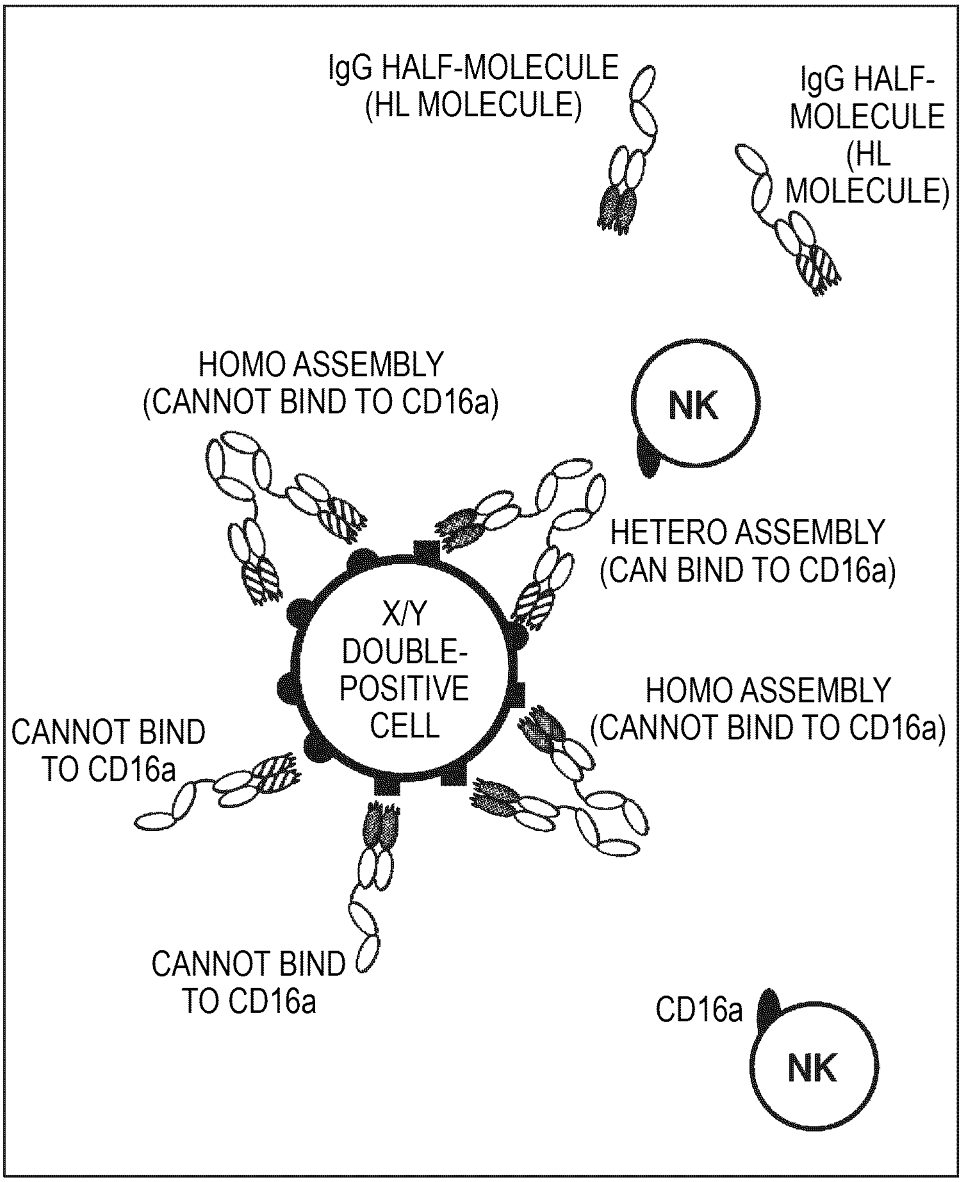
IgG HALF-MOLECULE
(HL MOLECULE)
IgG HALF-
MOLECULE
(HL
MOLECULE)
HOMO ASSEMBLY
(CANNOT BIND TO CD16a)
NK
HETERO ASSEMBLY
(CAN BIND TO CD16a)
X/Y
DOUBLE-
POSITIVE
CELL
HOMO ASSEMBLY
(CANNOT BIND TO CD16a)
CANNOT BIND
TO CD16a
CANNOT BIND
TO CD16a
CD16a
NK

FIG. 4

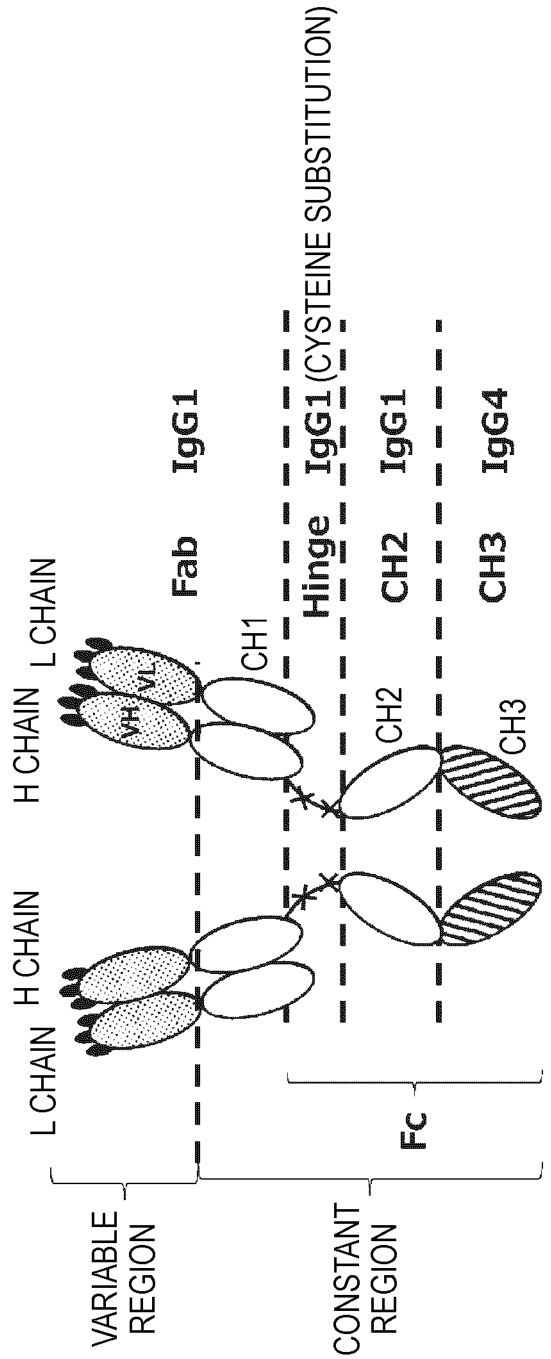
VARIABLE REGION
CONSTANT REGION
Fc
L CHAIN
H CHAIN
H CHAIN
L CHAIN
VH
VL
CH1
CH2
CH3
Fab
IgG1
Hinge
IgG1 (CYSTEINE SUBSTITUTION)
CH2
IgG1
CH3
IgG4

FIG. 5

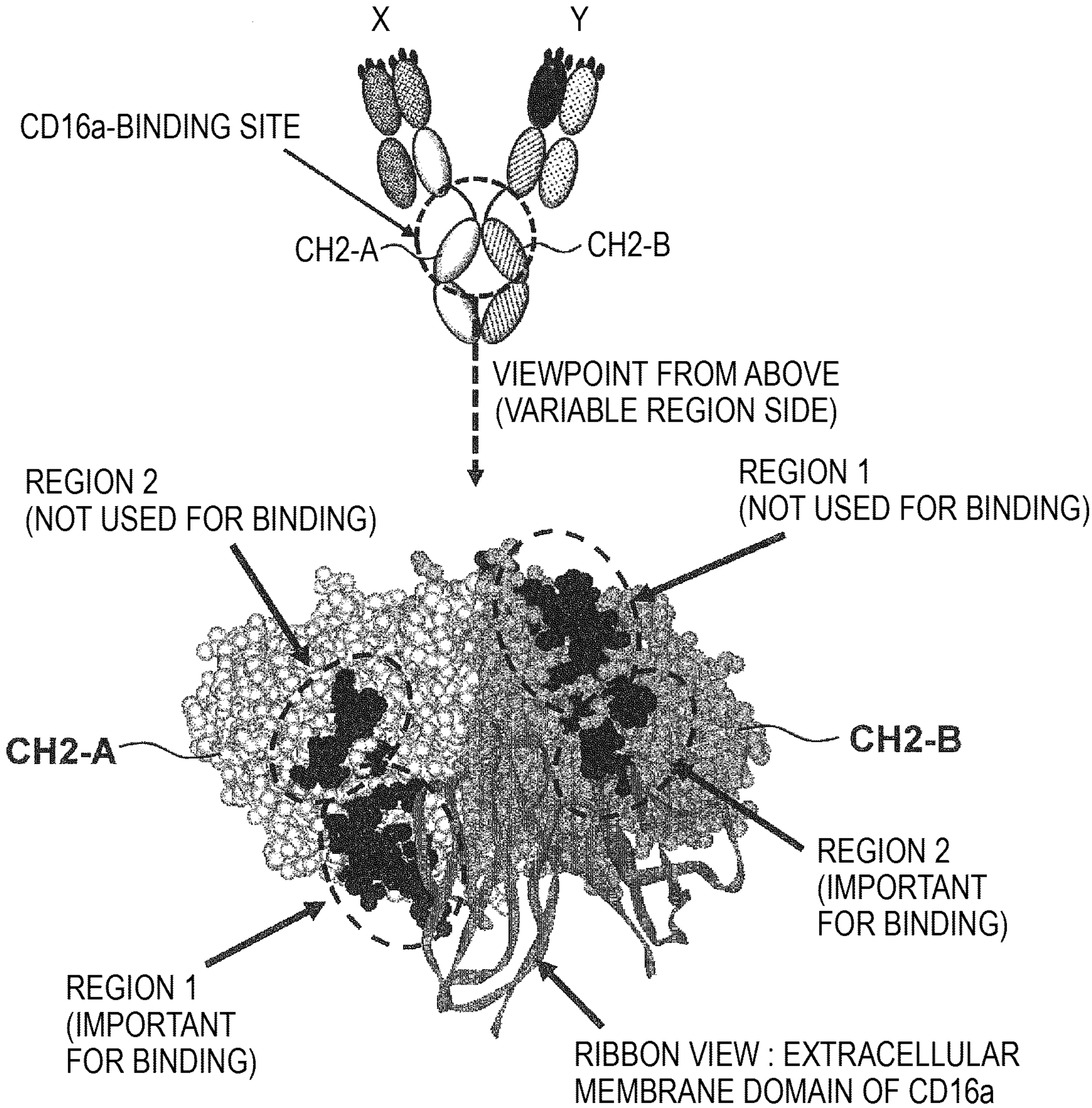
X
Y
CD16a-BINDING SITE
CH2-A
CH2-B
VIEWPOINT FROM ABOVE
(VARIABLE REGION SIDE)
REGION 2
(NOT USED FOR BINDING)
REGION 1
(NOT USED FOR BINDING)
CH2-A
CH2-B
REGION 2
(IMPORTANT
FOR BINDING)
REGION 1
(IMPORTANT
FOR BINDING)
RIBBON VIEW : EXTRACELLULAR
MEMBRANE DOMAIN OF CD16a

# FIG. 6

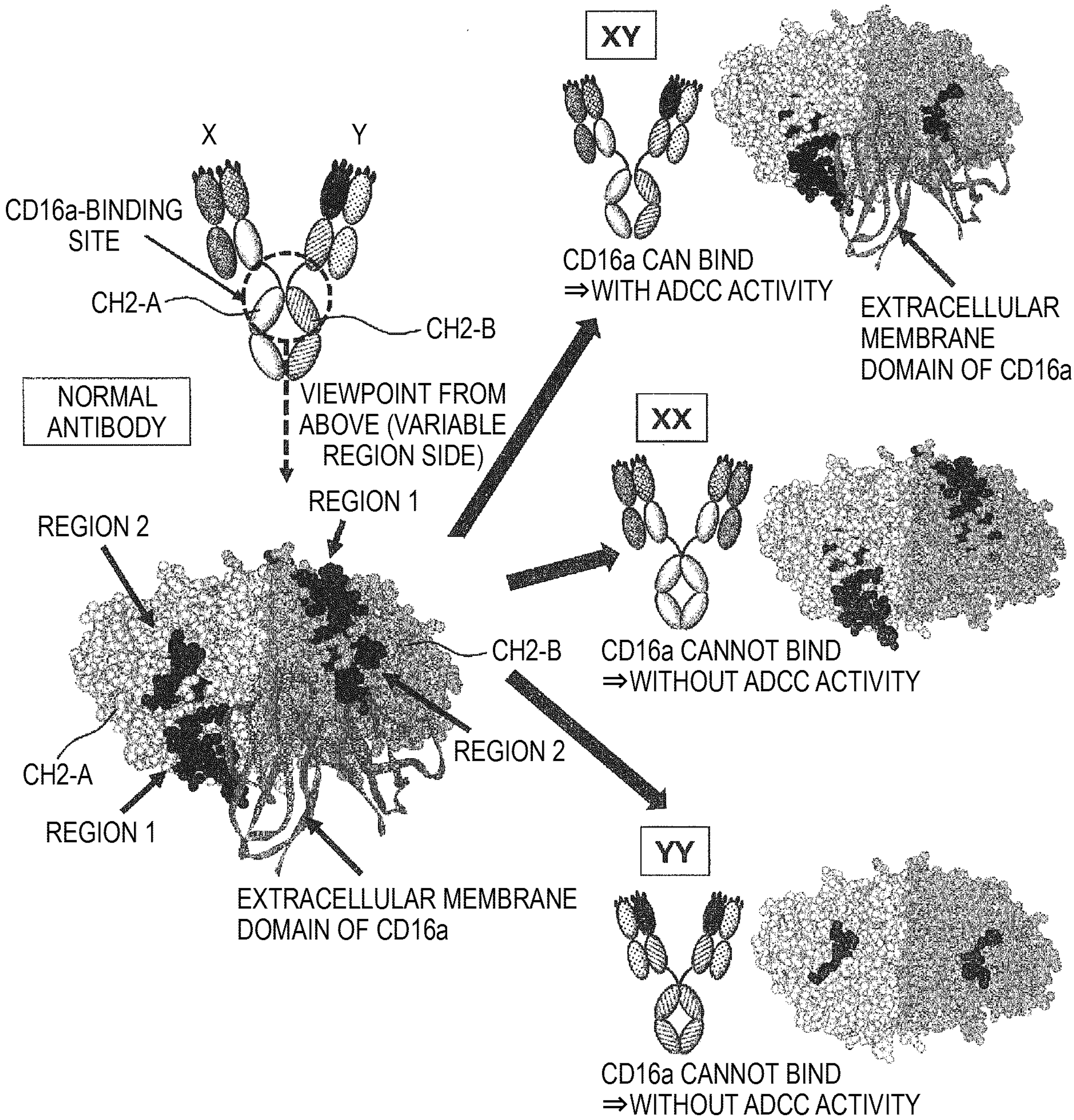
X
Y
CD16a-BINDING SITE
CH2-A
CH2-B
NORMAL ANTIBODY
VIEWPOINT FROM ABOVE (VARIABLE REGION SIDE)
REGION 1
REGION 2
CH2-B
REGION 2
CH2-A
REGION 1
EXTRACELLULAR MEMBRANE DOMAIN OF CD16a
XY
CD16a CAN BIND
⇒WITH ADCC ACTIVITY
EXTRACELLULAR MEMBRANE DOMAIN OF CD16a
XX
CD16a CANNOT BIND
⇒WITHOUT ADCC ACTIVITY
YY
CD16a CANNOT BIND
⇒WITHOUT ADCC ACTIVITY

FIG. 7

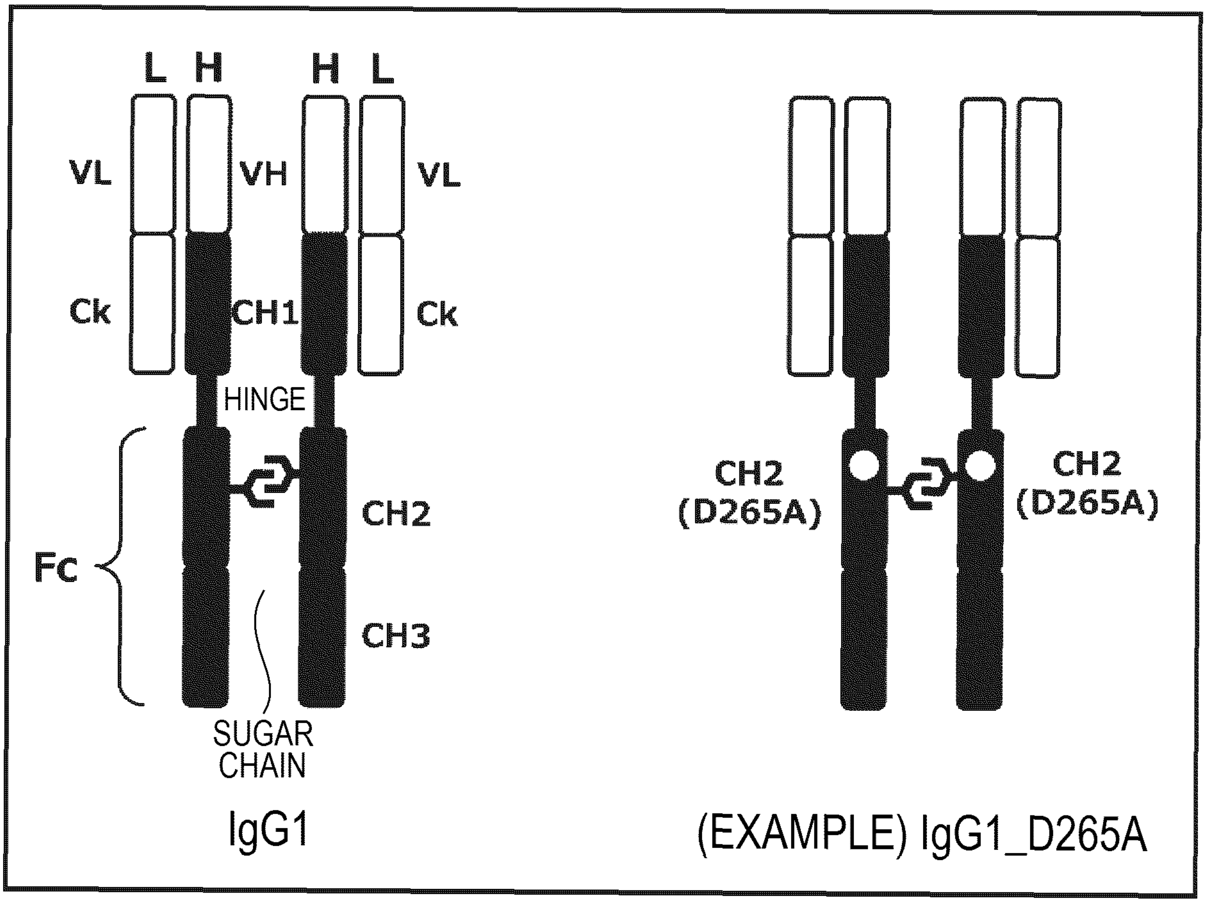
L
H
H
L
VL
VH
VL
Ck
CH1
Ck
HINGE
Fc
CH2
CH3
SUGAR CHAIN
IgG1
CH2 (D265A)
CH2 (D265A)
(EXAMPLE) IgG1_D265A

FIG. 8

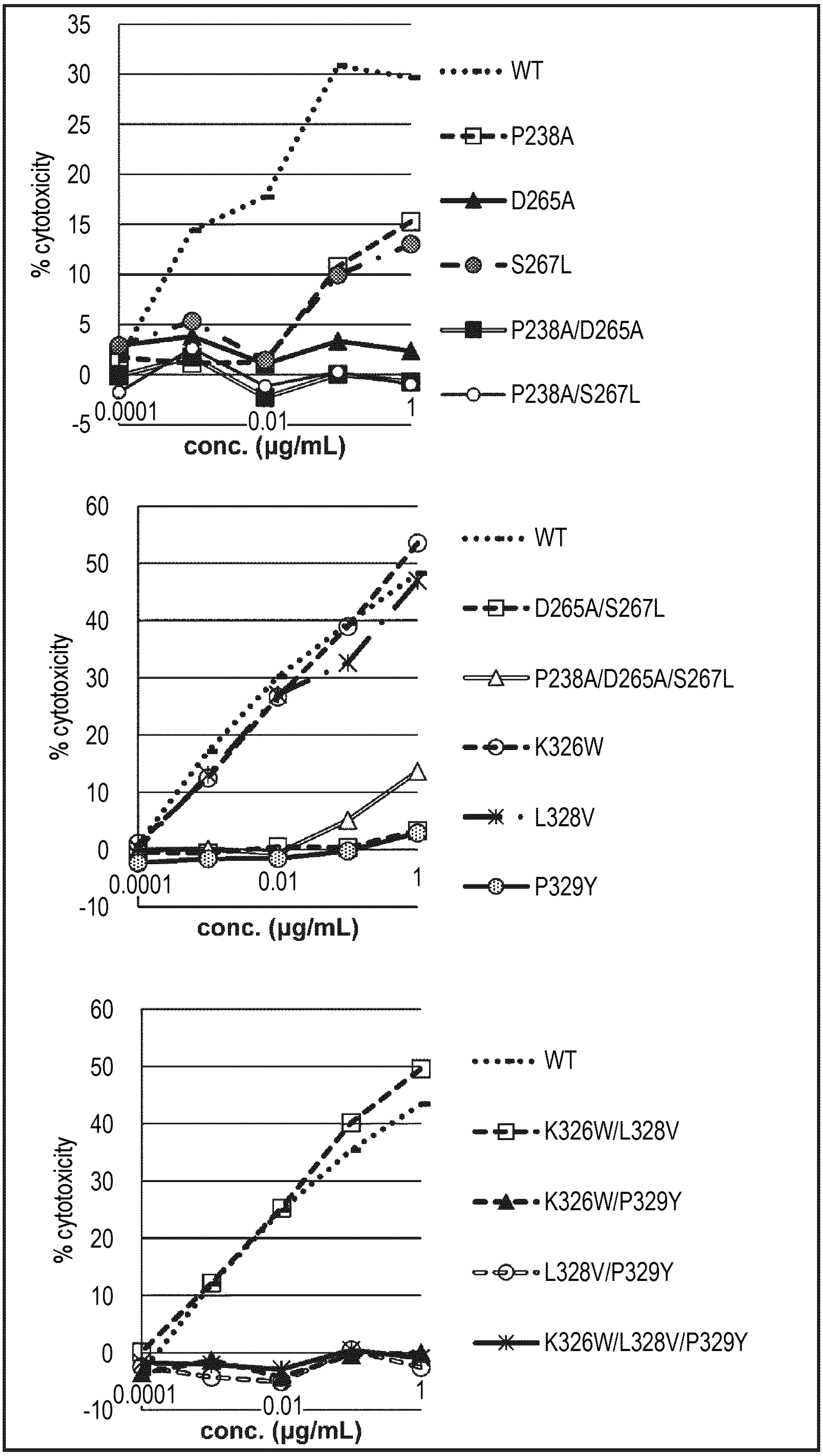
35
30
25
20
15
10
5
0
-5
% cytotoxicity
0.0001
0.01
1
conc. (µg/mL)
WT
P238A
D265A
S267L
P238A/D265A
P238A/S267L
60
50
40
30
20
10
0
-10
% cytotoxicity
0.0001
0.01
1
conc. (µg/mL)
WT
D265A/S267L
P238A/D265A/S267L
K326W
L328V
P329Y
60
50
40
30
20
10
0
-10
% cytotoxicity
0.0001
0.01
1
conc. (µg/mL)
WT
K326W/L328V
K326W/P329Y
L328V/P329Y
K326W/L328V/P329Y

FIG. 9

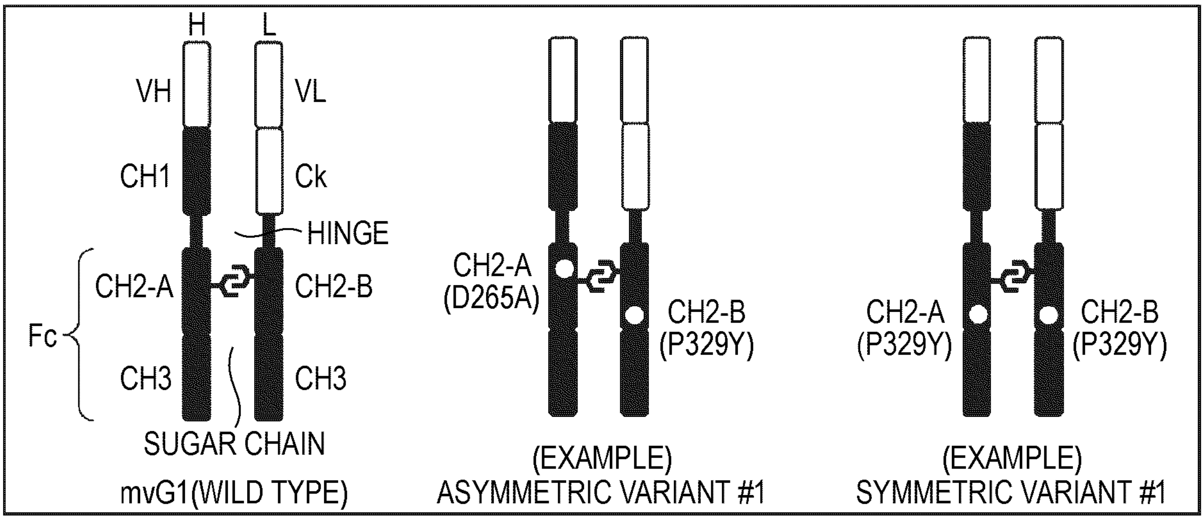
H
L
VH
VL
CH1
Ck
HINGE
CH2-A
CH2-B
Fc
CH3
CH3
SUGAR CHAIN
mvG1(WILD TYPE)
CH2-A
(D265A)
CH2-B
(P329Y)
(EXAMPLE)
ASYMMETRIC VARIANT #1
CH2-A
(P329Y)
CH2-B
(P329Y)
(EXAMPLE)
SYMMETRIC VARIANT #1

FIG. 10

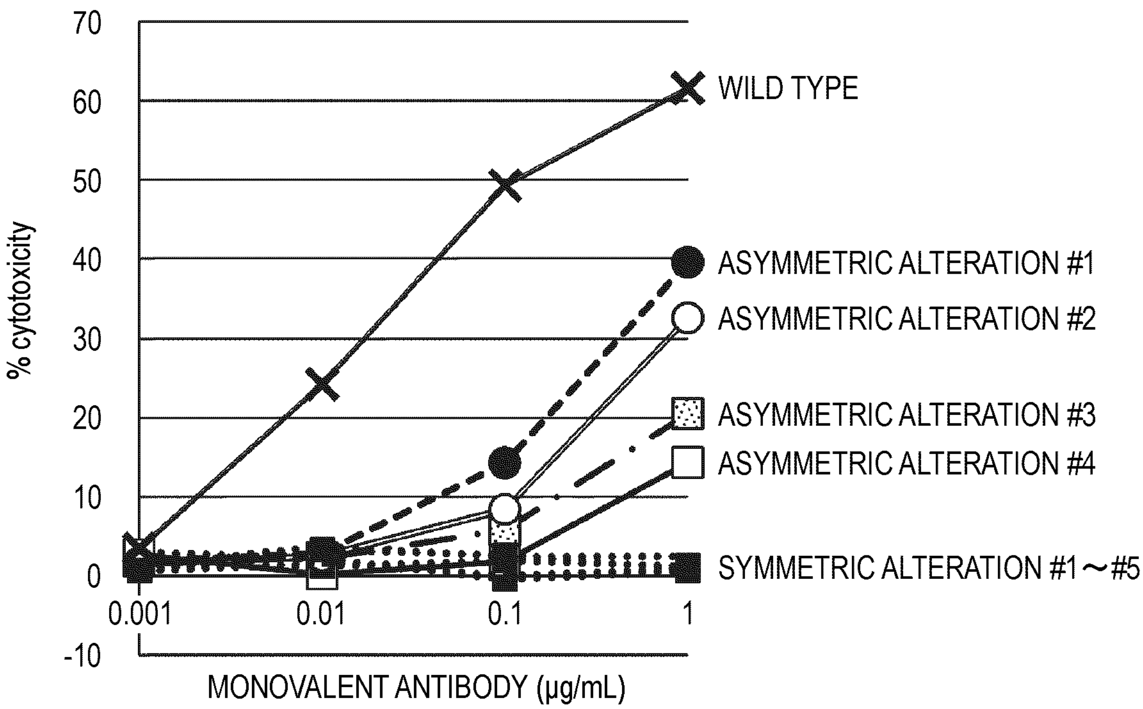
70
60
50
40
30
20
10
0
-10
% cytotoxicity
0.001
0.01
0.1
1
MONOVALENT ANTIBODY (μg/mL)
WILD TYPE
ASYMMETRIC ALTERATION #1
ASYMMETRIC ALTERATION #2
ASYMMETRIC ALTERATION #3
ASYMMETRIC ALTERATION #4
SYMMETRIC ALTERATION #1～#5

FIG. 11

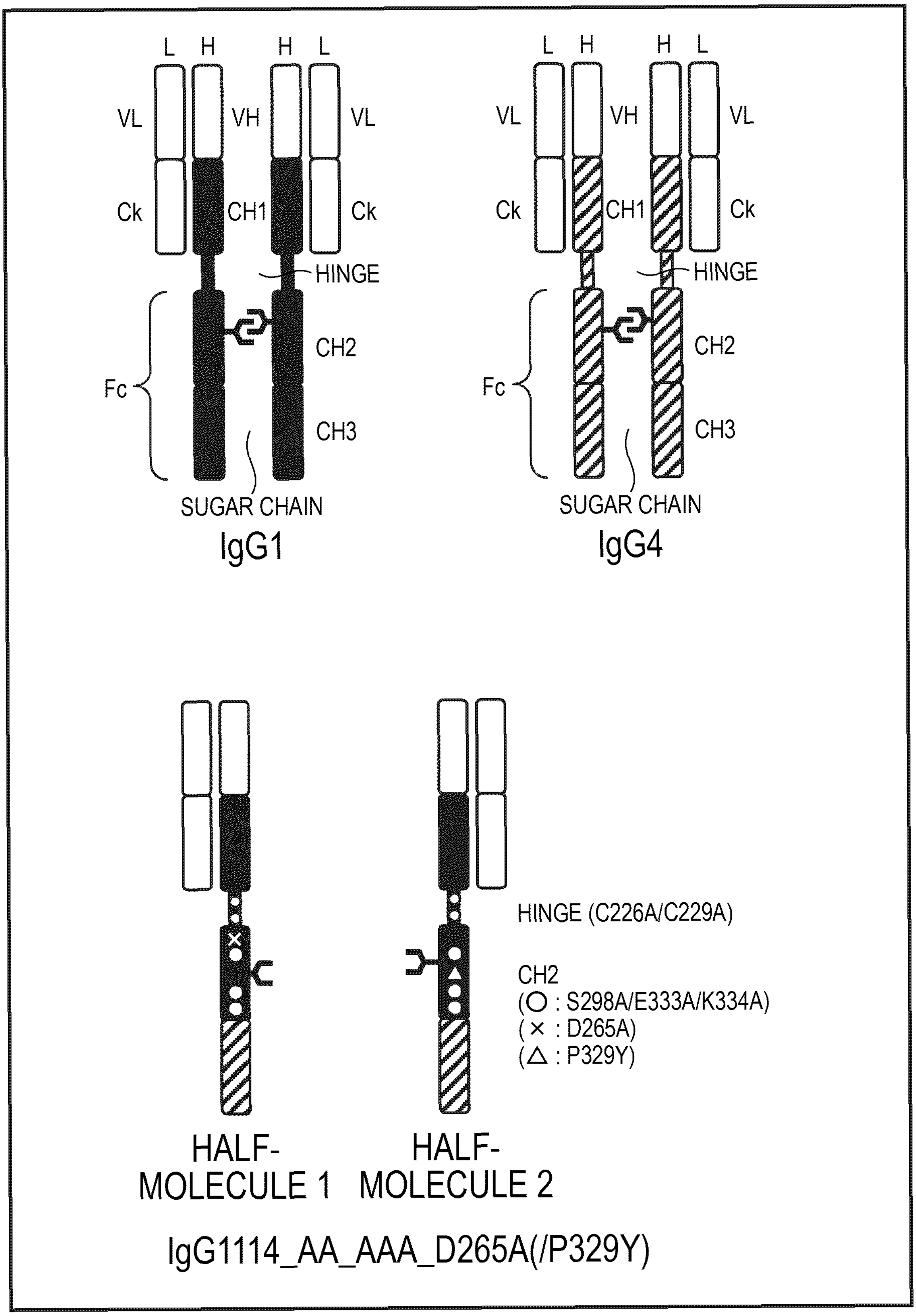
L
H
H
L
VL
VH
VL
Ck
CH1
Ck
HINGE
Fc
CH2
CH3
SUGAR CHAIN
IgG1
L
H
H
L
VL
VH
VL
Ck
CH1
Ck
HINGE
Fc
CH2
CH3
SUGAR CHAIN
IgG4
HINGE (C226A/C229A)
CH2
(○ : S298A/E333A/K334A)
(× : D265A)
(△ : P329Y)
HALF-MOLECULE 1
HALF-MOLECULE 2
IgG1114_AA_AAA_D265A(/P329Y)

FIG. 12

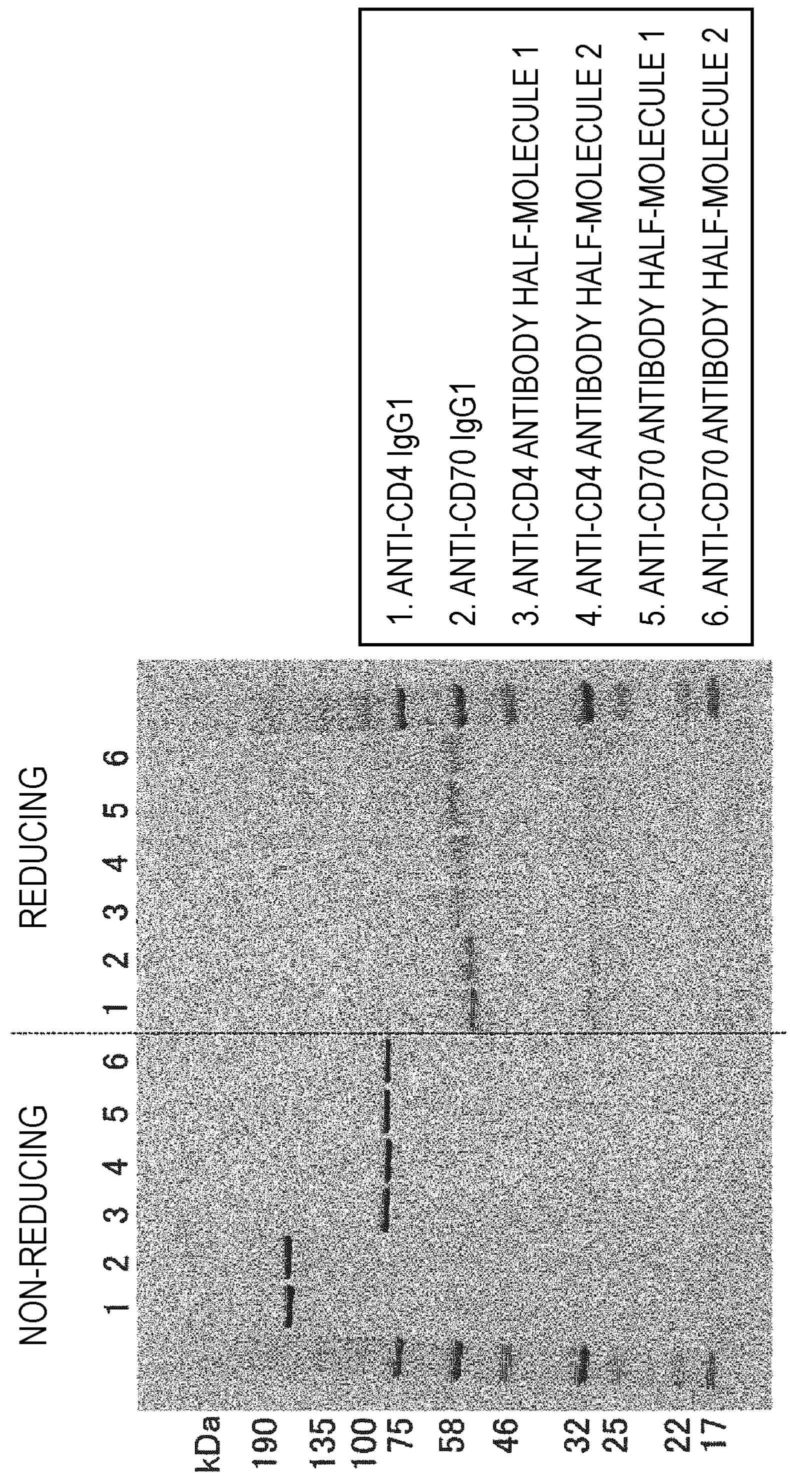
NON-REDUCING
REDUCING
1 2 3 4 5 6
1 2 3 4 5 6
kDa
190
135
100
75
58
46
32
25
22
17
1. ANTI-CD4 IgG1
2. ANTI-CD70 IgG1
3. ANTI-CD4 ANTIBODY HALF-MOLECULE 1
4. ANTI-CD4 ANTIBODY HALF-MOLECULE 2
5. ANTI-CD70 ANTIBODY HALF-MOLECULE 1
6. ANTI-CD70 ANTIBODY HALF-MOLECULE 2

# FIG. 13

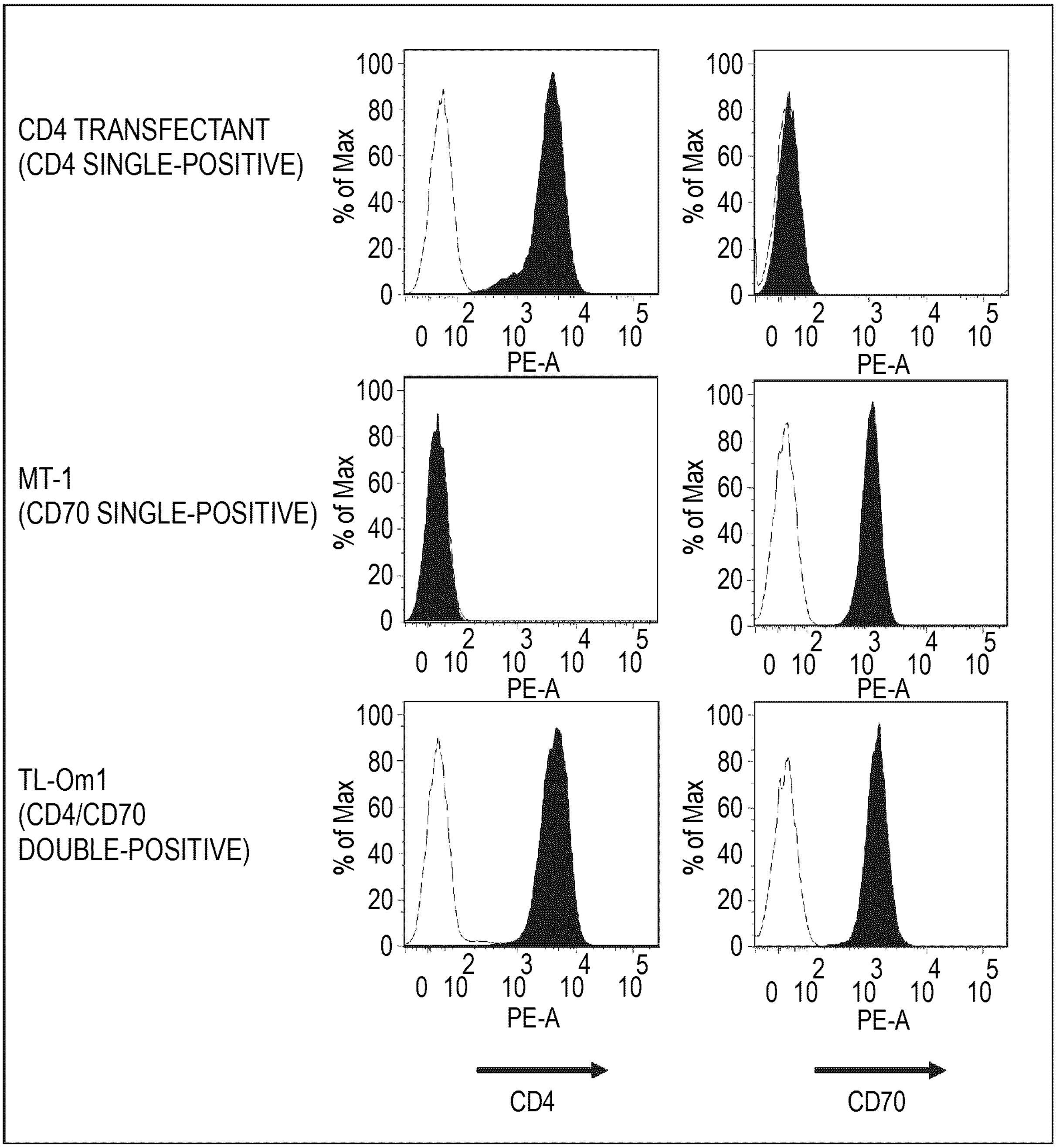
CD4 TRANSFECTANT
(CD4 SINGLE-POSITIVE)
MT-1
(CD70 SINGLE-POSITIVE)
TL-Om1
(CD4/CD70
DOUBLE-POSITIVE)
% of Max
100
80
60
40
20
0
0
10^2
10^3
10^4
10^5
PE-A
CD4
CD70

FIG. 14

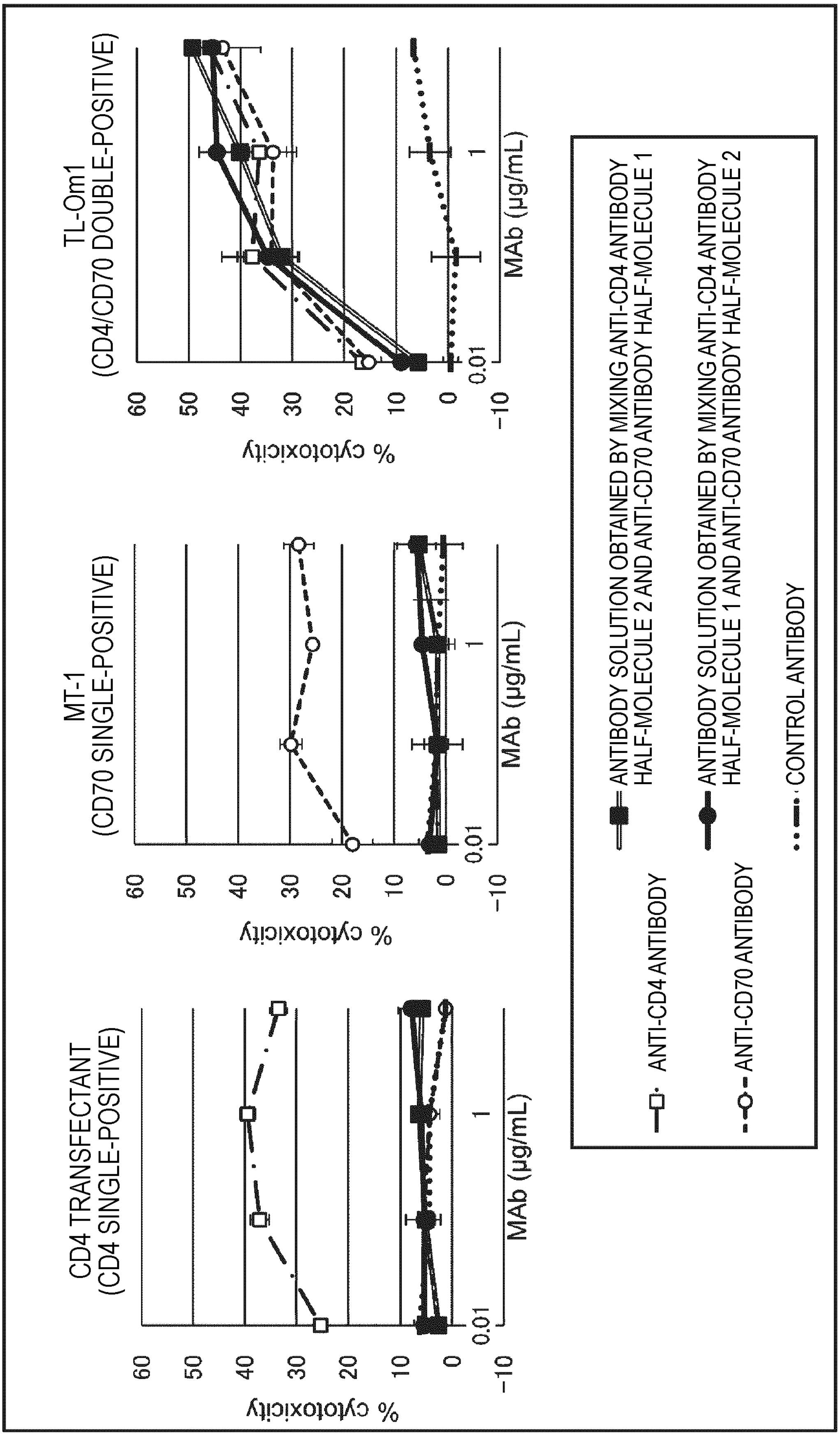
CD4 TRANSFECTANT
(CD4 SINGLE-POSITIVE)
MT-1
(CD70 SINGLE-POSITIVE)
TL-Om1
(CD4/CD70 DOUBLE-POSITIVE)
% cytotoxicity
60
50
40
30
20
10
0
−10
0.01
1
MAb (μg/mL)
ANTI-CD4 ANTIBODY
ANTI-CD70 ANTIBODY
ANTIBODY SOLUTION OBTAINED BY MIXING ANTI-CD4 ANTIBODY HALF-MOLECULE 2 AND ANTI-CD70 ANTIBODY HALF-MOLECULE 1
ANTIBODY SOLUTION OBTAINED BY MIXING ANTI-CD4 ANTIBODY HALF-MOLECULE 1 AND ANTI-CD70 ANTIBODY HALF-MOLECULE 2
CONTROL ANTIBODY

FIG. 15A

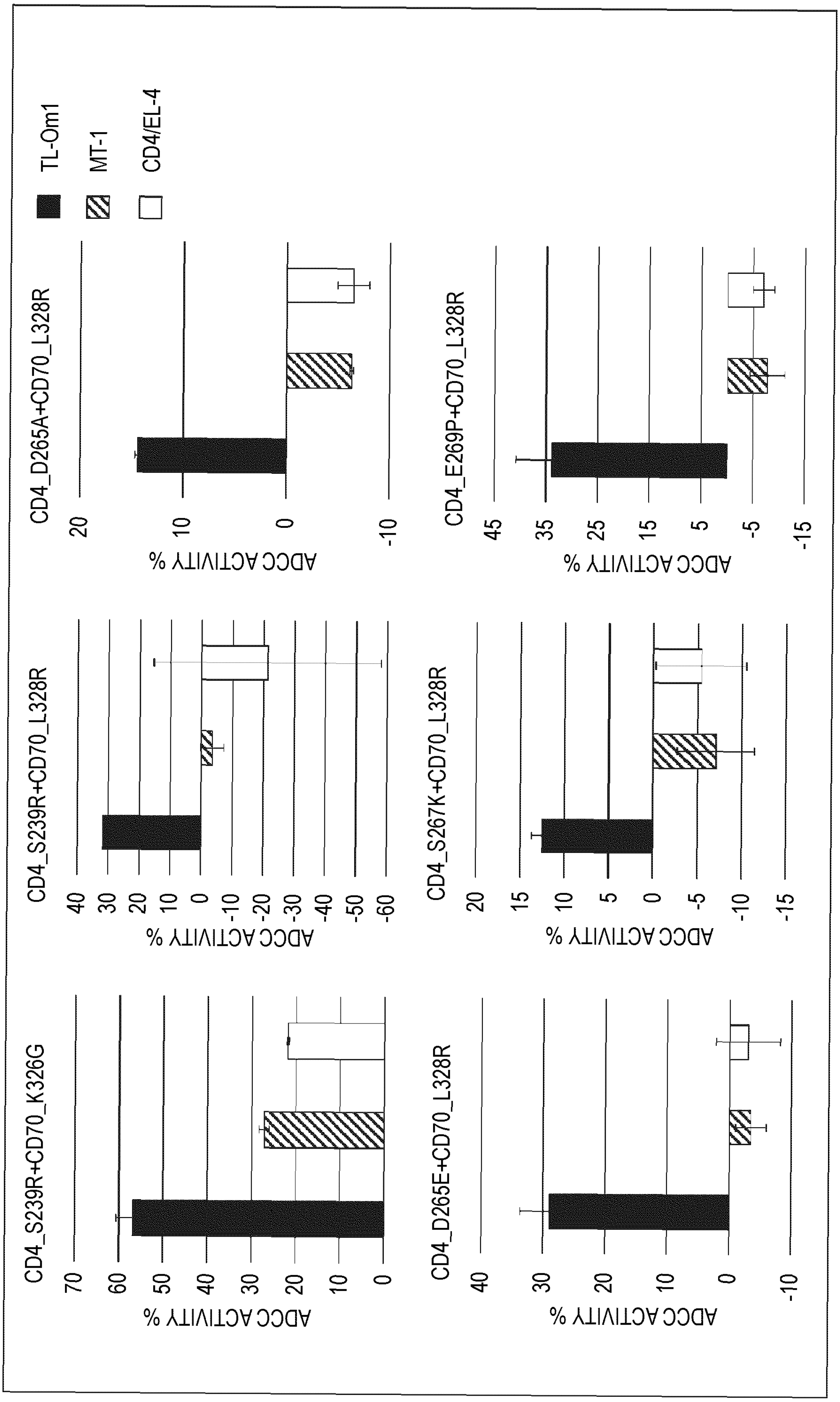

TL-Om1
MT-1
CD4/EL-4
CD4_S239R+CD70_K326G
CD4_S239R+CD70_L328R
CD4_D265A+CD70_L328R
CD4_D265E+CD70_L328R
CD4_S267K+CD70_L328R
CD4_E269P+CD70_L328R
ADCC ACTIVITY %

FIG. 15B

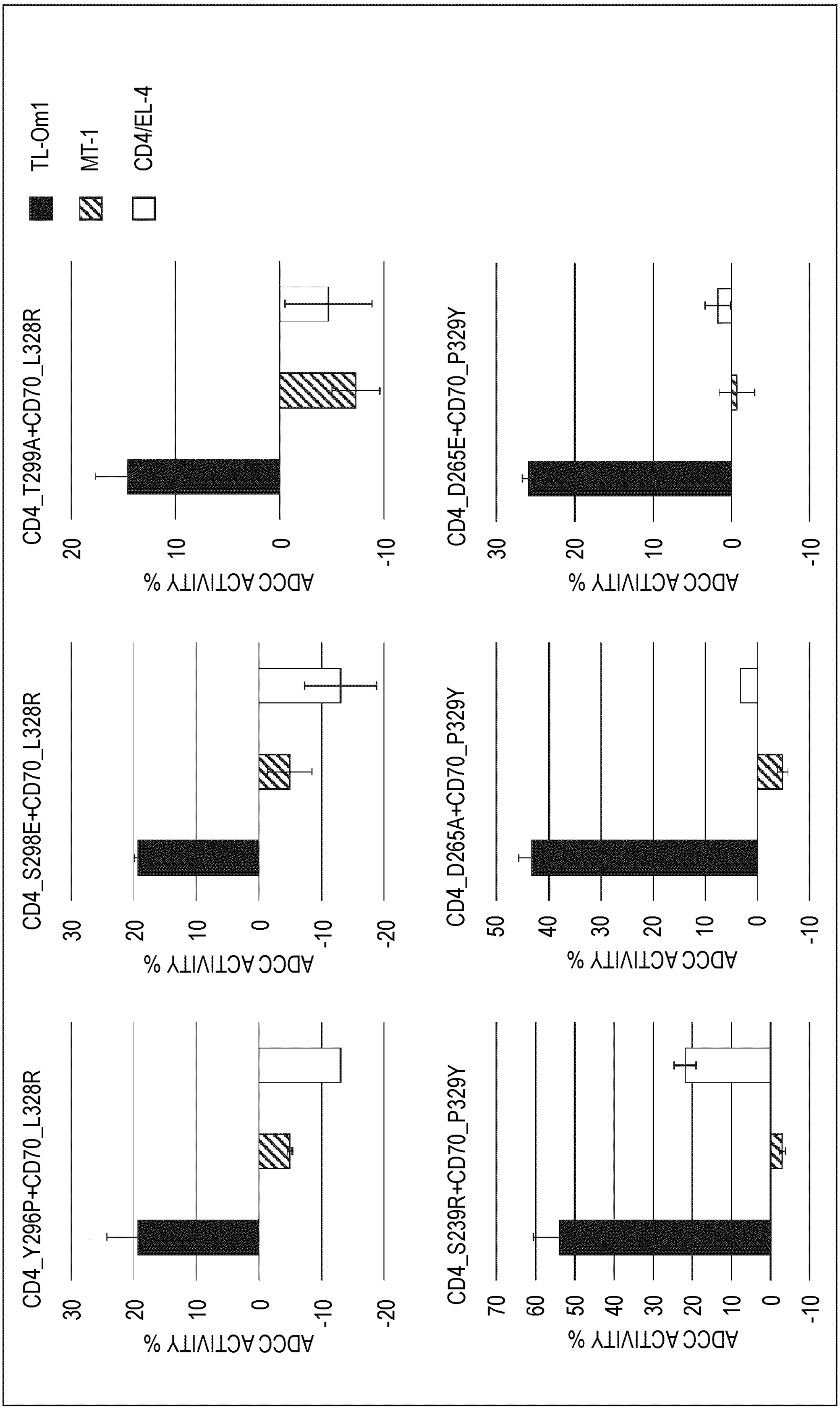
CD4_Y296P+CD70_L328R
CD4_S298E+CD70_L328R
CD4_T299A+CD70_L328R
CD4_S239R+CD70_P329Y
CD4_D265A+CD70_P329Y
CD4_D265E+CD70_P329Y
ADCC ACTIVITY %
TL-Om1
MT-1
CD4/EL-4

FIG. 15C

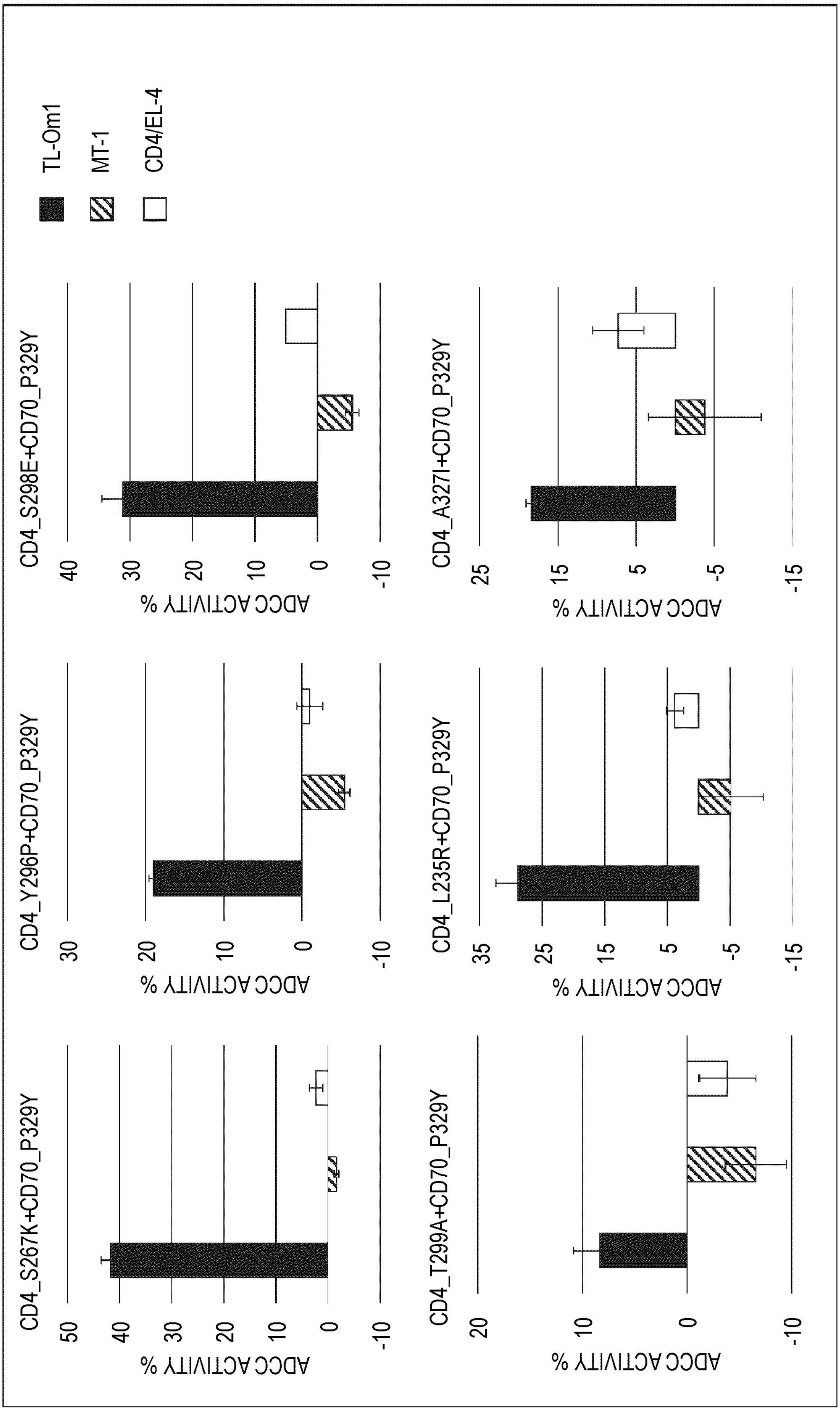
TL-Om1
MT-1
CD4/EL-4
CD4_S267K+CD70_P329Y
CD4_Y296P+CD70_P329Y
CD4_S298E+CD70_P329Y
CD4_T299A+CD70_P329Y
CD4_L235R+CD70_P329Y
CD4_A327I+CD70_P329Y
ADCC ACTIVITY %
50
40
30
20
10
0
-10
35
25
15
5
-5
-15

FIG. 15D

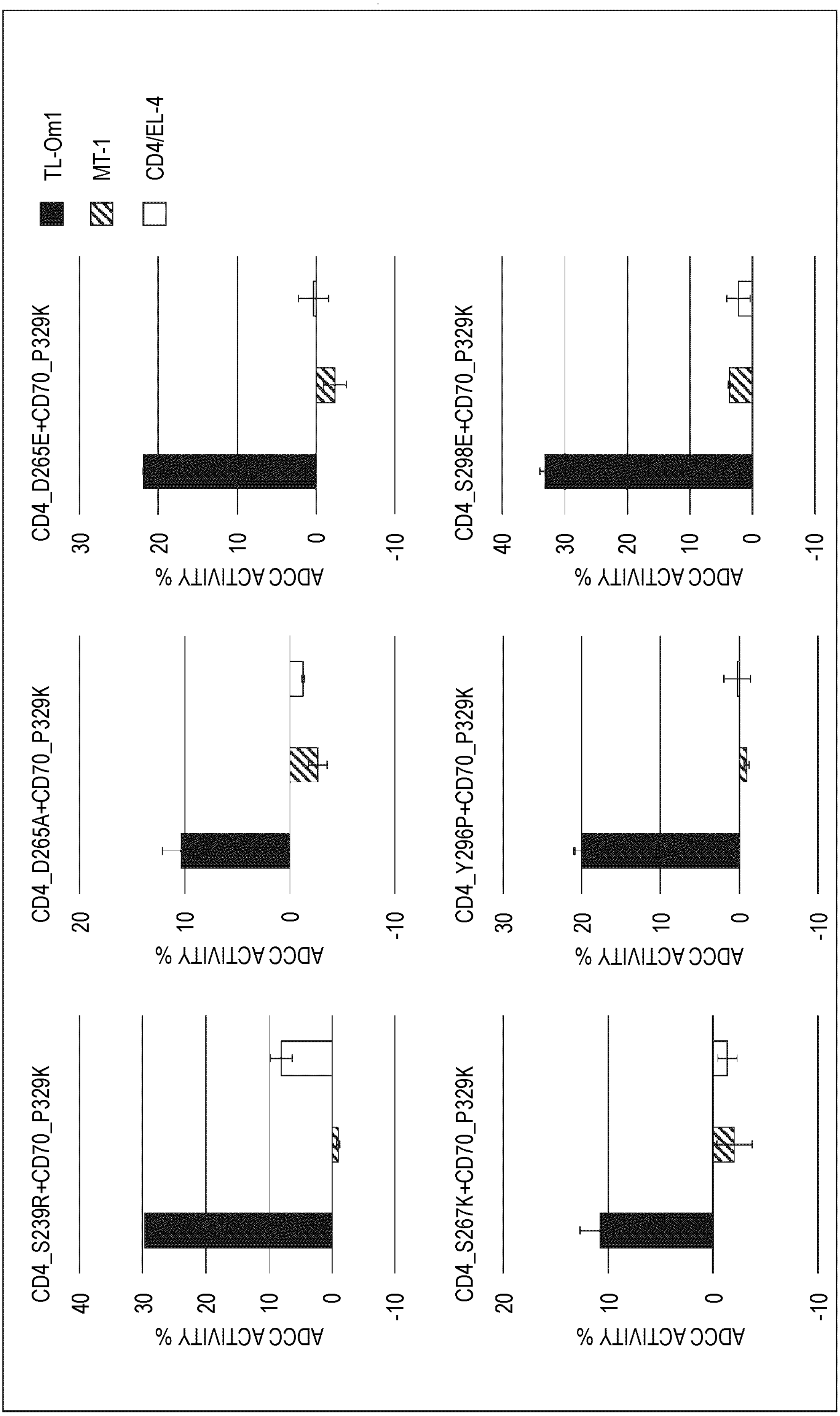
TL-Om1
MT-1
CD4/EL-4
CD4_S239R+CD70_P329K
CD4_D265A+CD70_P329K
CD4_D265E+CD70_P329K
CD4_S267K+CD70_P329K
CD4_Y296P+CD70_P329K
CD4_S298E+CD70_P329K
ADCC ACTIVITY %

FIG. 15E

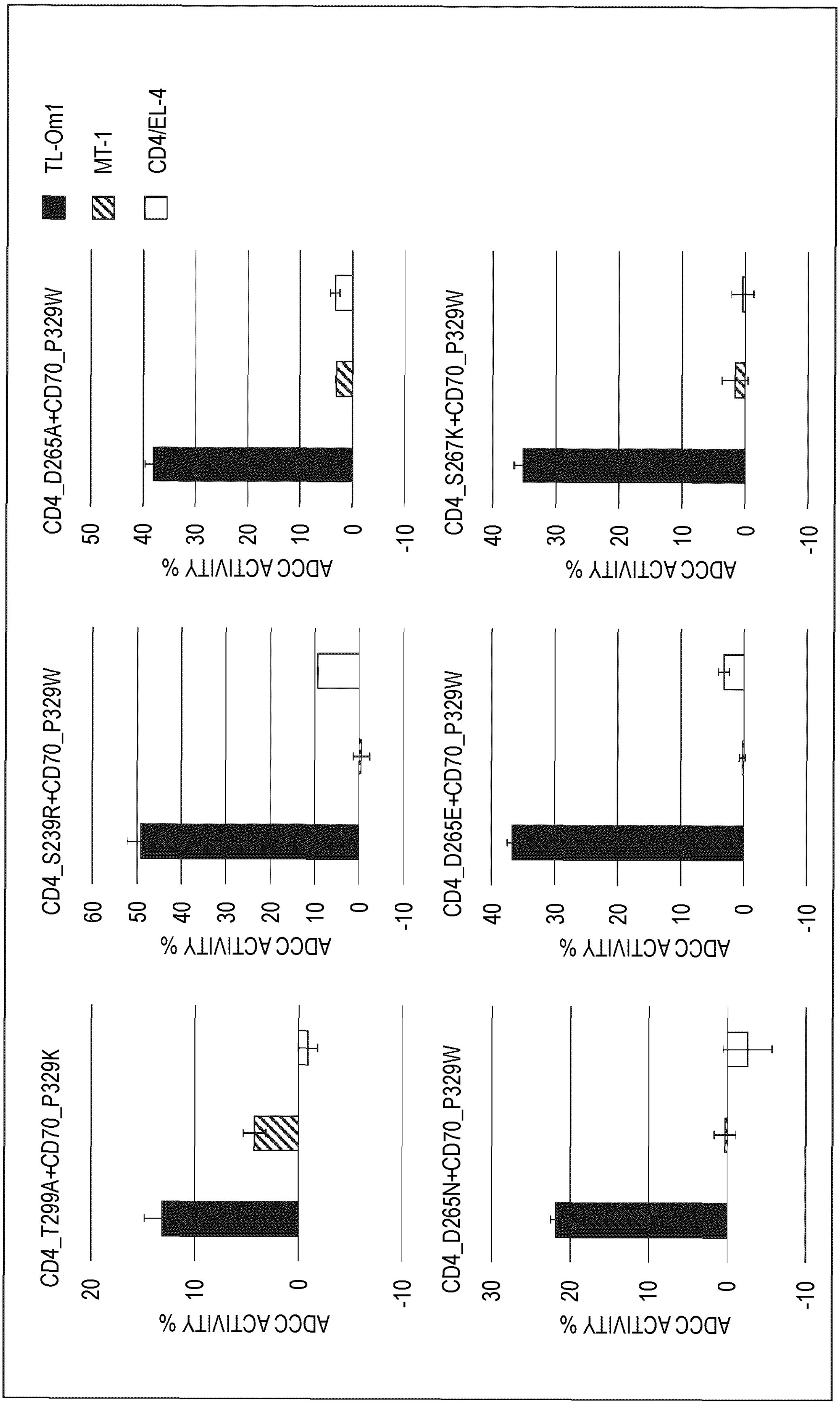

CD4_T299A+CD70_P329K
CD4_S239R+CD70_P329W
CD4_D265A+CD70_P329W
CD4_D265N+CD70_P329W
CD4_D265E+CD70_P329W
CD4_S267K+CD70_P329W
ADCC ACTIVITY %
TL-Om1
MT-1
CD4/EL-4

FIG. 15F

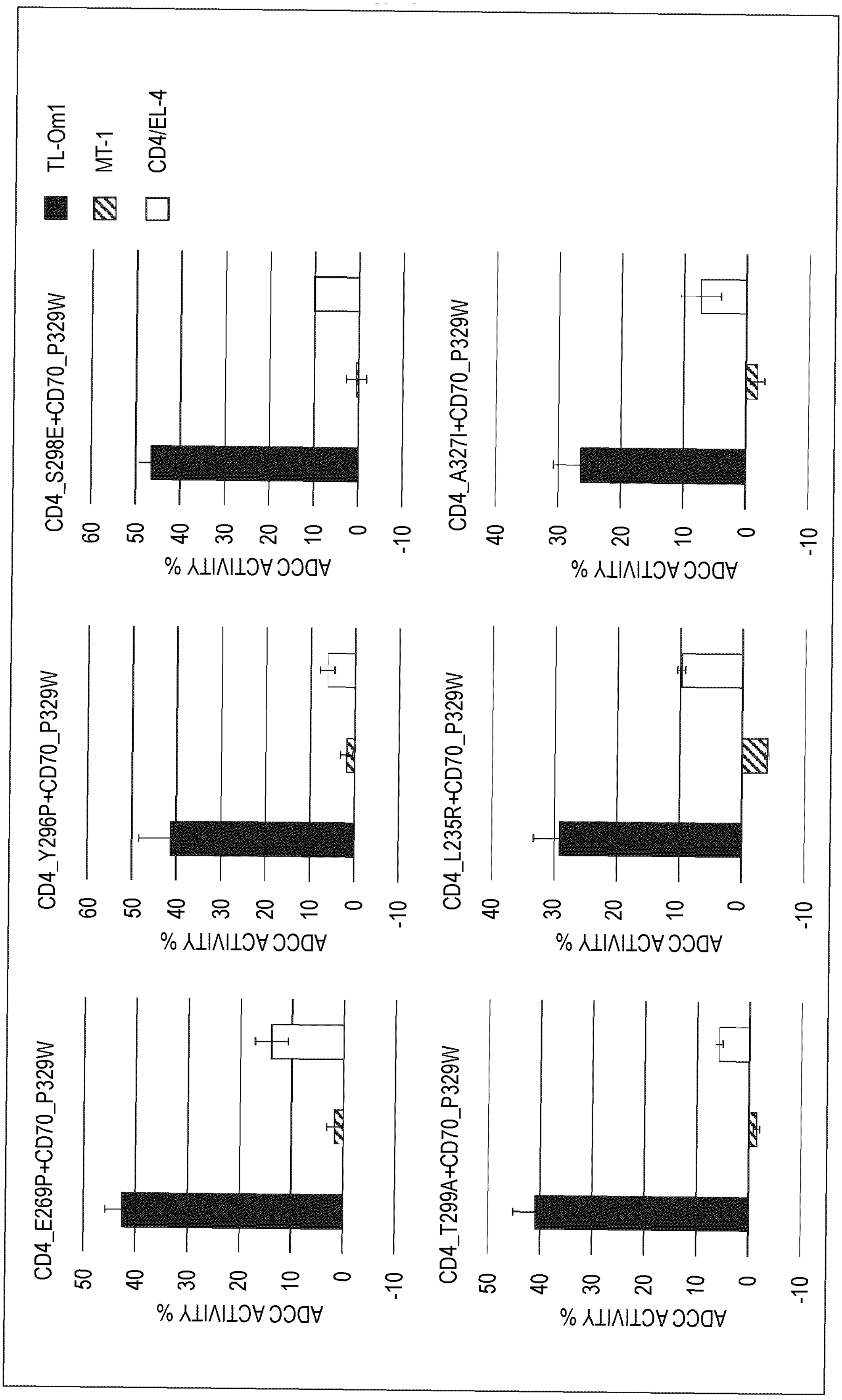
CD4_E269P+CD70_P329W
CD4_Y296P+CD70_P329W
CD4_S298E+CD70_P329W
CD4_T299A+CD70_P329W
CD4_L235R+CD70_P329W
CD4_A327I+CD70_P329W
ADCC ACTIVITY %
TL-Om1
MT-1
CD4/EL-4
60
50
40
30
20
10
0
-10

FIG. 15G

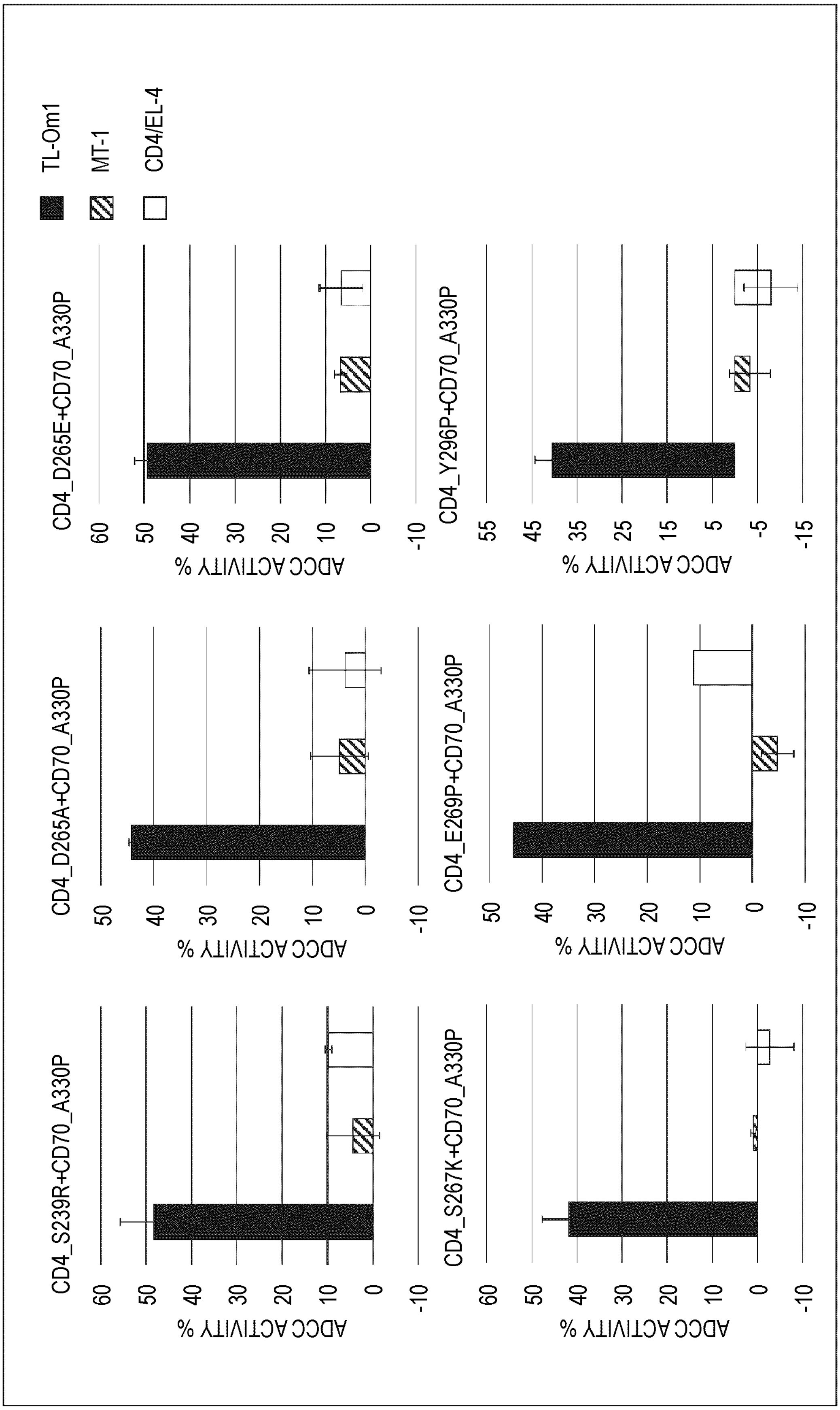
CD4_S239R+CD70_A330P
CD4_D265A+CD70_A330P
CD4_D265E+CD70_A330P
CD4_S267K+CD70_A330P
CD4_E269P+CD70_A330P
CD4_Y296P+CD70_A330P
ADCC ACTIVITY %
TL-Om1
MT-1
CD4/EL-4

FIG. 15H

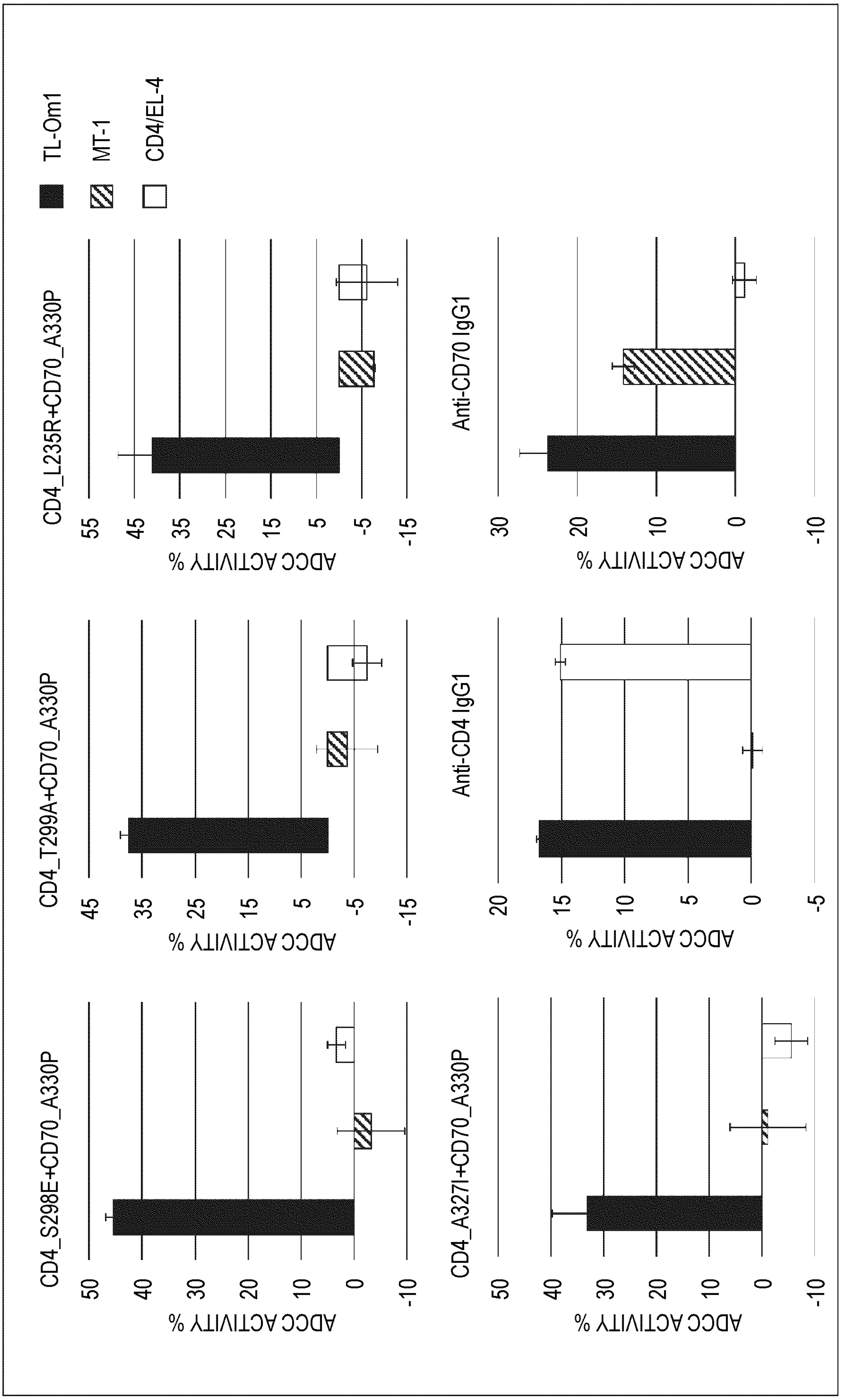
TL-Om1
MT-1
CD4/EL-4
CD4_S298E+CD70_A330P
CD4_T299A+CD70_A330P
CD4_L235R+CD70_A330P
CD4_A327I+CD70_A330P
Anti-CD4 IgG1
Anti-CD70 IgG1
ADCC ACTIVITY %
50
40
30
20
10
0
-10
45
35
25
15
5
-5
-15
55
15
5
-5

## INTERNATIONAL SEARCH REPORT

International application No.
PCT/JP2019/038421

A. CLASSIFICATION OF SUBJECT MATTER
Int.Cl. C12N15/13(2006.01)i, A61K39/395(2006.01)i, A61P43/00(2006.01)i, C07K16/28(2006.01)i, C07K16/46(2006.01)i, C12N1/15(2006.01)i, C12N1/19(2006.01)i, C12N1/21(2006.01)i, C12N5/10(2006.01)i, C12N15/63(2006.01)i, C12P21/08(2006.01)i
According to International Patent Classification (IPC) or to both national classification and IPC

B. FIELDS SEARCHED
Minimum documentation searched (classification system followed by classification symbols)
Int.Cl. C12N15/13, A61K39/395, A61P43/00, C07K16/28, C07K16/46, C12N1/15, C12N1/19, C12N1/21, C12N5/10, C12N15/63, C12P21/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII), CAplus/MEDLINE/EMBASE/BIOSIS(STN), WPIDS/WPIX(STN)

C. DOCUMENTS CONSIDERED TO BE RELEVANT

| Category* | Citation of document, with indication, where appropriate, of the relevant passages | Relevant to claim No. |
|---|---|---|
| Y | WO 2013/002362 A1 (CHUGAI PHARMACEUTICAL CO., LTD.) 03 January 2013, claims, examples, paragraphs [0020]-[0029], [0121], [0142] & EP 2728002 A1, claims, examples, paragraphs [0020]-[0029], [0125], [0148] & US 2014/0199294 A1 & KR 10-2014-0041787 A & CN 103827300 A | 1-30 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30.10.2019 | 19.11.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/038421

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2014/104165 A1 (CHUGAI PHARMACEUTICAL CO., LTD.) 03 July 2014, claims, examples, paragraphs [0019]-[0028], [0118], [0126] & EP 2940135 A1, claims, examples, paragraphs [0020]-[0029], [0124], [0133] & US 2015/0344570 A1 & KR 10-2015-0097786 A & CN 105102618 A | 1-30 |
| Y | JP 2008-511337 A (GENENTECH INC.) 17 April 2008, claims & US 2006/0204493 A1, claims & WO 2006/028936 A2 | 1-30 |
| Y | WO 2016/159213 A1 (CHUGAI PHARMACEUTICAL CO., LTD.) 06 October 2016, claims & US 2018/0057607 A, claims & WO 2016/159213 A1 | 1-30 |
| Y | WO 2008/090959 A1 (KYOWA HAKKO KOGYO KK) 31 July 2008, claims & US 2009/0004186 A1, claims & EP 2119780 A1 & KR 10-2009-0112723 A & CN 101679966 A | 1-30 |
| A | JP 2016-528166 A (F. HOFFMANN-LA ROCHE AG) 15 September 2016, entire text & US 2016/0068613 A & WO 2014/177459 A2 & CN 105164157 A & KR 10-2016-0003818 A | 1-30 |
| A | ESCOBAR-CABRERA, E. et al., Asymmetric Fc engineering for bispecific antibodies with reduced effector function, Antibodies, 16 May 2017, vol. 6, no. 2, pp. 7/1-7/16 | 1-30 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040259150
- WO 2005035586 A
- WO 200231140 A
- WO 0061739 A
- US 20070148165
- US 6737056 B
- US 7297775 B
- US 7317091 B
- JP H322979 A
- JP H2227075 A
- WO 9710354 A
- US 6001358 A
- WO 2010143698 A
- JP 2606856 B
- JP 2517813 B
- JP S63299 A
- WO 2000061739 A
- WO 2003085102 A
- JP H2257891 A
- JP S63309192 A
- WO 0231140 A
- WO 2013005649 A
- WO 2011108502 A
- WO 200703637 A
- JP 2018185367 A


### Non-patent literature cited in the description


- **CARTER P.** *Nat Rev Cancer,* 2001, vol. 1, 118-29
- **CARTRON G ; DACHEUX L ; SALLES G et al.** *Blood,* 2002, vol. 99, 754-8
- **WENG WK ; LEVY R.** *J Clin Oncol,* 2003, vol. 21, 3940-7
- Monoclonal Antibodies: Principles and Applications. Wiley-Liss, Inc, 1995, 45
- **AALBERSE RC ; SCHUURMAN J.** *Immunology,* 2002, vol. 105, 9-10
- **LABRIJN AF.** *Nat Biotechnol,* 2009, vol. 27, 767-71
- **SONDERMANN P.** *Nature,* 2000, vol. 406, 267-73
- **RADAEV S.** *J Biol Chem,* 2001, vol. 276, 16469-77
- **FERRARA C.** *Proc Natl Acad Sci,* 2011, vol. 108, 12660-74
- **MIZUSHIMA T.** *Genes Cells,* 2011, vol. 16, 1071-80
- **STROHL WR.** *Curr Opin Biotechnol,* 2009, vol. 20, 685-91
- **NIWA R.** *J Pharm Sci,* 2015, 930-41
- **BECK A.** *mAbs,* 2012, vol. 4, 419-25
- **GOEDE V.** *N Engl J Med,* 2014, vol. 370, 1101-10
- **BYRNE H.** *Trends Biotechnol,* 2013, vol. 31, 621-32
- **MAZOR Y.** *MAbs,* 2015, vol. 7, 377-89
- **KABAT et al.** Sequences of Proteins of Immunological Interest. US Dept. Health and Human Services, 1991
- **BRINKMANN U et al.** *MABS,* 2017, vol. 9, 182-212
- **FERNANDES CFC et al.** *Frontiers in Immunology,* 2017, vol. 8, 653
- **TOMIZUKA K. et al.** *Proc Natl Acad Sci USA.,* 2000, vol. 97, 722-7
- **WINTER G. et al.** *Annu Rev Immunol.,* 1994, vol. 12, 433-55
- **ROSEN A. et al.** *Nature,* 1977, vol. 267, 52-54
- *Chem. Today,* 2003, 65
- *Curr Opin Chem Biol.,* 2000, vol. 6, 645
- *Cancer Immunol. Immunother.,* 1993, vol. 36, 373
- *Annu. Rev. Immunol.,* 1991, vol. 9, 457-492
- Biochemical Experimentation Methods 23-Method for Studying Glycoprotein Sugar Chain. Japan Scientific Societies Press, 1989
- *J. Liq. Chromatogr.,* 1983, vol. 6, 1577
- Current Protocols in Molecular Biology, Antibodies, A Laboratory Manual. Molecular Cloning. Cold Spring Harbor Laboratory, 1988
- Monoclonal Antibodies: principles and practice. Acad. Press, 1993
- Antibody Engineering, A Practical Approach. IRL Press at Oxford University Press, 1996
- Molecular Cloning
- *Cytotechnology,* 1990, vol. 3, 133
- *Nature,* 1987, vol. 329, 840
- *J. Biochemistry,* 1987, vol. 101, 1307
- *Methods in Enzymol.,* 1987, vol. 154, 3

- Molecular Cloning: A Laboratory Manual. Cold Spring Harbor Lab. Press, 1989
- Current Protocols in Molecular Biology. vol. 1-34
- *Strategies,* 1992, vol. 5, 58
- *Nucleic Acids Research,* 1989, vol. 17, 9494
- *DNA Cloning: A Practical Approach,* 1985, vol. I, 49
- *Mol. Cell. Biol.,* 1983, vol. 3, 280
- *Gene,* 1985, vol. 33, 103
- *Strategies,* 1992, vol. 5, 81
- *Genetics,* 1954, vol. 39, 440
- *Science,* 1983, vol. 222, 778
- *Journal of Molecular Biology (J. Mol. Biol.,* 1983, vol. 166, 1
- *J. Mol. Biol.,* 1966, vol. 16, 118
- *Gene,* 1985, vol. 38, 275
- *Current Protocols in Molecular Biology,* vol. 1-34
- **SANGER et al.** *Proc. Natl. Acad. Sci. U. S. A.,* 1977, vol. 74, 5463
- Sequences of Proteins of Immunological Interest. US Dept. Health and Human Services, 1991
- *BIO/TECHNOLOGY,* 1991, vol. 9, 266
- *J. Mol. Biol.,* 1977, vol. 112, 535
- *Protein Engineering,* 1994, vol. 7, 1501
- Methods in Nucleic Acids Res. CRC press, 1991, vol. 283
- Methods in Nucleic Acids Res. CRC press, 1991
- *Proc. Natl. Acad. Sci. USA,* 1987, vol. 84, 7413
- Monoclonal Antibodies-Principles and practice. Academic Press, 1996
- Antibodies-A Laboratory Manual. Cold Spring Harbor Laboratory, 1988
- A manual for monoclonal antibody experiments. Kodansha scientific books, 1987
- *Proc. Natl. Acad. Sci. U. S. A.,* 1987, vol. 84, 7413
- Biomanual Series 4-Methods of Gene Transfer, Expression and Analysis (Yodosha). 1994
- Manipulating Mouse Embryo
- *Proc. Natl. Acad. Sci. USA,* 1980, vol. 77, 4216
- *Somatic Cell and Molecular genetics,* 1986, vol. 12, 55
- *Methods Enzymol.,* 1990, vol. 194, 182
- *Proc. Natl. Acad. Sci. U. S. A.,* 1978, vol. 84, 1929
- *J. Bacteriology,* 1983, vol. 153, 163
- *Proc. Natl. Acad. Sci. U. S. A.,* 1978, vol. 75, 1929
- Baculovirus Expression Vectors, A Laboratory Manual. W. H. Freeman and Company, 1992
- *Bio/Technology,* 1988, vol. 6, 47
- *American Journal of Clinical Nutrition,* 1996, vol. 63, 639S
- *American Journal of Clinical Nutrition,* 1996, vol. 63, 627S
- *Bio/Technology,* 1991, vol. 9, 830
- *Tissue Culture,* 1994, vol. 20
- *Tissue Culture,* 1995, vol. 21
- *Trends in Biotechnology,* 1997, vol. 15, 45
- *Nature,* 1970, vol. 227, 680
- Current Protocols in Molecular Biology; Antibodies, A Laboratory manual. Molecular Cloning. Cold Spring Harbor Laboratory, 1988
- Cancer Immunol. Immunother. 1993, vol. 36, 373
- *Cancer Immunol. Immunother,* 1993, vol. 36, 373
- *Agric. Biol. Chem.,* 1991, vol. 55 (1), 283-284
- *Anal. Biochem.,* 1988, vol. 171, 73
- Biochemical Experimentation Methods 23-Methods of Studies on Glycoprotein Sugar Chains. Japan Scientific Societies Press, 1989
- *J. Biochem.,* 1984, vol. 95, 197
- Monoclonal Antibodies: Principles and Applications. Wiley-Liss, Inc, 1995
- Enzyme Immunoassay. IGAKU-SHOIN Ltd, 1987
- Enzyme Antibody Technique. Gakusai Kikaku, 1985
- *Nature,* 2000, vol. 406, 267-73
- *J Biol Chem,* 2001, vol. 276, 16469-77
- *J. Immunol.,* 2011, vol. 187, 3238-3246
- *J. Immunol.,* 1992, vol. 149, 1779-1787